(19) Europäisches Patentamt
European Patent Office
Office européen des brevets

(11) EP 1 874 797 B1

(12) # FASCICULE DE BREVET EUROPEEN

(45) Date de publication et mention de la délivrance du brevet:
**21.10.2009 Bulletin 2009/43**

(21) Numéro de dépôt: **06755456.8**

(22) Date de dépôt: **27.04.2006**

(51) Int Cl.:
***C07K 1/00*** (2006.01) ***C07K 2/00*** (2006.01)
***A61K 47/48*** (2006.01) ***C08G 69/00*** (2006.01)
***A01N 61/00*** (2006.01)

(86) Numéro de dépôt international:
**PCT/FR2006/000952**

(87) Numéro de publication internationale:
**WO 2006/114528 (02.11.2006 Gazette 2006/44)**

(54) **PROCEDE DE PREPARATION DE POLYLYSINES DENDRIMERES GREFFES**

VERFAHREN ZUR HERSTELLUNG GEPFROPFTER POLYLYSIN-DENDRIMERE

METHOD OF PREPARING GRAFTED POLYLYSINE DENDRIMERS

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **28.04.2005 FR 0504309**

(43) Date de publication de la demande:
**09.01.2008 Bulletin 2008/02**

(73) Titulaires:
- **Centre National de la Recherche Scientifique 75794 Paris Cedex 16 (FR)**
- **Université Montpellier II 34095 Montpellier Cedex 05 (FR)**

(72) Inventeurs:
- **COMMEYRAS, Auguste F-34830 Clapiers (FR)**
- **COLLET, Hélène F-34090 Montpellier (FR)**
- **SOUAID, Eddy Jounieh (LB)**
- **COTTET, Hervé F-34000 Montpellier (FR)**
- **ROMESTAN, Bernard F-34980 Saint Gely du Fesc (FR)**
- **TRAMBOUZE, Odile, Yvonne, Marie F-34725 Saint Andre de Sangonis (FR)**

(74) Mandataire: **Grosset-Fournier, Chantal Catherine et al**
**Grosset-Fournier & Demachy**
**54, Rue Saint-Lazare**
**75009 Paris (FR)**

(56) Documents cités:
**WO-A-03/055935** **WO-A-03/064452**

- **RODRIGUEZ-HERNANDEZ JUAN ET AL: "Highly branched poly(L-lysine)" BIOMACROMOLECULES, ACS, WASHINGTON, DC, US, vol. 4, no. 2, mars 2003 (2003-03), pages 249-258, XP002250742 ISSN: 1525-7797 cité dans la demande**
- **KLOK H-A ET AL: "Dendritic-graft polypeptides" MACROMOLECULES, ACS, WASHINGTON, DC, US, vol. 35, no. 23, 5 novembre 2002 (2002-11-05), pages 8718-8723, XP002250744 ISSN: 0024-9297 cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne un procédé de préparation de polylysines dendrimères greffés, les polylysines dendrimères greffés obtenues par la mise en oeuvre dudit procédé, et l'utilisation desdites polylysines dendrimères, notamment dans le cadre de la préparation de compositions pharmaceutiques.

Deux méthodes sont décrites dans la littérature pour synthétiser des polylysines arborescentes. La méthode pas à pas initiée par Denkewalter et al [1,2] et la méthode par polymérisation d'acides aminés NCA initiée par Klok et al [3,4].

Les premières polylysines arborescentes (qui ont été qualifiées de dendrimères réguliers) ont été préparées dès 1981 par Denkewalter via des réactions successives de couplage et déprotection à partir de $N^{\alpha}$,$N'^{\varepsilon}$-di-(ter-butoxycarbonyl)-L-lysine [1,2].

Ces synthèses consistent à ajouter, à une lysine dont le groupement carboxylique est protégé par la benzhydrilamine (BHA), des monocouches successives de lysine, pour construire progressivement les composés suivants : BHALys, BHALysLys2, BHALysLys2Lys4, BHALysLys2Lys4Lys8, BHALysLys2Lys4Lys8Lys 16, BHALysLys2Lys4-Lys8Lys16Lys32, etc...

Les réactions de couplage sont réalisées en solvant anhydre ($CH_2Cl_2$, ou DMF).

Les composés obtenus portent en surface de nombreuses fonctions amines. Dans l'eau ils sont polycationiques.

Cette méthode de synthèse pas à pas est régulièrement reprise dans la littérature.

On la trouve par exemple utilisée dans les travaux de Matthews et al [5,6], de Baigude et al [7], de Menz et Chapman [8].

Par exemple, Matthews et al synthétisent dans un premier temps des polylysines identiques à celles de Denkewalter. Dans un deuxième temps ils en modifient leurs surfaces en liant covalentiellement à chaque fonction amine libre, des groupements portant des fonctions anioniques ($-CO_2^-$; $SO_3^-$, etc...) et obtiennent de nouveaux matériaux polyanioniques avec un coeur polylysine. Ces matériaux s'avèrent avoir des propriétés antivirales [5], antibactériennes et antiparasitaires [6] importantes. Baigude et al modifient les surfaces des dendrimères en liant leurs fonctions amines périphériques à des oligosaccharides et utilisent les matériaux obtenus pour produire des vaccins [7]. Takuro et al les utilisent naissantes pour transfecter des gènes [9]. Choi et al [10] construisent pas à pas des poly-L-lysine dendrimères à partir des deux $-NH_2$ du polyéthylèneglycol-$\alpha$,$\omega$-diamine, et montrent que les matériaux obtenus sont d'excellents transfecteurs de gènes. Ces multiples propriétés soulignent à l'évidence l'intérêt des polylysines arborescentes, comme tout ou partie d'une grande famille de nouveaux matériaux.

Ces polylysines dendrimères [1,2] sont considérées comme ayant un taux de ramification maximal.

Par ailleurs, l'intérêt de ces matériaux ne doit pas néanmoins masquer la complexité de la méthode de synthèse pas à pas. Par exemple six étapes, en plus des étapes de déprotections et de purifications, sont nécessaires pour oligocondenser 63 motifs Lysines. Il faut également noter la nécessité d'utiliser dans ces solvants anhydres des amorceurs non-lysine (BHA) qui peuvent poser des questions sur leurs possibles interactions biologiques.

Contrairement à la synthèse pas à pas précédente, la seconde méthode récemment proposée par Klok et al [3,4] consiste à polymériser les N-carboxyanhydrides de la lysine dans une procédure multi étape pour synthétiser des polylysines arborescentes. Les N-carboxyanhydrides d'aminoacides (NCA) sont des précurseurs bien connus des peptides [11]. Ces composés sont sensibles à l'hydrolyse, [12,13], ce qui n'empêche pas que l'eau soit utilisée comme solvant pour réaliser certaines études particulières [14-17]. Généralement, il est néanmoins préconisé de polymériser les N-carboxyanhydrides d'a-aminoacides en milieu anhydre [11,18,19]. Parmi les solvants anhydres recommandés on trouve le DMF, le dioxane, le DMSO, avec une préférence pour le DMF [20].

Les auteurs de cette seconde approche pour synthétiser des polylysines arborescentes à partir des N-carboxyanhydrides de la lysine [3] ont suivi les recommandations d'utilisation d'un milieu réactionnel anhydre. Il en a résulté une méthode de synthèse dans le DMF anhydre.

Dans cette méthode, la première étape consiste à copolymériser deux lysines N-carboxyanhydrides différemment protégées sur leur fonction epsilon (Z-Lys-NCA et Boc-Lys-NCA dans des rapports 5/1 à 2/1). Les groupements protecteurs sont choisis pour que leur déprotection puisse être sélective. Pour éviter de perdre des NCA par hydrolyse, la copolymérisation est réalisée dans un solvant (DMF) soigneusement rendu anhydre par les traitements appropriés. Dans ces conditions pour que la polymérisation puisse se produire, les traces d'acide provenant de la synthèse des NCA doivent être soigneusement éliminées [21]. Dans le DMF anhydre, la polymérisation est amorcée par une amine (hexylamine par exemple). Le temps de réaction est de 5 jours. En fin de réaction le milieu réactionnel est versé dans l'eau, et une polylysine linéaire poly([Z-L-lysine]-co-[Boc-L-lysine]) précipite. Après filtration et séchage, le produit subit un traitement acide ($CF_3CO_2H$ pendant 1 heure) qui supprime la fonction Boc, mais conserve la fonction Z. La poly([Z-L-lysine]-co-[L-lysine]) est ainsi formée. Elle est alors lavée et soigneusement séchée. Cette poly([Z-L-lysine]-co-[L-lysine]) linéaire, constitue un matériau nommé "coeur" par les auteurs à partir duquel des polylysines branchées sont obtenues dans des étapes ultérieures.

Ce matériau (coeur) est en effet mis en réaction, toujours dans le DMF anhydre pendant 5 jours, avec un

mélange des deux NCA précédents (Z-Lys-NCA et Boc-Lys-NCA) convenablement purifiés. Lors de cette réaction, les fonctions amines libres (ex Boc) de cette polylysine linéaire réagissent sur les Z-Lys-NCA et Boc-Lys-NCA, et il se forme une nouvelle {poly([Z-L-lysine]-co-[Boc-L-lysine]) branchée}. Après récupération du matériau formé par un processus analogue au précédent, ce matériau est soumis à un traitement acide ($CF_3CO_2H$) qui élimine les fonctions Boc, et conserve les fonctions Z. Le produit obtenu {poly([Z-L-lysine]-co-[L-lysine] branché) nommé G0}, est à nouveau soigneusement purifié et séché.

Ce produit est alors utilisé comme amorceur dans une nouvelle réaction dans le DMF anhydre pendant 5 jours sur un mélange des deux réactifs Z-Lys-NCA et Boc-Lys-NCA. Après récupération, séchage, traitement acide, et de nouveau séchage, un produit {poly([Z-L-lysine]-co-[L-lysine] branché) nommé G1} est obtenu.

Ce produit G1, est à son tour utilisé comme amorceur dans une réaction dans le DMF anhydre pendant 5 jours sur un mélange des deux réactifs Z-Lys-NCA et Boc-Lys-NCA. Aprés récupération, séchage, traitement acide, et de nouveau séchage, un produit {poly([Z-L-lysine]-co-[L-lysine] branché) nommé G2} est obtenu.

Les produits G0, G1, ou G2, sont alors soumis à un traitement en milieu très acide (HBr/ $CH_3CO_2H$). Ce traitement supprime les fonctions Z restantes, et on obtient des {poly(L-lysines branchées) G0, G1, ou G2}

Ces {poly-L-Lysines branchées} sont ensuite précipitées au diéthyléther, et lyophylisées.

Cette méthode permet donc effectivement d'obtenir des {poly-L-lysines branchées} de structures différentes de celles décrites initialement par Denkewalter et al.

Ces {Poly-L-Lysines branchées} [3], sont caractérisées (Tableau 1), par un taux de greffage égal ou inférieur à 30 % déduit du rapport Hb/Ha ≤ 0,08 de deux signaux 1H-NMR. Hb correspond au proton chiral des motifs lysines liées en epsilon aux lysines voisines, Ha correspond au proton chiral des motifs lysines liées en alpha aux lysines voisines, en excluant toutefois les protons des lysines N-terminales (Hc) et C-terminales (Hd) qui résonnent à un champ différent. Ces poly-L-lysines forment donc un réseau de polypeptides branchés (taux de greffage inférieur ou égal à 30 %) avec une amine primaire (hexylamine) comme amorceur de polymérisation.

**Tableau 1 : Caractéristiques des polylysines branchées décrites par Klok et al [3].**

| poly-L-lysines de Klok et al [3] | Mn (g/mole) déterminé par RMN | DPn | Points de branchements déterminés par le rapport $^1$H RMN (Hb/Ha) | Taux de greffage en % |
|---|---|---|---|---|
| Coeur | 2 670 | 20 | (Coeur des matériaux de Klok et al) Polylysine linéaire de 20 unités (Mn = 20) | |
| | | | 6 déterminés à partir du rapport Hb/Ha = 0,07 du G0 | 6/20 = 30% |
| G0 | 12400 | 96 | 14 déterminés à partir du rapport Hb/Ha = 0,08 du G1 | 14/96= 14% |
| G1 | 30 200 | 235 | 25 déterminés à partir du rapport Hb/Ha = 0,07 du G2 | 25/235 = 10% |
| G2 | 44600 | 347 | / | / |

*Les points de branchement sont les fonctions $NH_2$ en ε qui ont réagi sur les NCA. Le taux de greffage représentée le pourcentage des $NH_2$ en ε qui ont réagi par rapport au degré total de polymérisation d'un produit de génération n (DPn).*

Par conséquent, un objet de l'invention est de fournir un procédé de préparation de polypeptides, et notamment de polylysines dendrimères, permettant de réduire la complexité des procédés décrits ci-dessus.

En particulier, l'objet de la présente invention est de fournir un procédé de préparation de polypeptides, et notamment de polylysines dendrimères, plus rapide, et comportant moins d'étapes de synthèse et/ou de purification que les procédés de préparation déjà connus.

Un autre objet de l'invention concerne également les dendrimères pouvant être obtenus par la mise en oeuvre de ce procédé.

Enfin, un autre objet de l'invention concerne également l'utilisation des dendrimères obtenus par la mise en

oeuvre du procédé de l'invention pour la préparation de compositions pharmaceutiques ou antiseptiques, ainsi que les analyses de type immunologiques.

La présente invention concerne l'utilisation de monomères d'α-aminoacides activés pour la préparation de polypeptides hydrophobes sous forme de précipités, lesdits polypeptides résultant de la polymérisation desdits monomères d'α-aminoacides activés en solvant aqueux et étant susceptibles d'être resolubilisés dans ledit solvant.

On désigne par « monomère d'α-aminoacides activés » les α-aminoacides qui ont subi une modification qui les a rendus polymérisables dans des conditions normales de température et de pression.

On désigne par « polypeptides hydrophobes » les polypeptides insolubles dans l'eau ou dans les solvants aquo organiques utilisés. L'hydrophobie d'un soluté (polypeptide) peut être estimée par le coefficient de partage P du soluté entre l'eau et un solvant organique (généralement l'octanol pris comme référence). Le coefficient de partage P est défini comme le rapport de la concentration du soluté dans la phase organique divisée par la concentration en phase aqueuse. Le logarithme de P peut être estimé par les constantes de Rekker [22] ou déterminé expérimentalement par le rapport des concentrations dans la phase organique et dans la phase aqueuse.

On désigne par « solvant aqueux » tout solvant ou mélange de solvants miscibles comprenant majoritairement de l'eau (au moins 50% en poids).

On désigne par « resolubilisés » la remise en solution aqueuse d'un peptide hydrophobe par modification de ses constantes de Rekker ce qui peut être obtenu par exemple en débloquant le groupement protégeant la fonction epsilon d'une lysine.

Les avantages de cette utilisation sont que via un processus élémentaire cyclique facile à mettre en oeuvre : on peut 1/ synthétiser des peptides de taille contrôlée et les récupérer facilement, 2/ les resolubiliser par déprotection, 3/ accroître leur taille de manière également contrôlée et les récupérer tout aussi facilement du milieu réactionnel.

Dans un mode de réalisation particulier de l'utilisation définie ci-dessus, les α-aminoacides activés sont choisis parmi des α-aminoacides N-carboxyanhydrides (NCA), des α-aminoacides-N,N'-carbonyldiimidazole, des α-aminoacides-sulfure de carbonyle, des α-aminoacides-anhydride carbonique, et des amino thioacides-agents oxydants.

Les α-aminoacides N-carboxyanhydrides (NCA) sont préparés selon une des méthodes décrites [23-29] mais également par des méthodes indirectes analysées par Pascal et al [30].

Dans un autre mode de réalisation particulier de l'utilisation définie ci-dessus, les α-aminoacides activés sont des α-aminoacides N-carboxyanhydrides (NCA) de formule (1) suivante :

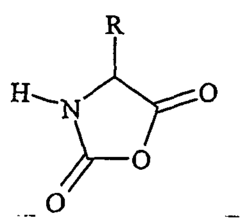

dans laquelle R représente une chaîne latérale d'un α-aminoacide naturel ou modifié.

On désigne par acide aminé naturel les acides aminés qui se trouvent dans les systèmes vivants.

On désigne par acide aminé modifié les acides aminés naturels qui ont subit une modification sur leur chaîne latérale (R).

Dans un mode de réalisation plus particulier de l'utilisation définie ci-dessus, on utilise des monomères de L-lysine-NCA pour la préparation de polylysine en solvant aqueux.

On désigne par polylysine tout polymère dont l'élément unitaire majoritaire est la lysine.

Dans les monomères de L-lysine-NCA, le groupement amine en ε peut être protégé ou non.

On désigne par « protection du groupement amine » la fixation réversible d'un groupement chimique, dit groupement protecteur sur le groupement amine de façon à diminuer, voire à abolir sa réactivité et à modifier l'hydrophobie de la molécule.

La présente invention concerne également un procédé de préparation d'une homo ou hétéropolylysine dendrimère greffé, à partir d'un amorceur comprenant au moins un groupement amine primaire ou secondaire, comprenant une étape d'addition d'un monomère de L-lysine-NCA, et éventuellement d'un ou plusieurs autres monomères d'α-aminoacides-NCA, notamment choisis parmi la liste comprenant la L-ornithine-NCA, l'acide L-glutamique-NCA et sa γ-amide, l'acide L-aspartique-NCA et sa β-amide, l'acide L-diamino-2,4,-butyrique-NCA et sa β-amide, la L-tyrosine-NCA, la L-sérine-NCA, la L-thréonine-NCA, la L-phénylalanine-NCA, la L-valine-NCA, la L-leucine-NCA, la L-isoleucine-NCA, la L-alanine-NCA, et la glycine-NCA, audit amorceur dans un solvant aqueux. Ces autres monomères (NCA) peuvent être utilisés dans la dernière étape de construction du dendrimère greffé pour modifier les fonctions de surface du matériau final.

On désigne par "homopolylysine" une polylysine constituée uniquement de lysine.

On désigne par "hétéropolylysine" une polylysine constituée de plus de 30% en mole de lysine et contenant également des unités de nature différente.

On désigne par dendrimère greffé, notamment polylysine dendrimère greffé, une macromolécule dont la structure est plus flexible, plus étendue et moins régulière que celle des dendrimères (décrits initialement par Denkewalter [1]) mais plus structurée que celle des molécules branchées de Klok et al [3,4]. De telles structures flexibles ont des propriétés uniques différentes de celles des dendrimères traditionnels ainsi que cela est indiqué page 18 dans l'ouvrage "Dendrimers and other dendritic polymers" édité par Fréchet et Tomalia [31].

On désigne par « amorceur » toute espèce chimique susceptible d'initier un processus de polymérisation. Dans le cas des acides aminés activés les amorceurs sont des espèces nucléophiles telles que : une $C_1$-$C_{12}$ alkylamine primaire, une $C_1$-$C_{12}$ alkyldiamine, une $C_1$-$C_{12}$ alkylamine secondaire ou tertiaire, une arylamine polyaromatique détectable en UV ou par fluorescence, une alkyl- ou arylamine primaire ou secondaire portant un atome radioactif détectable, un acide aminé ayant une ou plusieurs fonctions amines libres, un peptide ayant une ou plusieurs fonctions amines libres, un dendrimère ayant une ou plusieurs fonctions amines libres, un polymère ayant une ou plusieurs fonctions amines libres.

Pour la synthèse du dendrimère de la génération i (i ≥ 2), l'amorceur utilisé correspond au dendrimère greffé déprotégé de génération i-1.

Lors de la synthèse du dendrimère de génération 1 selon l'invention, il est possible de ne pas ajouter d'amorceur, i.e. un amorceur externe, au mélange réactionnel lorsque l'on veut produire des peptides ne comportant que des motifs identiques à celui du (ou des) aminoacide(s) activé(s) (NCA) de départ. En effet, dans ce cas, l'hydrolyse du (ou des) NCA (initiée par la présence d'eau dans le solvant) libère le ou les acide(s) aminé(s) nécessaire(s) à l'amorçage. Ainsi, pour la synthèse de la première génération (nommée P1), on peut utiliser ou non un amorceur externe et dans le cas où on en utilise un, il peut être différent du monomère d'aminoacide utilisé.

Dans le cas particulier de la synthèse de dendrimère d'homopolylysine, si on n'utilise pas d'amorceur externe dans l'étape de synthèse du produit de première génération (P1), on obtient un résultat identique à celui obtenu en utilisant comme amorceur la lysine protégée en ε. Avec une lysine non protégée comme amorceur externe, l'allongement de la chaîne a comme point de départ les deux fonctions amines en α et en ε. Dans ces deux cas le rendement de la réaction est de 5% supérieur au cas où on n'utilise pas d'amorceur externe. Par contre, dans chaque étape subséquente conduisant aux produits de génération 2 (P2), de génération 3 (P3) ou de génération n (Pn), l'amorceur utilisé est le produit obtenu dans l'étape Pn-1.

Dans un mode de réalisation particulier du procédé de préparation défini ci-dessus, le monomère est uniquement de la L-lysine-NCA.

Dans un autre mode de réalisation particulier du procédé de préparation défini ci-dessus, la L-lysine-NCA est protégée en $N^ε$, notamment par un groupement choisi parmi -COH (Formyl), $-COCF_3$ (TFA), $-OCOC(CH_3)_3$ (Boc), $-COOCH_2Φ$ (Z),

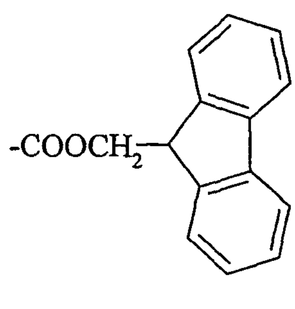

(Fmoc)

ou $-C(Φ)_3$ (trityl).

Les groupements ci-dessus ont été classés par ordre d'hydrophobie croissante, leurs constantes de Rekker respectives sont données par log P = -1,528, -0,946, 0,964, 1,124, 2,849, 5,81.

Avantageusement, la nature du groupement protecteur permet de moduler la taille de la polylysine obtenue. En effet, plus le groupement protecteur sera hydrophobe et moins le nombre d'unités de lysine protégées présentes dans la polylysine nécessaire pour entraîner la précipitation de la polylysine sera important. Ainsi, la taille de la polylysine obtenue avec des groupements protecteurs faiblement hydrophobes (Formyl) sera supérieure à la taille de polylysines obtenues avec des groupements plus hydrophobes (TFA).

Dans un autre mode de réalisation particulier du procédé de préparation défini ci-dessus, l'amorceur est choisi parmi la L-lysine, la L-ornithine, une homopolylysine, un poly(éthylène glycol)-α,ω-diamine, une hétéropolylysine, un hétéropeptide, ou un homopeptide.

On désigne par homopeptide un peptide dont tous les résidus sont identiques. On désigne par hétéropeptide un peptide constitué de résidus de nature différente.

Dans un autre mode de réalisation du procédé de préparation défini ci-dessus, le pH du solvant est d'environ 3 à 9.

L'utilisation de cet intervalle de pH correspond au domaine de stabilité de la liaison peptidique [32] et conduit à une augmentation du rendement de la réaction par rapport à des pH hors de cet intervalle.

Selon un mode de réalisation préféré, l'invention concerne un procédé de préparation d'une homo ou hétéropolylysine dendrimère greffé de génération 1 (P1) tel que défini ci-dessus, comprenant les étapes suivantes :

- l'addition de la L-lysine-NCA protégée en $N^{\varepsilon}$ à un amorceur dans un solvant aqueux, à un pH approprié, pour obtenir de la polylysine dendrimère greffé protégée de génération 1 sous forme d'un précipité,
- la déprotection de la polylysine dendrimère greffé protégée de génération 1 obtenue à l'étape précédente, pour obtenir de la polylysine dendrimère greffé de génération 1.

On désigne par « génération » le produit obtenu lors de chaque phase de croissance de cette macromolécule.

On désigne par « génération 1 » le produit de la première réaction de polymérisation de la TFA-L-Lysine NCA, sans amorceur externe, ou avec la TFA-L-lysine ou tout autre amorceur défini ci-dessus.

On notera que selon l'invention le dendrimère greffé de génération 1 (P1) est linéaire.

Dans un mode de réalisation préféré du procédé de préparation d'une homopolylysine dendrimère greffé de génération 1 tel que défini ci-dessus, l'amorceur est de la L-lysine protégée en $N^{\varepsilon}$ ou non protégée.

L'utilisation de L-lysine protégée en $N^{\varepsilon}$ comme amorceur permet d'obtenir un homopolypeptide de L-lysine.

L'utilisation de L-lysine non protégée permet l'élongation de deux chaînes homopolypeptidiques de L-lysine à partir des amines en $\alpha$ et en $\varepsilon$ respectivement.

Dans un autre mode de réalisation du procédé de préparation d'une hétéropolylysine dendrimère de génération 1 tel que défini ci-dessus, l'amorceur est un poly(ethylene glycol)-$\alpha,\omega$-diamine, dont la masse moléculaire peut être comprise entre 100 Da et 10 000 Da, notamment entre 1 000 Da et 10 000 Da.

Dans un autre mode de réalisation préféré de l'invention, le procédé de préparation d'une homopolylysine dendrimère de génération 1 tel que défini ci-dessus, comporte :

- une étape d'addition de L-lysine-NCA protégée en $N^{\varepsilon}$ par un groupement TFA, à une solution aqueuse de pH d'environ 6 à environ 8, sans ajout d'amorceur ou avec un amorceur L-lysine protégée en $N^{\varepsilon}$ par un groupement TFA, pour obtenir un précipité de polylysine dendrimère protégée de génération 1, et
- une étape de déprotection du polymère de polylysine obtenu à l'étape précédente pour obtenir une polylysine dendrimère de génération 1, linéaire, de masse moléculaire moyenne approximative 1400 Da, notamment 1 450 Da, présentant un indice de polymolécularité d'environ 1,2 et correspondant à un degré de polymérisation moyen de 8 unités de lysine.

Dans ce qui précède et ce qui suit, on désigne par « masse moléculaire moyenne » la masse moléculaire moyenne en nombre $M_n = \frac{\sum_i N_i M_i}{\sum_i N_i}$ où la somme porte sur toutes les masses moléculaires possibles, $N_i$ étant le nombre de molécules ayant la masse moléculaire $M_i$. La masse moléculaire moyenne en poids est définie par L'indice de polydispersité (ou polymolécularité) est défini par $I=M_w/M_n$. De la même façon, on définit le degré de polymérisation moyen en nombre par $DP_n = \frac{\sum_i N_i DP_i}{\sum_i N_i}$ et le degré de polymérisation moyen en poids par

$$DP_w = \frac{\sum_i N_i DP_i^2}{\sum_i N_i DP_i}$$

. On désigne par "degré de polymérisation moyen" le nombre moyen de monomères contenu dans la chaîne de polymère.

Les masses moléculaires moyennes (en nombre ou en poids), le degré de polymérisation moyen ainsi que

l'indice de polymolécularité ont été déterminées de façon absolue par chromatographie d'exclusion stérique couplée à la diffusion de la lumière et à un détecteur d'indice de réfraction différentiel. Pour la première génération ($P_1$, polymère de faible masse moléculaire) la détermination de la masse moléculaire absolue a été réalisée par électrophorèse capillaire. Le résultat obtenu est en accord avec les résultats obtenus en RMN, et par spectrométrie de masse MALDI-TOF.

La présente invention concerne également un procédé de préparation d'une homo ou hétéropolylysine dendrimère greffé de génération n tel que défini ci-dessus, dans lequel n est un nombre entier de 2 à 10 :

- comportant une étape d'addition de la L-lysine-NCA protégée en $N^{\varepsilon}$ à un amorceur constitué d'une polylysine dendrimère greffé de génération n-1, dans un solvant aqueux, à un pH approprié, pour obtenir de la polylysine dendrimère greffé protégée de génération n sous forme d'un précipité,

    o ladite polylysine dendrimère greffé de génération n-1 étant elle-même obtenue à partir de l'addition de la L-lysine-NCA protégée en $N^{\varepsilon}$ à un amorceur constitué d'une polylysine dendrimère greffé de génération n-2, dans un solvant aqueux, à un pH approprié, pour obtenir une polylysine dendrimère greffé protégée de génération n-1, et la déprotection de ladite polylysine,
    o ladite polylysine dendrimère greffé de génération n-2 étant elle-même obtenue comme indiqué à propos de la polylysine dendrimère greffé de génération n-1,
    o et lorsque n = 2, la polylysine dendrimère greffé de génération 1 est telle que définie ci-dessus, ladite polylysine dendrimère greffé de génération 1 formant le coeur de la polylysine dendrimère greffé de génération n,

- une étape de déprotection de la polylysine dendrimère greffé protégée de génération n pour obtenir la polylysine dendrimère greffé de génération n.

Par "pH approprié", on désigne un pH compris entre 3 et 9 et plus particulièrement un pH de 6,5.

Dans un mode de réalisation particulier, l'invention concerne un procédé de préparation d'une homopolylysine dendrimère greffé de génération n (Pn) tel que défini ci-dessus, dans lequel la L-lysine-NCA est protégée en $N^{\varepsilon}$ par du TFA, le coeur de ladite homopolylysine dendrimère greffé de génération n étant formé d'une polylysine linéaire comprenant environ 8 résidus de L-lysine, telle que pouvant être obtenue par la mise en oeuvre du procédé de préparation d'une homopolylysine dendrimère de génération 1 tel que défini ci-dessus, et le degré de ramification de ladite homopolylysine dendrimère greffé de génération n étant d'environ 40% à environ 100%, notamment d'environ 60% à environ 100%.

On désigne indifféremment par « degré de ramification », « taux de greffage », ou « taux de branchement » d'une polylysine dendrimère greffé de génération n (Pn), le pourcentage des $\varepsilon$ $NH_2$ de Pn qui ont réagi par rapport au degré total de polymérisation du produit de génération n (DPn).

Le moyen préféré pour déterminer le degré de ramification est la mesure du rapport entre l'intensité des signaux RMN des protons Hb et Ha. Ces signaux correspondent : Hb (4,01 ppm) à la résonance du proton porté par les carbones chiraux des motifs lysines liés à des fonctions amines en $\varepsilon$, Ha (4,08 ppm) à la résonance des protons portés par les carbones chiraux des motifs lysines liés à des fonctions amines en $\alpha$, en excluant les protons portés par les motifs lysines N-terminal (Hc) et C-terminal (Hd) qui résonnent entre 3,5 et 3,9 ppm. Le rapport entre l'intensité de ces deux signaux permet d'apprécier le degré de ramification de ces dendrimères greffés [3].

Avantageusement, les rapports Hb/Ha des polylysines dendrimères greffés selon l'invention sont d'environ 0,2 à environ 0,8, notamment d'environ 0,25 à environ 0,60.

Dans un mode de réalisation préféré, l'invention concerne un procédé de préparation d'une homopolylysine dendrimère greffé de génération 2 (P2) tel que défini ci-dessus comportant :

- une étape d'addition de la L-lysine-NCA protégée en $N^{\varepsilon}$ par du TFA à un amorceur constitué d'une polylysine dendrimère de génération 1,

    o ladite polylysine dendrimère de génération 1 étant telle que celle obtenue par la mise en oeuvre du procédé de préparation d'une homopolylysine dendrimère de génération 1 comprenant environ 8 unités de lysine, tel que défini ci-dessus,
    o le rapport massique (L-lysine-NCA protégée en N$\varepsilon$ par du TFA) / (polylysine dendrimère de génération 1) étant d'environ 2,6 à environ 3,9, notamment environ 3,

  ladite étape d'addition ayant lieu dans un solvant aqueux, à un pH approprié, pour obtenir une polylysine dendrimère protégée de génération 2,
- une étape de déprotection de la polylysine obtenue à l'étape précédente, pour obtenir une polylysine dendrimère greffé de génération 2 (P2) ayant un poids moléculaire moyen d'environ 6 000 à environ 14 000 Da, notamment

d'environ 8 350 Da, de préférence d'environ 8 600 Da, une polydispersité d'environ 1,4, et d'environ 40 à environ 60, notamment à environ 48, groupements $-NH_2$ externes libres.

L'homopolylysine dendrimère greffé de génération 2 ci-dessus est également **caractérisée en ce que** son rapport Hb/Ha est d'environ 0,1 à environ 0,8, notamment d'environ 0,2 à environ 0,3, plus particulièrement environ 0,25. Par ailleurs, lors de la synthèse de l'homopolylysine dendrimère greffé de génération 2 ci-dessus, l'amorceur P1 subit un taux de greffage d'environ 50% à environ 100%, notamment d'environ 80% à environ 100%, plus particulièrement environ 100%.

On désigne par « groupements $-NH_2$ externes libres » les groupements $-NH_2$ des fonctions amine en $\varepsilon$ déprotégés et les groupements $NH_2$ en position $\alpha$.

Le mode préféré pour déterminer la présence des groupements $-NH_2$ libres est la RMN. Par exemple lorsqu'on utilise la $N^{\varepsilon}$-trifluoacétyllysine, l'élimination du groupement protecteur et l'émergence de la fonction amine libre, sont suivies en RMN du $^{19}F$ par la disparition du signal du groupement trifluoroacétyle (à -76 ppm) et l'apparition simultanée du signal du trifluoroacétate (à -75,8 ppm). Cette disparition est totale en 15 heures à 40°C dans une solution eau/méthanol/ammoniac 1 M).

Dans un autre mode de réalisation préféré, l'invention concerne un procédé de préparation d'une homopolylysine dendrimère greffé de génération 3 (P3) tel que défini ci-dessus comportant :

- une étape d'addition de la L-lysine-NCA protégée en $N^{\varepsilon}$ par du TFA à un amorceur constitué d'une polylysine dendrimère greffé de génération 2,

  o ladite polylysine dendrimère greffé de génération 2 étant telle que celle obtenue par la mise en oeuvre du procédé défini ci-dessus,
  o le rapport massique (L-lysine-NCA protégée en $N^{\varepsilon}$ par du TFA) / (polylysine dendrimère greffé de génération 2) étant d'environ 2,6 à environ 3,9, notamment environ 3,

  ladite étape d'addition ayant lieu dans un solvant aqueux, à un pH approprié, pour obtenir une polylysine dendrimère greffé protégée de génération 3 (P3),
- une étape de déprotection de la polylysine obtenue à l'étape précédente pour obtenir une polylysine dendrimère greffé de génération 3 ayant un poids moléculaire moyen d'environ 15 000 à environ 30 000 Da, notamment d'environ 21 500 Da, de préférence d'environ 22 000 Da, une polydispersité d'environ 1,4, et d'environ 100 à environ 150, notamment à environ 123, groupements $-NH_2$ externes libres.

L'homopolylysine dendrimère greffé de génération 3 ci-dessus est également **caractérisée en ce que** son rapport Hb/Ha est d'environ 0,2 à environ 0,8, notamment d'environ 0,5 à environ 0,7, plus particulièrement environ 0,6. Par ailleurs, lors de la synthèse de l'homopolylysine dendrimère greffé de génération 3 ci-dessus, l'amorceur P2 subit un taux de greffage d'environ 50% à environ 90%, notamment d'environ 70% à environ 90%, plus particulièrement environ 81 %.

Dans un autre mode de réalisation préféré, l'invention concerne un procédé de préparation d'une homopolylysine dendrimère greffé de génération 4 (P4) tel que défini ci-dessus comportant :

- une étape d'addition de la L-lysine-NCA protégée en $N^{\varepsilon}$ par du TFA à un amorceur constitué d'une polylysine dendrimère greffé de génération 3,

  o ladite polylysine dendrimère greffé de génération 3 étant telle que celle obtenue par la mise en oeuvre du procédé défini ci-dessus,
  o le rapport (L-lysine-NCA protégée en $N\varepsilon$ par du TFA) / (polylysine dendrimère greffé de génération 3 étant d'environ 2,6 à environ 3,9, notamment environ 3,

  ladite étape d'addition ayant lieu dans un solvant aqueux, à un pH approprié, pour obtenir une polylysine dendrimère greffé protégée de génération 4 (P4),
- une étape de déprotection de la polylysine obtenue à l'étape précédente pour obtenir une polylysine dendrimère greffé de génération 4 ayant un poids moléculaire moyen d'environ 50 000 à environ 80 000 Da, notamment d'environ 64 000 Da, de préférence d'environ 65 000 Da ou 65 300 Da, une polydispersité d'environ 1,4, et d'environ 300 à environ 450, notamment environ 365 groupements $-NH_2$ externes libres.

L'homopolylysine dendrimère greffé de génération 4 ci-dessus est également **caractérisée en ce que** son rapport Hb/Ha est d'environ 0,2 à environ 0,8, notamment d'environ 0,4 à environ 0,5, plus particulièrement environ

0,46. Par ailleurs, lors de la synthèse de l'homopolylysine dendrimère greffé de génération 4 ci-dessus, l'amorceur P3 subit un taux de greffage d'environ 50% à environ 90%, notamment d'environ 70% à environ 90%, plus particulièrement environ 80%.

Dans un autre mode de réalisation préféré, l'invention concerne un procédé de préparation d'une homopolylysine dendrimère greffé de génération 5 (P5) tel que défini ci-dessus comportant :

- une étape d'addition de la L-lysine-NCA protégée en $N^{\varepsilon}$ par du TFA à un amorceur constitué d'une polylysine dendrimère greffé de génération 4,

  o ladite polylysine dendrimère greffé de génération 4 étant telle que celle obtenue par la mise en oeuvre du procédé défini ci-dessus,
  o le rapport (L-lysine-NCA protégée en Nε par du TFA) / (polylysine dendrimère greffé de génération 4) étant d'environ 2,6 à environ 3,9, notamment environ 3,

  ladite étape d'addition ayant lieu dans un solvant aqueux, à un pH approprié, pour obtenir une polylysine dendrimère greffé protégée de génération 5,
- une étape de déprotection de la polylysine obtenue à l'étape précédente pour obtenir une polylysine dendrimère greffé de génération 5 ayant un poids moléculaire moyen d'environ 140 000 à environ 200 000 Da, notamment d'environ 169 000 Da, de préférence d'environ 172 000 Da ou 172 300 Da, une polydispersité d'environ 1,4, à d'environ 900 à environ 1100, notamment à environ 963, groupements $-NH_2$ externes libres.

L'homopolylysine dendrimère greffé de génération 5 ci-dessus est également **caractérisée en ce que** son rapport Hb/Ha est d'environ 0,2 à environ 0,8, notamment d'environ 0,3 à environ 0,5, plus particulièrement environ 0,4. Par ailleurs, lors de la synthèse de l'homopolylysine dendrimère greffé de génération 5 ci-dessus, l'amorceur P4 subit un taux de greffage d'environ 50% à environ 90%, notamment d'environ 60% à environ 70%, plus particulièrement environ 65%.

La présente invention concerne également un procédé de préparation d'une homo- ou hétéropolylysine dendrimère greffé tel que défini ci-dessus, dans lequel l'amorceur, notamment celui utilisé pour la préparation de la génération 1, est fixé de façon covalente au dendrimère greffé, ledit amorceur comportant un produit marqueur, ce qui rend le dendrimère facilement détectable par les moyens suivants : absorption UV, émission fluorescente, opacité aux rayons X ou sensibilité aux champs magnétiques. Les formules respectives suivantes sont prises comme exemples de produits marqueurs sans être limitatives : acide 7-amino-1,3-naphtalène-disulphonique, aminofluorescéine, 3-(4-carboxybenzoyl)quinoline-2-carboxaldéhyde, rhodamine sulfochlorure (Texas Red), agents de contraste polyiodés tels que les amidotrizoates ou l'ioxaglate, ou encore des clusters de métaux (Au, Fe, Ga, Mn) portant des fonctions aldéhydes tels que $Au_{11}(P(C_6H_4)CONHCH_2CHO)_3)_7$), des fonctions acides carboxyliques ou d'autres fonctions actives sur les amines, utilisés en médecine dans un but diagnostique pour renforcer le contraste en imagerie aux rayons X et par résonance magnétique (IRM). Lorsque les molécules susmentionnées marquées portent des fonctions amines, telles que par exemple les deux premières citées on peut les utiliser pour amorcer la polymérisation des NCA. Elles sont alors liées de façon covalente au dendrimère greffé de première génération (P1) par une liaison amide sur le C terminal. Lorsque par exemple les molécules marquées portent au contraire des fonctions telles que aldéhydes, sulfochlorures, acides carboxyliques, capables de réagir sur les amines, alors on peut, en utilisant les méthodes de l'art, les lier de façon covalente au N terminal du dendrimère (P1) avant la déprotection des fonctions $N^{\varepsilon}$-TFA. Ces opérations sont effectuées dans le DMF, et le produit réactionnel est ensuite récupéré par précipitation dans l'eau. Après déblocage des fonctions $N^{\varepsilon}$-TFA on poursuit la construction du dendrimère. Dès la deuxième génération du dendrimère, et a fortiori la troisième, les produits marqueurs se retrouvent "enfouis" au coeur du dendrimère. Ils sont ainsi invisibles par les systèmes immunitaires comme le dendrimère lui-même ainsi que cela est montré dans l'exemple 22 plus loin. En étant portés par les dendrimères, ces produits marqueurs sont devenus solubles dans les milieux physiologiques. La toxicité de ces produits marqueurs (lorsqu'ils sont employés seuls) est masquée aux organismes par les dendrimères. Ces produits marqueurs peuvent alors être suivis par absorption UV, par fluorescence, par RX ou par imagerie par résonance magnétique (IRM) selon la nature du produit de marquage.

Le procédé de préparation de polylysine dendrimère greffé selon l'invention présente de nombreux avantages.

Un premier avantage est que dans un solvant aqueux le monomère activé (TFA-L-Lys-NCA) est utilisable brut de réaction, c'est-à-dire sans aucune purification préalable, contrairement aux recommandations de la littérature [21]. Les acides chlorhydrique ou nitreux, formés lors de la synthèse des NCA [25, 29, 32] ne sont, en solvant aqueux, absolument pas gênants quel que soit le pH compris entre 3 et 9 auquel est conduite la polymérisation.

Un deuxième avantage est que les réactions de polymérisation selon l'invention sont rapides. Elles sont totales en un temps compris entre 2 heures et 30 min, fonction de la température comprise entre -10 et +60 °C, et du pH compris entre 3 et 9.

Un troisième avantage est que les polypeptides formés (quelles que soient les générations) précipitent en solvant aqueux et sont facilement récupérables par filtration ou centrifugation. Cet avantage est augmenté du fait que les peptides qui précipitent ont un faible indice de polydispersité. En effet, les polymères d'aminoacides courts sont solubles, ce qui autorise leur allongement, mais précipitent au delà d'une certaine longueur ce qui bloque leur allongement. La conséquence est que les produits obtenus ont une courbe de distribution étroite, dont la masse moyenne dépend notamment de la balance hydrophile-hydrophobe entre le porteur initial (amorceur) et le produit final. Cette balance est également fonction de la nature du groupement utilisé pour protéger la fonction amine en $\varepsilon$.

Un quatrième avantage du procédé de l'invention est qu'en milieu aqueux on peut amorcer les réactions de polymérisation dans un milieu acide contrairement aux procédés antérieurs qui nécessitent des solvants anhydres. Ainsi, la première réaction de polymérisation est amorcée soit par la TFA-L-Lys provenant de l'hydrolyse des NCA, soit par la TFA-L-Lys ajoutée au milieu réactionnel, de telle sorte qu'après déblocage, la polylysine dendrimère de génération 1 (P1) est strictement constituée de lysine. Cette pureté qualitative (biologiquement intéressante) se retrouve obligatoirement dans les générations suivantes puisque P1 est amorceur pour la polylysine dendrimère greffé de génération 2 (P2), lequel est amorceur pour la polylysine dendrimère greffé de génération 3 (P3) etc...

Cette possibilité n'interdit pas d'amorcer la polymérisation par des amines autres que les précédentes telles que par exemple des aminoacides, des peptides, des alkylamines, des alkyldiamines, des arylamines.

Un cinquième avantage du procédé de l'invention est qu'il est aisé de contrôler, et de programmer les masses moyennes en nombre (Mn) des polylysines dendrimères greffés de la génération i attendue (Pi). Dans la réaction de formation des polylysines dendrimères greffés, le choix du rapport {TFA-L-Lys-NCA} / amorceur, est en effet important. Pour un rapport *ad hoc* la polylysine dendrimère greffé obtenue est monomodale, de faible polydispersité et de Mn donnée. Mais si l'on utilise un excès de monomère activé la Mn moyenne du Pi obtenu sera plus élevée que lorsqu'on utilise le rapport *ad hoc.* Un trop fort excès de monomère activé conduit, à coté du Pi attendu, à la formation de traces de P1 que l'on élimine par microfiltration. Cette propriété permet ainsi d'obtenir pour chaque génération, des polylysines dendrimères greffés de masses moyennes en nombre contrôlables à plus de 30 % des masses moyennes *ad hoc*. On peut en conséquence en partant d'une génération P1 de Mn 1 450 Da, obtenir des polylysines dendrimères greffés de masses variables comprises entre les minima et maxima suivants :

6 000 Da < Mn P2 < 14 000 Da, 15 000 Da < Mn P3 < 30 000 Da, 50 000 Da < Mn P4 < 80 000 Da, 140 000 Da < Mn P5 < 200 000 Da.

De préférence :

Mn P2 < 12 000 Da, Mn P3 < 28 600 Da, Mn P4 < 84 000 Da, Mn P5 < 224 000 Da.

En variant la nature du groupement protecteur de la fonction amine en epsilon, on obtient selon l'hydrophobie de ce groupement, des composés de P1 à Pi de Mn, plus faible si le groupement protecteur est plus hydrophobe, ou plus élevé si le groupement protecteur est plus hydrophile. Ce procédé permet donc de synthétiser très facilement des polylysines dendrimères greffés ayant les masses moyennes (Mn) souhaitées.

Les polylysines dendrimère greffé de l'invention ont des masses molaires qui augmentent plus vite en fonction du nombre de génération que pour des dendrimères de lysine de l'art antérieur en raison du fait que l'amorceur que l'on peut utiliser pour la génération 2 est une polylysine linéaire. Sur cet aspect, si les polylysines dendrimères greffés de l'invention peuvent être comparées aux polylysines branchées décrites par Klok et al [3], elles diffèrent structurellement de ces dernières par leur taux de greffage beaucoup plus élevé.

C'est pour ces raisons que les polylysines dendrimères de l'invention sont nommées Dendrimères-Greffés de Lysine (DGL).

La présente invention concerne également une polylysine dendrimère greffé telle que pouvant être obtenue par la mise en oeuvre d'un procédé de préparation, tel que défini ci-dessus, notamment par la mise en oeuvre d'un procédé de préparation d'une homo ou hétéropolylysine dendrimère greffé de génération n, où n est supérieur à 1, tel que défini ci-dessus.

Dans un mode de réalisation particulier, le dendrimère greffé de lysine (ou polylysine dendrimère greffé) tel que défini ci-dessus, est **caractérisé en ce que** les groupements $-NH_2$ externes sont totalement ou partiellement liés de manière covalente ou non-covalente, à des groupes choisis parmi la liste comprenant des oses, des acides nucléiques, des protéines, ou des groupements portant des fonctions carboxyliques, sulfoniques, phosphoriques, des polyoxydes d'éthylène, des chaînes hydrocarbonées ou perfluorohydrocarbonées, des aldéhydes ou leur précurseur, ou des fonctions réactives masquées (isocyanates) telles que des groupements carbamoyle ou chloroéthylnitrosourées.

Ces modifications de fonctionnalisation de leur surface leur confèrent de nouvelles propriétés physiques, et au delà de nouvelles activités biologiques.

Dans un autre mode de réalisation particulier, les dendrimères greffés de lysine (ou polylysines dendrimères

greffés) tels que définis ci-dessus sont **caractérisés en ce qu**'ils peuvent être fixés sur un support de manière covalente ou non-covalente, notamment par liaisons électrostatiques, permettant au support de conserver ses propriétés.

Un premier avantage est qu'en conservant leurs propriétés, lesdits supports (particules, fibres, ou surfaces) acquièrent les propriétés des dendrimères. La mise en oeuvre de ces supports permet alors de les utiliser comme filtres, tissus, revêtement de surface, particules abrasives, dans des environnements divers.

Le deuxième avantage est lié au fait qu'en fixant sur support les dendrimères greffés de la présente invention, on crée un grand nombre de fonctions réactives (amines ou acides) par unité de surface de ces supports et on modifie ainsi leur propriété. On tire alors partie de cette modification pour, par exemple fixer sur lesdits supports des concentrations d'anticorps, d'aptamères ou des fragments d'ADN supérieures à celles qu'il est possible de fixer sur les plaques habituellement utilisées pour les dosages immunologiques, ce qui permet d'augmenter la sensibilité de ces dosages à partir de ces récepteurs hautement spécifiques.

Un troisième avantage est que la polylysine dendrimère greffé ainsi fixée conserve ses propriétés par rapport à son état non-fixé.

Par support nous entendons les matériaux inorganiques (silice, alumine, oxydes métalliques,) ou organiques (polyacryliques, polyuréthanes, polyépoxy, polyisocyanate, polysaccharides, polyamides).

Selon un mode de réalisation préféré, les polylysines dendrimères greffés de l'invention sont furtives vis-à-vis des systèmes immunitaires vis-à-vis desquels elles sont mises en présence, et peuvent avantageusement être utilisées comme porteurs d'haptènes ou d'antigènes contre lesquels les systèmes immunitaires réagissent pour former des anticorps.

La présente invention concerne également l'utilisation des polylysines dendrimères greffés définies ci-dessus, pour la préparation de complexes antigène - polylysine dendrimère greffé ou de complexes haptène - polylysine dendrimère greffé, destinés à la production d'anticorps dirigés contre ledit antigène ou ledit haptène.

En effet, les dendrimères greffés de lysine selon l'invention sont **caractérisés en ce qu**'ils sont furtifs vis-à-vis des systèmes immunitaires, c'est-à-dire que les systèmes immunitaires ne produisent pas d'anticorps dirigés contre les dendrimères greffés de lysine. Toutefois, si les dendrimères greffés de lysine selon l'invention portent un haptène, ou un antigène fixé à leur surface, les systèmes immunitaires peuvent produire des anticorps contre ces haptènes ou antigènes sans en créer contre le porteur dendrimère greffé de lysine, contrairement à ce qui se produit lorsque les porteurs sont des protéines telles que la BSA (sérum albumine bovine), HSA (sérum albumine humaine), KLH (hémocyanine de patelle).

L'avantage est que les anticorps ainsi produits spécifiquement contre les antigènes et les haptènes ne sont pas mélangés à des anticorps spécifiques du porteur contrairement à ce qui se passe lorsqu'ils sont produits par les techniques habituelles, et plus directement utilisables en tant qu'outils analytiques ultérieurs.

Par haptène on entend une molécule de faible masse molaire non antigénique par elle-même.

La présente invention concerne également une composition de dendrimères greffés de lysine (ou polylysines dendrimère greffé) telle que pouvant être obtenue par la mise en oeuvre d'un procédé de préparation tel que défini ci-dessus.

La présente invention concerne également un dendrimère greffé de lysine (ou polylysine dendrimère greffé), tel que défini ci-dessus, en tant qu'antibactérien ou antifongique, sous réserve qu'il ne soit pas utilisé pour le traitement thérapeutique du corps humain ou animal.

En particulier en phase homogène, la polylysine dendrimère greffé (ou dendrimère greffé de lysine) selon l'invention est utilisée pour le traitement de surfaces dans le cadre d'un traitement antiseptique ou pour prévenir des risques de contamination microbiologiques de ces surfaces.

Les dendrimères greffés de lysine peuvent également être utilisés pour le traitement de réservoirs de liquides à risque de contamination microbiologique, tels que des réserves d'eau par exemple.

Les polylysines dendrimères greffés de l'invention peuvent être utilisées, fixées sur support comme défini ci-dessus, comme filtres et revêtement antimicrobien, comme particules antimicrobiennes circulantes pour lutter contre les biofilms, comme revêtement anti-biofilm, notamment pour protéger les coques de bateau du phénomène de fouling.

La présente invention concerne également une composition pharmaceutique, **caractérisée en ce qu**'elle comprend, à titre de substance active au moins une polylysine dendrimère greffé (ou dendrimère greffé de lysine) telle que définie ci-dessus, en association avec un véhicule pharmaceutiquement acceptable.

La présente invention concerne également l'utilisation d'une polylysine dendrimère greffé (ou dendrimère greffé de lysine) telle que définie ci-dessus, pour la préparation d'un médicament destiné au traitement des infections bactériennes ou fongiques, ou des cancers.

Dans un mode de réalisation particulier de l'utilisation telle que définie ci-dessus d'une polylysine dendrimère greffé (ou dendrimère greffé de lysine) pour la préparation d'un médicament les infections bactériennes sont choisies parmi les infections à bactéries GRAM- et GRAM+ appartenant notamment aux familles choisies dans la liste comprenant les *Pseudomonadaceae*, en particulier du genre *Pseudomonas*, les *Legionellaceae*, en particulier du genre *Legionella*, les *Enterabacteriaceae*, en particulier des genres *Escherichia*, *Salmonella*, *Shigella*, et *Yersinia*, les *Vibrionaceae*, les

*Pasteurellaceae*, les *Alcaligenaceae*, en particulier du genre *Bordetella*, les *Brucellaceae*, en particulier du genre *Brucella*, les *Francisellaceae*, en particulier du genre *Francisella*, les *Neisseriaceae*, les *Micrococcaceae,* en particulier des genre *Staphylococcus*, *Streptoccus*, et *Listeria.*

## DESCRIPTION DES FIGURES

### Figure 1

Chromatogrammes obtenus pour les différentes générations de polylysines dendrimères de l'invention (P1 à P5) en CES (Chromatographie d'Exclusion Stérique) couplée à la diffusion statique de lumière (LS). Conditions expérimentales : colonne Superose 12 (Amersham Pharmacia Biotech) ; débit 0,4 mL/min, température : 25°C, éluant 1, concentrations injectées : 5g/L, volume d'injection: 100 μL. Les chromatogrammes présentent le signal d'indice de réfraction (en ordonnée) en fonction du volume d'élution (en mL, en abscisse).

### Figure 2A et Figure 2B

Les Figures 2A et 2B représentent la variation de la masse molaire moyenne en nombre (en g/mol, en ordonnées) des polylysines dendrimères de l'invention (losanges) en fonction de la génération (en abscisse) et la comparaison avec les masses molaires théoriques des dendrimères monofonctionnels (carrés) et difonctionnels (triangles) décrits par H.A. Klok et al.

Les masses molaires des DGL ont été déterminées par CES couplée à la diffusion de la lumière ($P_2$ à $P_5$) et par électrophorèse capillaire ($P_1$). Les masses molaires des dendrimères de polylysine ont été calculées en supposant que la structure dendritique est parfaite (toutes les fonctions amines des monomères lysine sont supposées réagir). Le coeur du dendrimère monofonctionnel correspond à la benzhydrylamine. Le coeur du dendrimère difonctionnel correspond au 1,4-diaminobutane.

Les équations obtenues par ajustement non linéaire des données expérimentales selon une loi exponentielle sont présentées dans les figures avec les coefficients de corrélation $R^2$.

### Figure 3

La Figure 3 représente le rayon hydrodynamique des dendrimères greffés de lysine de l'invention (en nm, en ordonnées) en fonction de la génération (en abscisse) déterminé à l'aide de trois méthodes expérimentales différentes : la TDA (Analyse par diffusion de Taylor, carrés), la DLS (Diffusion de lumière quasi-élastique, losanges), et la CES-3D (Chromatographie d'Exclusion Stérique triple détection, diffusion de lumière, détecteur viscosimétrique et indice de réfraction, triangles).

### Figure 4

La Figure 4 représente le rayon hydrodynamique des dendrimères greffés de lysine de l'invention (en nm, en ordonnées) en fonction de la génération (en abscisse) déterminé par TDA dans les trois tampons étudiés dans les exemples, tampon 1 (losanges), tampon 2 (carrés), tampon 3 (triangles).

### Figure 5

La Figure 5 représente le rayon hydrodynamique (en nm) des DGL en fonction de la masse molaire (en g/mol), en échelle bi-logarithmique. Les droites représentent les ajustements linéaires correspondant à la relation $R_h \sim M^{1/\nu}$. Les valeurs de $\nu$ sont égales à : 2,72 ± 0,22 (tampon 1)(carrés) ; 2,80 ± 0,09 (tampon 2)(ronds) ; 2,74 ± 0,08 (tampon 3) (triangles).

### Figures 6A, 6B, 6C, 6D, 6E, 6F, 6G, 6H et 6I

Les figure 6A, 6B, 6C et 6D représentent les spectres de $^1H$ RMN à 400 MHz dans $D_2O$ à 25°C entre 3,7 et 4,4 ppm, des tri- (Figure 6D) et pentalysines linéaires (Figure 6C), ainsi que ceux des dendrimères greffés de lysine de l'invention (générations 1 à 5 (P1 à P5)(Figures 6A et 6B).

La Figure 6H représente la formule chimique de la trilysine ; la Figure 6G représente la formule chimique de la pentalysine ; la Figure 6F représente la formule chimique du P1 et la Figure 6E représente la formule chimique du P2. La région spectrale représentée correspond au proton porté par le carbone chiral des L-lysines. Selon son environnement, ce proton résonne à des champs différents. La Figure 6I représente les 4 environnements différents (Ha, Hb,

Hc, Hd) observés dans ces différents produits. Les protons Ha, Hb, Hc et Hd portés par les carbones chiraux sont respectivement représentés par un cercle blanc, un cercle noir, l'absence de cercle et un cercle gris. Ha correspond au proton chiral d'une lysine de coeur liée à sa voisine par une liaison peptidique. Hc correspond au proton chiral d'une lysine N terminale. Hd correspond au proton chiral d'une lysine C terminale. Hb correspond au proton chiral d'une lysine de coeur du dendrimère liée à la chaîne latérale $NH_2$ d'une lysine voisine. Cette liaison est de type amidique et non peptidique.

Dans le spectre de la trilysine (Figure 6D), on note la présence de trois signaux différents Ha, Hc et Hd d'égale intensité. Dans le spectre de la pentalysine (Figure 6C), l'intensité de Ha est trois fois supérieure (3 lysines de coeur) à celle de Hc ou Hd qui sont d'égale intensité. Par comparaison, dans le spectre du composé P1 (Figure 6B), l'intensité du signal Ha est d'environ 7 fois supérieure à celle des signaux Hc ou Hd qui sont eux d'intensité égale, en conséquence le composé P1 est une polylysine linéaire de longueur moyenne égale à 9 lysines (7 de coeur et 2 terminales). Les signaux Hb, caractérisant les liaisons amidiques, ne sont observables que sur les spectres des composés P2, P3, P4, P5. Dans chacun de ces spectres, l'intensité du signal Hb est proportionnelle au nombre de lysines liées aux chaînes latérales des lysines voisines ($\varepsilon NH_2$) par des liaisons amidiques. Ha est proportionnel au nombre de lysines liées à leur voisine en $\alpha$-$NH_2$ par une liaison peptidique (à l'exception des résidus N-terminaux et C-terminaux). Le rapport Hb/Ha est ainsi lié au nombre de chaînes latérales ($\varepsilon NH_2$) qui dans une génération donnée ont réagi sur les NCA pour conduire à la génération suivante.

Le rapport Hb/Ha permet de déterminer le taux de greffage (ou de branchement) d'une polylysine de génération (n). Ce taux de greffage est le pourcentage des $\varepsilon NH_2$ d'une génération (n) qui ont réagi sur des NCA pour donner la génération (n+1).

Ce taux de greffage (**Tableau 6**) est de 100 % pour le passage de P1 à P2 (Hb/Ha=0,25), ce qui signifie que les 8 chaînes latérales du P1 ont réagi sur les NCA pour donner P2, de 81 % pour le passage de P2 à P3 (Hb/Ha=0,60), ce qui signifie que sur 48 chaînes latérales du P2, 39 ont réagi sur les NCA pour donner P3, de 80 % pour le passage de P3 à P4 (Hb/Ha=0,46), de 65 % pour le passage de P4 à P5 (Hb/Ha=0,40), et de 64% pour le passage de P5 à P6 (Hb/Ha=0,42) (non montré sur la figure).

**Figure 7**

Représentation schématique des dendrimères greffés de lysine de la génération 1 (P1) à la génération 5 (P5).

Définition de la structure des Pn : P1, a = 8 (DPn = 8) ; P2, structure du P1 et $\sum_{i=1}^{2} b_i = 40$ (DPn = 48) ; P3, structure du P2 et $\sum_{i=1}^{4} c_i = 98$ (DPn = 123) ; P4, structure du P3 et $\sum_{i=1}^{8} d_i = 237$ (DPn = 365) ; P5, structure du P4 et $\sum_{i=1}^{16} e_i = 614$ (DPn = 963).

**Figure 8**

La figure 8 représente l'électrophérogramme montrant la séparation du $P_1$ déprotégé (poly-L-lysine linéaire) dans les conditions expérimentales suivantes : Capillaire en silice vierge, 60 cm (50 cm à la fenêtre de détection) $\times$ 50 $\mu$m. Electrolyte : tampon phosphate (125 mM $H_3PO_4$ 125mM $Na^+H_2PO_4^-$ pH=2,16) avec 5% de dextrane. Tension appliquée : +20kV. Détection UV à 200 nm. Concentration de l'échantillon : 3g/l. Injection : 2 psi pendant 9 secondes. Cette Figure représente l'absorbance en mAU en fonction du temps en minutes.

Cet électrophérogramme comparé à celui obtenu en analysant, dans les mêmes conditions, les di-, tri-, tétra- et penta-lysines linéaires (peptides commerciaux obtenus chez Sigma-Aldrich) confirme que le composé (P1 déprotégé) est un mélange d'oligolysines linéaires de longueurs comprises entre 3 et environ 20 résidus lysines. A partir de cet électrophérogramme, il a été possible de déterminer le degré de polymérisation moyen en nombre ($DP_n$=8) et l'indice de polymolécularité (I=1,2). Les degrés de polymérisation moyens en nombre $DP_n$ et en poids $DP_w$ ont été calculés à partir des relations suivantes :

$$DP_n = \frac{\sum_{i=1}^{\infty} i\,N_i}{\sum_{i=1}^{\infty} N_i} \quad \text{et} \quad DP_w = \frac{\sum_{i=1}^{\infty} i^2\,N_i}{\sum_{i=1}^{\infty} i\,N_i}$$

où Ni est le nombre de macromolécules ayant un degré de polymérisation i. Dans le cas d'une détection UV où l'absorption est due aux monomères, la réponse du détecteur est proportionnelle à la concentration massique du polymère. $DP_n$ et $DP_w$ peuvent alors s'exprimer en fonction de la hauteur $h_i$ (ou bien, dans le cas où les pics sont intégrables, en fonction de l'aire corrigée par le temps de migration, $A_i$) de chaque pic électrophorétique correspondant au polymère de degré de polymérisation i :

$$DP_n = \frac{\sum_{i=1}^{\infty} h_i}{\sum_{i=1}^{\infty} \frac{h_i}{DP_i}} = \frac{\sum_{i=1}^{\infty} A_i}{\sum_{i=1}^{\infty} \frac{A_i}{DP_i}} \quad \text{et} \quad DP_w = \frac{\sum_{i=1}^{\infty} i\,h_i}{\sum_{i=1}^{\infty} h_i} = \frac{\sum_{i=1}^{\infty} i\,A_i}{\sum_{i=1}^{\infty} A_i}$$

**Figures 9A et 9B**

Les Figures 9A et 9B représentent les spectres de masse (MALDI-TOF) effectués sur un appareil Applied Biosystems Voyager DE-STR en mode reflectron et linéaire. La longueur du tube de vol est de 2 m (linéaire) ou 3 m (reflectron) et le laser a une longueur d'onde $\lambda$=337 nm (laser $N_2$). La matrice est l'acide $\alpha$-cyano-4-hydroxycinnamique (CHCA) à 10 $g.L^{-1}$ dans le DMF pour le P1 TFA protégé en $\varepsilon$ et dans le $CH_3CN/H_2O$ pour le P1 déprotégé. L'échantillon est préparé selon une proportion de 10/1 (v/v) matrice/échantillon et déposé par la suite (1 $\mu$l) sur la cible. Le spectre est obtenu par accumulation de 300 tirs laser et la tension d'accélération fixée à 20kV.

La Figure 9A correspond à P1 TFA protégé lavé à 0°C par de l'eau pure et la Figure 9B correspond à P1 déprotégé, lavé à 0°C 4 fois par une solution de bicarbonate d'ammonium. Ces Figures représentent le pourcentage d'intensité des pics en fonction de la masse m/z.

Ces spectres, parfaitement reproductibles, des composés P1 TFA protégés et P1 déprotégés montrent que lorsque la polymérisation de la $N^{\varepsilon}$-TFA-L-Lysine NCA est conduite dans l'eau à pH 6,5, sans amorceur, on obtient une polylysine linéaire (P1) sans produits secondaires apparents, et "vivante", c'est-à-dire avec une fonction $-CO_2H$ sur le résidu C-terminal, et une fonction $-NH_2$ sur le résidu N-terminal.

**Figure 10**

La Figure 10 est le spectre de masse MALDI-TOF du produit de la réaction de polymérisation de la $N^{\varepsilon}$-TFA-L-Lysine NCA dans l'eau à pH 6,5, en présence d'aminofluorescéine. Le précipité obtenu dans cette réaction à été lavé 4 fois par une solution de $HCO_3Na$ 0,1 N, traité par une solution ammoniacale, et lyophilisé. Le spectre de masse du lyophilisat, montre que le produit obtenu est une fluorescéine amide ($^+H_2(Lys)_n$-$NHC_{20}H_{11}O_5$) portant un nombre de lysine n compris entre 2 et 20. Pour une $\lambda$ excitation à 490 nm, ce matériau fluoresce dans une solution de bicarbonate de sodium 0,1 M à pH 8,5 avec une $\lambda$ maximale à 517 nm et un seuil de détectabilité inférieur à $10^{-3}$ $mg.L^{-1}$. Ce $\lambda$ max d'émission est à comparer à celui de l'aminofluorescéine libre (513 nm) dans les mêmes conditions.

**Figure 11**

La Figure 11 représente l'évolution pondérale moyenne (en g, en ordonnées) en fonction du temps (en jours, en abscisse) dans un groupe de rats mâles Sprague-Dawley (n=6) recevant les 4 premiers jours 360 mg de dendrimère greffé de lysine (DGL) P3 et chaque jour suivant pendant 180 jours 30 mg de DGL P3 de l'invention (courbe noire). Les rats contrôles (n=100) reçoivent 0,5 ml de sérum physiologique (solvant de la solution)(courbe grise).

**Figure 12**

La Figure 12 représente le pourcentage de survie (en ordonnées) par rapport à un témoin (losanges) de

bactéries *Legionella pneumophila* mises en contact avec des polylysines dendrimère greffé de l'invention de génération 2 à 10 mg/L (cercles), de génération 2 à 100 mg/L (triangles), de génération 3 à 10 mg/L (carrés), et de génération 3 à 100 mg/L (étoiles) en fonction du temps (en jours, en abscisse).

**Figure 13**

ELISA sur plaque maxisorp à différentes dilutions. LOT A J65 sur plaque coatée au GDL-haptene (cercles noirs) ou DGL natif (cercles blancs), LOT B sur plaque cotée au DGL-haptène (triangles noirs) et LOT C sur plaque coatée au DGL natif (croix). L'absorbance (DO) est représentée en fonction du logarithme de la dilution du sérum.

**Figure 14**

ELISA Covalink. LOT A J21 (triangles noirs), LOT A J36 (carrés noirs), LOT B (tirets). L'absorbance (DO) est représentée en fonction du logarithme de la dilution du sérum.

**Figure 15**

ELISA sur plaques Maxisorp à différentes dilutions de sérums pour le LOT A à J65 sur plaque coatée à la BSA-haptène (carrés noirs) ou BSA native (croix). L'absorbance (DO) est représentée en fonction du logarithme de la dilution du sérum.

## EXEMPLES

### EXEMPLE 1

**Polylysine dendrimère de génération 1 (P1)**

1/ On synthétise 94 g de $N^{\varepsilon}$-TFA-L-Lysine-NCA par une des méthodes de l'art, notamment telle que décrite ou rappelée par Collet et al [29] par exemple.
2/ Au produit brut de la réaction ci-dessus on ajoute 1 litre d'une solution aqueuse de $HCO_3Na$ 0,1 M maintenue à 0°C.
3/ Après 30 mn on récupère par filtration ou centrifugation le précipité blanc de Poly-$N^{\varepsilon}$-TFA-L-Lysine linéaire, formé dans le milieu.
4/ Ce précipité, placé dans 1 litre de solution aqueuse ammoniacale à pH 11 pendant 15 heures à 40 °C, perd les groupements protecteurs TFA de ses chaînes latérales et devient soluble dans l'eau.
5/ Le volume du solvant est réduit de moitié dans un évaporateur rotatif.
6/ Après lyophilisation on obtient 54 g d'une poly-L-Lysine linéaire de polydispersité 1,2 (mesurée en EC (Electrophorèse Capillaire, voir **Exemple 7**)). Sa masse moyenne absolue, mesurée par electrophorèse capillaire (EC) et confirmée par MALDI-TOF et RMN, est de 1 450 Da.

### EXEMPLE 2

**Dendrimère Greffé de Lysine (DGL) de génération 2 (P2)**

1 / On synthétise 94 g de monomère $N^{\varepsilon}$-TFA-L-Lysine-NCA comme précédemment.
2/ Au produit brut de la réaction on ajoute 1 litre d'une solution aqueuse 0,1 M de $HCO_3Na$ maintenue à 0°C, dans laquelle on a dissout 30 g du produit de la génération 1 de l'**Exemple 1** en tant qu'amorceur de polymérisation.
3/ Après 30 min, on récupère par filtration ou centrifugation le précipité blanc de Poly-$N^{\varepsilon}$-TFA-L-Lysine greffé, formé dans le milieu.
4/ Ce précipité, placé dans 1 litre de solution aqueuse ammoniacale à pH 11 pendant 15 heures à 40 °C, perd les groupements protecteurs TFA des chaînes latérales des motifs ajoutés lors de la polymérisation et devient soluble dans l'eau.
5/ Le volume du solvant est réduit de moitié dans un évaporateur rotatif.
6/ Après lyophilisation on obtient 70 g de poly-L-Lysine dendrimère greffé de polydispersité 1,40 (mesurée en CES). Sa masse moyenne mesurée par LS est de 8 600 Da.

En faisant varier le rapport en masse monomère / amorceur de 3,9 à 2,6 on obtient des produits de génération 2 dont les masses varient entre 8 600 Da et 12 000 Da.

**EXEMPLE 3**

**Dendrimère Greffé de Lysine (DGL) de génération 3 (P3)**

1/ On synthétise 94 g de $N^{\varepsilon}$-TFA-L-Lysine-NCA comme précédemment.
2/ Au produit brut de la réaction on ajoute 1 litre d'une solution aqueuse de $HCO_3Na$ 0,1 M maintenue à 0˚C, dans laquelle on a dissout 30 g du produit de la génération 2 de l'**Exemple 2.**
3/ Après 30 min, on récupère par filtration ou centrifugation le précipité blanc de Poly-$N^{\varepsilon}$-TFA-L-Lysine dendrimère greffé, formé dans le milieu.
4/ Ce précipité, placé dans 1 litre de solution aqueuse ammoniacale à pH 11 pendant 15 heures à 40 ˚C, perd les groupements protecteurs TFA des chaînes latérales des motifs ajoutés lors de la polymérisation et devient soluble dans l'eau.
5/ Le volume du solvant est réduit de moitié dans un évaporateur rotatif.
6/ Après lyophilisation on obtient 70 g d'une poly-L-Lysine dendrimère greffé de polydispersité 1,46 (mesurée en CES). Sa masse moyenne mesurée par LS est de 22 000 Da.

En faisant varier le rapport monomère / amorceur de 3,9 à 2,6 on obtient des produits de génération 3 dont les masses varient entre 22 000 Da et 28 600 Da.

**EXEMPLE 4**

**Dendrimère Greffé de Lysine (DGL) de génération 4 (P4)**

1/ On synthétise 94g de $N^{\varepsilon}$-TFA-L-Lysine-NCA comme précédemment.
2/ Au produit brut de la réaction on ajoute 1 litre d'une solution aqueuse de $HCO_3Na$ 0,1 M maintenue à 0˚C, dans laquelle on a dissout 30 g du produit de la génération 3 de l'**Exemple 3.**
3/ Après 30 mn on récupère par filtration ou centrifugation le précipité blanc de Poly-$N^{\varepsilon}$-TFA-L-Lysine greffé, formé dans le milieu.
4/ Ce précipité, placé dans 1 litre de solution aqueuse ammoniacale à pH 11 pendant 15 heures à 40˚C, perd les groupements protecteurs TFA des chaînes latérales des motifs ajoutés lors de la polymérisation et devient soluble dans l'eau.
5/ Le volume du solvant est réduit de moitié dans un évaporateur rotatif.
6/ Après lyophilisation on obtient 70 g d'une poly-L-Lysine dendrimère greffé de polydispersité 1,36 (mesurée en CES) et de masse moyenne 65 300 Da (mesurée par LS).

En faisant varier le rapport monomère / amorceur de 3,9 à 2,6 on obtient des produits de génération 3 dont les masses varient entre 65 300 Da et 85 000 Da.

**EXEMPLE 5**

**Dendrimère Greffé (DGL) de génération 5 (P5)**

1/ On synthétise 94g de $N^{\varepsilon}$-TFA-L-Lysine-NCA comme précédemment.
2/ Au produit brut de la réaction on ajoute 1 litre d'une solution aqueuse 0,1 M de $HCO_3Na$ maintenue à 0˚C, dans laquelle on a dissout 30 g du produit de la génération 4 de **l'Exemple 4.**
3/ Après 30 min, on récupère par filtration ou centrifugation le précipité blanc de Poly-$N^{\varepsilon}$-TFA-L-Lysine greffé de génération 5, formé dans le milieu.
4/ Ce précipité, placé dans 1 litre de solution aqueuse ammoniacale à pH 11 pendant 15 heures à 40 ˚C, perd les groupements protecteurs TFA des chaînes latérales des motifs ajoutés lors de la polymérisation et devient soluble

dans l'eau.

5/ Le volume du solvant est réduit de moitié dans un évaporateur rotatif.

6/ Après lyophilisation on obtient 70 g d'une Poly-L-Lysine dendrimère greffée de polydispersité 1,46 (mesurée en CES). Sa masse moyenne mesurée par LS est de 172 300 Da.

En faisant varier le rapport monomère / amorceur de 3,9 à 2,6, on obtient des produits de génération 3 dont les masses varient entre 172 300 Da et 224 000 Da.

**EXEMPLE 6**

**Dendrimères Greffés de lysine (DGL) de générations 6, 7 et 8 (P6, P7 et P8)**

Cette synthèse a été répétée pour les générations suivantes 6, 7, 8. Leurs masses (Mn) évaluées par extrapolation sont dans l'ordre égales à 430 000 Da, 1 130 000 Da, et 3 000 000 Da.

**EXEMPLE 7**

**Détermination des masses molaires moyennes et des indices de polydispersité**

La chromatographie d'exclusion stérique couplée à la diffusion statique de lumière multi-angle et à un détecteur d'indice de réfraction différentiel (CES-2D) a permis d'obtenir la distribution en masse molaire des 4 générations de DGL (P2-P5). La distribution en masse molaire du coeur P1 linéaire a été obtenue par EC (confirmé par MALDI-TOF et RMN). Les mesures ont été réalisées dans un éluant aqueux constitué de 50 g/L de $H_2PO_4^-Na^+$ (éluant 1, pour la définition des éluants, voir le **Tableau 2**) à pH 4,5. Les chromatogrammes obtenus sont présentés **Figure 1.** Bien qu'il ait été constaté que le stockage des DGL à l'abri de l'humidité permettait de diminuer la formation d'agrégats, ce phénomène n'a cependant pas pu être complètement évité. Ainsi, la **Figure 1** montre malgré ces précautions la formation d'agrégats (épaulements aux faibles volumes d'élution), notamment pour les P4 et P5. Les résultats de masses molaires moyennes sont regroupés dans le **Tableau 3**, avec les valeurs des incréments d'indice de réfraction (dn/dC) et les degrés de polymérisation estimés. Il convient de noter que, bien que cette méthode permette une détermination des masses molaires absolues, les valeurs de masses molaires données doivent prendre en compte une certaine proportion de contre-ions (ions phosphates $H_2PO_4^-$ dans l'éluant). Cette proportion, bien qu'elle soit difficile à évaluer expérimentalement, peut être estimée par la théorie de la condensation décrite par Manning. Dans le cadre de cette théorie, le taux de condensation est donné par le rapport entre la distance moyenne entre deux charges dans la polylysine (0,34 nm) et la longueur de Bjerrum (0,69 nm à 25°C). La masse molaire moyenne d'un monomère chargé dans un tampon phosphate pH 4,5 est donc de : $129\times0{,}49+227\times0{,}51=179$ g/mol. A partir de cette moyenne, il est possible d'estimer le nombre moyen de monomères N. Les résultats de masses molaires obtenus dans le tampon 2 par CES-3D (Viscotek) présentent des valeurs de masses molaires beaucoup plus élevées, notamment pour les P4 et P5, probablement à cause de la formation d'agrégats en quantité plus importante. Par la suite, les valeurs obtenues par CES-2D seront retenues.

La variation de la masse molaire des DGL est représentée sur les **Figures 2A-2B** en échelle linéaire **(Figure 2A)** ou en échelle semi-logarithmique **(Figure 2B**). A titre de comparaison, sont aussi représentées les masses molaires attendues (théoriques) pour des dendrimères de la polylysine amorcés, soit avec une monoamine (dendrimère 1, avec un coeur benzhydrylamine, [5, 33]),ou bien avec un amorceur diamine (dendrimère 2 amorcé avec un coeur 1,4-diaminobutane, [7]. Il est intéressant de noter que, dans tous les cas (y compris les DGL), la croissance de la masse molaire suit une loi exponentielle. Ainsi, la masse molaire entre deux générations successives est multipliée par un même facteur. Cependant dans le cas des DGL, le facteur multiplicateur est de 3,2 ($e^{1,162}$), contre 2,12 pour le dendrimère 2, et seulement 1,95 pour le dendrimère 1. Les masses molaires des DGL présentent, par conséquent, une loi de croissance exponentielle, caractéristique des dendrimères, cependant, l'augmentation de la masse molaire s'effectue pour les DGL de façon beaucoup plus rapide que pour les dendrimères de même nature.

On notera que les indices de polydispersité sont relativement faibles compris entre 1,2 (génération 1) et 1,46 (génération 5), ce qui démontre que le procédé de synthèse proposé permet facilement d'obtenir des composés de polydispersité contrôlée.

Les résultats du calcul du nombre moyen de monomères incorporés par amorceurs potentiels (fonction amine $\alpha$ ou $\varepsilon$) lors du passage d'une génération i à une génération i+1 sont donnés dans le **Tableau 4.** Ce résultat est cohérent avec les résultats de viscosité intrinsèque qui prévoit une densité minimale (viscosité intrinsèque maximale) pour un dendrimère de fonctionnalité 3 à la génération 4 (voir **Exemple 9).**

**EXEMPLE 8**

**Détermination des rayons hydrodynamiques (ou des coefficients de diffusion) des DGL**

Afin de connaître la dimension caractéristique des DGL en solution, les rayons hydrodynamiques ont été déterminés à partir des coefficients de diffusion moléculaire en utilisant la relation de Stoke-Einstein :

$$D_m = \frac{kT}{6\pi\eta R_h} \quad (1)$$

où η est la viscosité du milieu, k la constante de Boltzmann et T la température en K. Pour déterminer $D_m$, deux méthodes expérimentales ont été utilisées : la diffusion quasi-élastique (DLS) ou statique de la lumière (couplée ou non à une CES), et la méthode de diffusion de Taylor (TDA). Cette dernière méthode permet de mesurer le coefficient de diffusion d'une espèce à partir de l'élargissement d'une bande de soluté sous l'influence d'un flux hydrodynamique dont le profil de vitesse est connu pour être dispersif (profil parabolique de Poiseuille).

Les mesures par TDA ont été effectuées à l'aide d'un appareillage d'électrophorèse capillaire, en utilisant des capillaires en silice de 50 μm d.i. (60 cm × 50 cm jusqu'au détecteur) dont la surface interne a préalablement été modifiée par greffage d'un polycation (poly(diallyl-diméthyl-ammonium)) afin de limiter l'adsorption des solutés DGL à la surface du capillaire. La dispersion de la bande de soluté sous l'influence de l'écoulement de Poiseuille a été mesurée à l'aide d'un spectrophotomètre UV. Le coefficient de diffusion est par la suite déterminé selon la méthode initialement décrite par Taylor, puis reprise par Bello et al. [34]. En parallèle à la méthode TDA, les mesures par DLS ont été effectuée à l'aide d'un appareil zetasizer (Malvern). Enfin, des mesures de $R_h$ ont aussi pu être obtenues à partir de la CES-3D (Viscotek). L'ensemble des données numériques de $R_h$ est regroupé dans le **Tableau 3.**

La **Figure 3** présente l'augmentation des rayons hydrodynamiques des DGL en fonction de la génération, dans le tampon 2 et pour les trois méthodes expérimentales utilisées. Il apparaît que les valeurs obtenues par TDA sont inférieures à celles obtenues par DLS ou par CES-3D. Cette différence est probablement due à la présence d'agrégats dans l'échantillon qui, même en faible proportion, tend à augmenter les valeurs de $R_h$ obtenues par DLS. En effet, les agrégats de haut poids moléculaire représentent une contribution importante du signal de la lumière diffusée (qui varie avec la masse molaire à la puissance 6), ce qui entraîne une surévaluation de la valeur des $R_h$ correspondant aux unimères [35]. En CES-3D, la séparation partielle des agrégats par rapport aux unimères limite cet effet, ce qui peut expliquer que les valeurs en CES-3D se rapprochent plus des valeurs de TDA. En conclusion, les valeurs obtenues en TDA semblent être les valeurs les plus proches de la réalité, car la contribution des agrégats dans le cas d'une détection par absorbance UV est proportionnelle à leur concentration massique qui est relativement faible.

L'influence du pH du tampon (tampon 1 *vs* tampon 3), à force ionique à peu près constante (de l'ordre de 0,5 M), montre qu'à pH 7,0 les DGL ont un rayon hydrodynamique significativement plus faible qu'à pH 4,5 **(Figure 4).** Ce résultat est logique compte tenu de la déprotonation des fonctions amines qui doit commencer à être effective à pH 7,0, limitant ainsi les répulsions électrostatiques. Cet effet de pH devrait même être encore plus important à plus faible force ionique et/ou pour des valeurs de pH au-delà de 7. L'ajout de 3% d'acétonitrile dans le tampon 1 tend aussi à diminuer, mais de façon modérée, le rayon hydrodynamique. Cela pourrait s'expliquer par une diminution de la qualité du solvant.

En conclusion, les DGL ont un rayon hydrodynamique qui varie quasi-linéairement avec le nombre de générations. On retrouve, de nouveau, un comportement similaire à celui de dendrimères. Par ailleurs, l'augmentation du pH et l'ajout d'acétonitrile tendent à diminuer leurs rayons hydrodynamiques. On notera aussi que la méthode TDA semble être la méthode la plus adéquate (et la plus répétable avec des écart-types relatifs de l'ordre de 2-3% contre 10-20% pour la DLS) pour la mesure des $R_h$.

**EXEMPLE 9**

**Détermination de la viscosité intrinsèque**

La viscosité intrinsèque [η] est une grandeur inversement proportionnelle à la densité de la molécule dans le solvant. Cette grandeur dépend par conséquent de la masse moléculaire et du volume (ou du rayon hydrodynamique) de la molécule. Elle se détermine par des mesures de viscosité, en extrapolant à concentration nulle la viscosité réduite ou inhérente. La CES-3D a permis d'obtenir des valeurs de viscosité intrinsèques. Celles-ci sont données dans le **Tableau 3.** Il est intéressant de constater que [η] passe par un maximum pour la génération 4 comme la théorie le prévoit pour un dendrimère de fonctionnalité 3.

Une étude à partir des rayons hydrodynamiques et des volumes de Van der Waals ($V_w$) permet de montrer que la densité des DGL évolue de la même façon que pour un dendrimère de fonctionnalité 3. Le volume de Van der Waals représente le volume réellement occupé par les atomes (sans prendre en compte les phénomènes de solvatation). Il peut être calculé [36] par une somme d'incréments, chaque incrément correspondant au volume d'un atome (tabulé en fonction de la nature du ou des atomes voisins). Pour la polylysine de degré de polymérisation n, le volume de Van der Waals est donné par la relation suivante :

$$V_w\,(nm^3) = 0{,}1226 \times DP_n + 18{,}6\;10^{-3} \qquad (2)$$

Par la suite, on peut calculer la fraction de volume libre f selon l'équation :

$$f=1-(V_w/V_h) \qquad (3)$$

Le **Tableau 5** présente les valeurs de $V_w$, $V_h$ et f pour P1 et les 4 générations de DGL. On observe sur ces valeurs un maximum de f pour P4.

Une autre preuve du comportement dendritique des DGL est apportée par la Figure 5 qui représente la variation du rayon hydrodynamique des DGL en fonction de leur masse molaire en échelle bi-logarithmique. Les exposants obtenus lors de la linéarisation de ces données sont de l'ordre de 2,8, ce qui est en accord avec les valeurs obtenues pour des dendrimères trifonctionnels. Ces exposants sont de bons indicateurs du taux de branchement des structures dendritiques, sachant qu'une valeur de 3 correspond à une structure de ramification maximale.

**EXEMPLE 10**

**Analyse du taux de greffage des DGL**

On désigne par taux de greffage d'une polylylsine dendrimère de génération n (Pn), le pourcentage des fonctions $NH_2$ ε qui ont réagi par rapport au degré total de polymérisation de ce produit de génération n. (DPn). Les taux de greffage des PKDG de l'invention sont représentés dans le **Tableau 6** suivant.

**Tableau 6: Principales caractéristiques des DGL de l'invention, en particulier points de branchements et taux de greffage.**

| Poly-L-lysines de l'invention | Mn (g/mole) par EC pour P1, par CES-double détection pour P2 à P5, et par extrapolation pour P6 | DPn | Points de branchements déterminés par le rapport $^1H$ RMN (Hb/Ha) | Taux de greffage en % |
|---|---|---|---|---|
| P1 | 1 450 | 8 | (Coeur des matériaux de cette invention) Polylysine linéaire de 8 unités (Mn = 8) | |
| | | | 8 déterminé à partir du rapport Hb/Ha = 0,25 du P2 | 8/8 = 100% |
| P2 | 8 600 | 48 | 39 déterminé à partir du rapport Hb/Ha = 0,60 du P3 | 39/48 = 81% |
| P3 | 22000 | 123 | 98 déterminé à partir du rapport Hb/Ha = 0,46 du P4 | 98/123 = 80% |

(suite)

| Poly-L-lysines de l'invention | Mn (g/mole) par EC pour P1, par CES-double détection pour P2 à P5, et par extrapolation pour P6 | DPn | Points de branchements déterminés par le rapport $^1$H RMN (Hb/Ha) | Taux de greffage en % |
|---|---|---|---|---|
| P4 | 65 300 | 365 | 237 déterminé à partir du rapport Hb/Ha = 0,40 du P5 | 237/365 = 65% |
| P5 | 172000 | 963 | 614 déterminé à partir du rapport Hb/Ha = 0,42 du P6 | 614/963 = 64% |
| P6 | 439 800 | 2457 | / | / |

On obtient les taux de greffage donnés dans le **tableau 6** par une analyse progressive de la construction des dendrimères greffés en partant de la génération 1 à la génération n. Le taux de greffage de P1 à P2 se calcule à partir des valeurs expérimentales des degrés de polymérisation ; DP = 8 pour P1, DP = 48 pour P2 et du rapport expérimental Hb/Ha = 0,25 (**Figures 6A-6I**). A partir de ces valeurs, la variation de DP est de 40. Considérant que le nombre maximal de fonctions réactives est de 8 ε $NH_2$ et 1 α $NH_2$, que dans l'eau carbonatée et à pH 6,5 la réactivité des fonctions α $NH_2$ (pK =7,2) est seulement légèrement supérieure à celle des fonctions ε $NH_2$ (pK = 11,2), on en déduit que les 9 $NH_2$ du P1 incorporent 4,4 monomères en moyenne. Ainsi, dans le P2, le nombre maximum de Hb est de 8, celui de Ha de 30 (40 - 9 Hc + 1 Hd qui en RMN résonnent à des champs différents). Ces valeurs conduisent à un rapport Hb/Ha = 8/30 = 0,26 identique au rapport expérimental. Ceci indique que les 8 ε $NH_2$ du P1 ainsi que la fonction α-$NH_2$ du P1 ont réagi pour donner P2, ce qui montre que les 8 ε$NH_2$ sur la totalité des 8 ε$NH_2$ du P1 ont réagi pour donner le P2. Ainsi le taux de greffage du P1 est de 8/8 = 100%.

Après déblocage, P2 porte 40 ε$NH_2$ et 9 α$NH_2$. Le taux de greffage du P2 pour passer au P3 se calcule à partir des valeurs expérimentales de DP = 48 pour P2, DP = 123 pour P3, de la variation de DP = 75 et du rapport expérimental Hb/Ha = 0,60 (**Figures 6A-6I**).

Le seul moyen de satisfaire cette valeur expérimentale est d'admettre que le nombre de ε$NH_2$ réactifs du P2 est de 31 sur les 40 possibles (9 ε$NH_2$ sont donc non réactifs). Les 75 Lys (variation de DP) peuvent alors se répartir comme suit : 10 ε$NH_2$ s'allongent chacun d'une lysine, 21 ε$NH_2$ de deux lysines, 4 α$NH_2$ de deux lysines et 5 α$NH_2$ de trois lysines, ceci pour tenir compte de la réactivité des α$NH_2$ légèrement supérieure à celle des ε$NH_2$. Cette solution correspond à un nombre de Hb = 31 + 8 = 39, avec 64 Ha et 20 Hc + 1Hd, ce qui donne un rapport Hb/Ha = 39/64 = 0,60 égal au rapport expérimental: Ceci montre que 39 ε$NH_2$ sur la totalité des 48 ε$NH_2$ du P2 ont réagi pour donner le P3, et que le taux de greffage du P2 est de 39/48 = 81%.

Un raisonnement analogue s'appuyant sur les rapports expérimentaux Hb/Ha permet d'obtenir les taux de greffage des différents P3, P4, P5 indiqués dans le **tableau 6**.

Ces taux de greffages, montrent que les dendrimères greffés de lysines de la présente invention sont très différents, et notamment beaucoup plus denses que les polylysines branchées décrites par Klok et al [3] (voir le **tableau 1**). La haute densité de greffage des composés de l'invention, directement donnée par la réactivité des NCA sur les fonctions $NH_2$ α et ε dans l'eau bicarbonatée à pH environ 6,5 est une spécificité supplémentaire de la présente invention.

**Tableau 2 : Définition des tampons et éluants utilisés pour la détermination des grandeurs physico-chimiques des DGL**

| | |
|---|---|
| Tampon 1 | $H_2PO_4^-Na^+$ 50g/L, pH 4,5 dans l'eau (force ionique 0,61 M) |
| Tampon 2 | Tampon 1 + 3% acétonitrile (en poids) |
| Tampon 3 | Tampon physiologique : 3 8 mM $H_2PO_4^-$ $Na^+$ + 38 mM $HPO_4^{2-}$, 2$Na^+$ + 9 g/L NaCl, ajusté à pH 7,0 (force ionique 0,48 M) |

**Tableau 3 : Tableau regroupant l'ensemble des déterminations physico-chimiques effectuées sur les DGL**

| | | P1 | P2 | P3 | P4 | P5 | Méthode |
|---|---|---|---|---|---|---|---|
| Tampon 1 | $M_n$ (g/mol) | 1 450 | 8 600 | 22 000 | 65 300 | 172 300 | CES-2D |
| | DPn | 8 | 48 | 123 | 365 | 963 | |
| | I | 1,2 | 1,40 | 1,46 | 1,36 | 1,46 | |
| | dn/dC (mL/g) | 0,112 | 0,127 | 0,134 | 0,137 | 0,136 | |
| | $R_h$ (nm) | 1,1 (3%) | 2,1 (0,9%) | 3,2 (2%) | 3,92 (5%) | 6,74 (2%) | TDA |
| | $R_h$ (nm) | 1,18 (17%) | 2,33 (9%) | 3,41 (8%) | 6,31 (8%) | 8,38 (8%) | DLS |
| Tampon 2 | $R_h$ (nm) | 0,95 (2,4%) | 1,82 (1,1%) | 2,64 (0,2%) | 3,94 (2,8%) | 5,13 (0,8%) | TDA |
| | $R_h$ (nm) | 1,1 (26%) | 2,21 (8%) | 3,73 (12%) | 5,98 (6%) | 8,5 (13%) | DLS |
| Tampon 2 | $M_n$ (g/mol) | | 8 600 | 22 000 | 65 300 | 172 300 | CES-3D |
| | $R_h$ (nm) | | 1,79 | 2,95 | 4,95 | 7,99 | |
| | [η] (dL/g) | | 5,6 | 6,6 | 7,1 | 7,1 | |
| Tampon 3 | $R_h$ (nm) | (0,74) (11,4%) | 1,34 (4,6%) | 2,05 (0,63%) | 2,87 (2,2%) | 4,24 (0,26%) | TDA |
| | $R_h$ (nm) | 1,01 (ND) | 1,69 (ND) | 3,28 (13%) | 5,89 (15%) | 6,85 (17%) | DLS |

*I représente l'indice de polymolécularité. $DP_n$ représente le degré de polymérisation moyen. Entre parenthèses sont donnés les écart-types relatifs. Le nombre n de déterminations expérimentales indépendantes est de 5. CES-2D : chromatographie d'exclusion stérique double détection (diffusion de lumière et indice de réfraction) dans les conditions expérimentales décrites Figure 1 ; CES-3D : chromatographie d'exclusion stérique triple détection (diffusion de lumière, détecteur viscosimétl-ique et indice de réfraction). Conditions expérimentales de la CES-3D: tampon 5% $NaH_2PO_4$ + 3% Acétonitrile en volume, pH~4. Débit : 0,6 mL/min. Volume injecté : 100□L. Colonne Superose 12 HR 10/30. Température : 28°C.*

**Tableau 4 : Calcul du nombre moyen de monomères incorporés par amorceurs potentiels (fonction amine α ou ε) lors du passage d'une génération i à une génération i+1**

| Passage génération i à i+1 | $M_{t+1}$-$M_1$ (g/mol) | $N_{t+1}$-$N_t$ | nombre d'amorceurs à i | nombre moyen de monomères incorporés / amorceur |
|---|---|---|---|---|
| 1/2 | 7150 | 40 | 9 | 4,4 |
| 2/3 | 13 400 | 75 | 48 | 1,55 |
| 3/4 | 43 300 | 242 | 123 | 1,96 |
| 4/5 | 107 000 | 598 | 366 | 1,64 |

**Tableau 5 : Volumes hydrodynamiques, volumes de Van der Waals et fraction de volume libre des DGL en fonction du nombre de génération dans le tampon 2**

| DGL | Vh ($nm^3$) | VW ($nm^3$) | f |
|---|---|---|---|
| 1 | 3,59 | 1,0 | 0,721 |
| 2 | 25,3 | 5,9 | 0,766 |
| 3 | 77,1 | 15,1 | 0,804 |

(suite)

| DGL | Vh ($nm^3$) | VW ($nm^3$) | f |
|---|---|---|---|
| 4 | 256 | 44,8 | 0,825 |
| 5 | 565 | 118 | 0,791 |

Les degrés de polymérisation pris en compte pour les calculs de VW sont ceux donnés dans le **Tableau 2.**

## EXEMPLE 11

### Synthèses de triblock copolymères : poly(L-lysine) greffée-bloc-poly(éthylène glycol)-bloc-poly(L-lysine) greffée

1/ On synthétise 0,5 g de $N^\varepsilon$-TFA-L-Lysine-NCA.
2/ Au produit brut de la réaction on ajoute 5 ml d'une solution aqueuse 0,1 M de $HCO_3Na$ maintenue à 0˚C, dans laquelle on a dissout 0,42 g de poly(éthylène glycol)-$\alpha$,$\varepsilon$-diamine de 3400 Da (Shearwater Corporation).
3/ Après 30 min, on récupère par filtration ou centrifugation le précipité blanc de {poly(TFA-L-lysine) linéaire-bloc-poly(éthylène glycol)-bloc-poly(TFA-L-lysine) linéaire} formé dans le milieu.
4/ Ce précipité, placé dans 10 ml de solution aqueuse ammoniacale à pH 12 pendant 15 heures à 40˚C, perd les groupements protecteurs TFA des chaînes latérales des motifs ajoutés lors de la polymérisation et devient soluble dans l'eau.
5/ Le volume du solvant est réduit de moitié dans un évaporateur rotatif.
6/ Après lyophilisation on obtient 0,45 g d'une {poly(L-lysine) linéaire-bloc-poly(éthylène glycol)-bloc-poly(L-lysine) linéaire} de génération 1. Sa polydispersité est de 1,07 (mesurée en CES). Sa masse moyenne mesurée par LS est de 15 500 Da.

En répétant cette réaction, et en utilisant comme amorceur 0,15 g du produit obtenu lors de la réaction précédente, on obtient 0,3 g d'une {poly(L-lysine) greffée-bloc-poly(éthylène glycol)-bloc-poly(L-lysine) greffée}, de polydispersité 1,3 et de masse moyenne égale à 23 800 Da mesurée par CES-double détection (Indice de réfraction-diffusion de lumière).

## Exemple 11A

### Synthèse de DGL fluorescent de génération 1 (P1)

A 500 mg de $N^\varepsilon$-TFA-L-Lysine-NCA de l'exemple 1, on ajoute 5 ml d'une solution aqueuse de $HCO_3Na$ 0,1 M maintenue à 0˚C dans laquelle on a dissout 5 mg d'aminofluorescéine. La fonction amine de l'aminofluorescéine réagit sur le carbone 5 du NCA et forme la H-($N^\varepsilon$-TFA-lysine)-NH-fluorescéine, qui, à son tour, réagit sur d'autres NCA pour former H-($N^\varepsilon$-TFA-lysine)$_n$-NH-fluorescéine qui précipite avec un $DP_n$ égal à 8.

Le précipité traité comme celui de l'exemple 1 conduit à 240 mg de poly-L-Lysine linéaire de formule ($^+H_2$ $(Lys)_n$-$NHC_{20}H_{11}O_5$) avec n compris entre 2 et 20, caractérisé par MALDI-TOF ainsi que cela est montré dans la figure 10. Excité à 490 nm, ce matériau fluoresce dans une solution de bicarbonate de sodium 0,1 M à pH 8,5 avec une longueur d'onde maximale à 517 nm et un seuil de détectabilité inférieur à $10^{-3}$ ma.$L^{-1}$ par un spectrofluorimètre SLMAminco MC 200 monochromator dans une cellule de 1 cm. Cette longueur d'onde maximale d'émission est à comparer à celle de l'aminofluorescéine libre (513 nm) dans les mêmes conditions. Pour observer la fluorescence du dendrimère, on dissout 5 mg de ce matériau dans 10 ml d'une solution de bicarbonate 0,1 N. 100 $\mu$l de cette dernière solution sont dilués au centième par la solution de bicarbonate 0,1 N. Cette solution est excitée à 490 nm. L'émission de fluorescence est observée à une longueur d'onde maximale à 517 nm. Par dilutions successives le seuil de détectabilité d'un spectrofluorimètre SLMAminco MC 200 monochromator utilisant une cellule de 1 cm est atteint lorsque la concentration est égale à $10^{-3}$ mg.$L^{-1}$. La longueur d'onde maximale d'émission 517 nm est à comparer à celle de l'aminofluorescéine libre (513 nm) dans les mêmes conditions.

### Exemple 11B

### Synthèse de DGL fluorescent de génération 2 (P2)

Cette synthèse est effectuée de façon analogue à celle décrite dans l'exemple 2 en utilisant comme amorceur le produit de la réaction décrite dans l'exemple 11A. Le produit obtenu fluoresce à 517 nm (excitation 490 nm). Son seuil de détectabilité est de l'ordre de $10^{-3}$ $mg.L^{-1}$ dans des conditions identiques à celles de l'exemple 11A.

### Exemple 11C

### Synthèse de DGL fluorescent de génération 3 (P3)

Cette synthèse est effectuée de façon analogue à celle décrite dans l'exemple 3 en utilisant comme amorceur le produit de la réaction décrite dans l'exemple 11B. Le produit obtenu fluoresce à 519 nm (excitation 490 nm). Son seuil de détectabilité est de l'ordre de $10^{-2}$ $mg.L^{-1}$ dans des conditions identiques à celles de l'exemple 11A.

### Exemple 11D

### Synthèse de DGL fluorescent (rouge) de génération 1 (P1)

On dissout 200 mg de Poly-N-TFA-L-Lysine linéaire de génération 1 (P1) de $DP_n$ 8, obtenu comme dans l'exemple 1, dans 2 ml de diméthylformamide (DMF) anhydre. A cette solution on ajoute 5 mg de rhodamine sulfochlorure. Le milieu homogène obtenu est maintenu à 25˚C pendant 24 heures sous agitation magnétique. On ajoute alors au milieu réactionnel 10 ml d'une solution aqueuse de bicarbonate de sodium 0,1N. Un précipité rouge clair se forme immédiatement dans le milieu. Ce précipité est séparé par centrifugation à 5000 t/min pendant 10 min. Il est ensuite remis en suspension dans une solution de 10 ml de bicarbonate de sodium 0,1 N et centrifugé, et ceci 10 fois pour éliminer la rhodamine qui n'aurait pas réagi. Les dernières eaux de lavage sont incolores, alors que les derniers précipités restent de couleur rouge clair, ce qui montre que la rhodamine est bien fixée sur le P1 protégé. Le précipité P1 est alors placé dans 50 ml d'une solution aqueuse d'ammoniaque 1 N / méthanol 50/50 pendant 15 heures pour débloquer les fonctions epsilon de leurs groupements protecteurs TFA. Le milieu réactionnel est ensuite réduit de moitié sous vide pour éliminer le méthanol et l'ammoniaque. Après lyophilisation, on obtient 150 mg d'un produit rouge clair. Sa fluorescence rouge vif caractérise ce nouveau dendrimère de première génération marqué à la rhodhamine (texas red).

### EXEMPLE 12

### Modifications des fonctions amines libres des DGL

A un gramme de DGL P2 de Mn 8 600 Da en solution dans 50 $cm^3$ de solvant eau-acétonitrile 80/20, on ajoute 1 gramme de 3,5-diméthylester-dicarboxyphényl isothiocyanate. La solution résultante est maintenue à 40˚C pendant 10 heures. Deux grammes d'hydrogénocarbonate d'ammonium sont ajoutés à cette solution. Après 5 heures de contact à 40˚C, le milieu est dialysé et lyophilisé. On obtient 1,50 g de DGL P2, dans lequel chaque fonction amine est liée par une fonction thiourée à un phényl-3,6-dicarboxylate de sodium. La RMN dans $D_2O$ du produit obtenu montre la présence des signaux du DGL associés à ceux du groupement aromatique.

### EXEMPLE 13

### Fixation ionique des DGL sur support

3 g de DGL P3 de Mn 22 000 Da sont incubés avec 24 g de charbon Norit (GAC 1240 PLUS) dans 70 ml d'eau distillée stérile pendant 24 heures. Le charbon est ensuite lavé avec 250 ml d'eau distillée stérile. Après évaporation de cette dernière, on récupère 1,82 g de P3, ce qui montre que 1,18 g de P3 sont adsorbés sur 24 g de charbon Norit. L'activité de 1 g de ce charbon sur lequel est adsorbé 49 mg de P3 est testée sur *Micrococcus lysodeikticus*, en la comparant à celle d'un témoin support sans P3.

**EXEMPLE 14**

**Fixation covalente des DGL sur support**

Un oligomère d'hexaméthylène-düsocyanate [OCN-$(CH_2)_6$-(NHCONH-$(CH_2)_6)_n$-NCO] est utilisé pour fixer de façon covalente les DGL sur des résines support. On mélange vigoureusement des quantités connues de DGL (P1, P2, P3) dans environ 5 g d'oligomère de façon à avoir des pourcentages de DGL variables de 0 à 20%, et on étale le mélange sur des plaques de verre. Après 48 heures, on broie la résine obtenue, jusqu'à obtenir des particules comprises entre 0,1 et 1 mm, et on la lave abondamment avec 250 ml d'acétone, 250 ml d'eau, 250 ml de méthanol, 250 ml d'éther éthylique et on sèche.

**Exemple 14A**

**Fixation covalente du P2 et du P3 sur silice activée**

25 mg de DGL fluorescents obtenus dans les exemples 11B et 11C sont dissous dans 25 mL d'une solution aqueuse de $HCO_3Na$ 0,1 N à 25°C. A cette solution, agitée par un dégagement d'azote bulle à bulle, on ajoute 1 g de grains de silice de 50 microns, surface spécifique (500 $m^2/g$), portant 1,1 meq de Si-$(CH_2)_3$NCO. Après 2 heures, les grains de silice sont séparés, lavés à l'eau et séchés. 20 mg de ces DGL, mesurés par différence avec la solution initiale, sont ainsi fixés sur 1 g de support. La surface de ces supports, observée au microscope à fluorescence, présente la fluorescence verte caractéristique de la fluorescéine.

**EXEMPLE 15**

**Etude de la toxicité *in vivo* des DGL**

Les expérimentations ont été réalisées sur des rats de souche Sprague-Dawley âgés de 4 à 8 semaines (Elevage CER Janvier, Le Genest St-Isle).

**A. Toxicité aiguë** :

5 rats reçoivent une injection de 2mL d'une solution de DGL P3 (60 mg/ml) trois fois par jour pendant 4 jours. Les résultats obtenus sont présentés dans le **Tableau 7** suivant :

**Tableau 7 : étude de la toxicité aiguë d'une solution de DGL P3**

| Poids jour 1 | | Poids jour 4 | | Var % | Var % |
|---|---|---|---|---|---|
| moyenne | E.T. | moyenne | E.T. | moyenne | E.T. |
| 252 | 10 | 280 | 11 | 11,0 | 10,0 |
| Aucun effet toxique n'est visible. | | | | | |

**B. Toxicité chronique:**

Les rats reçoivent une injection sous-cutanée de 0,5 mL de DGL P3 (60mg/ml) chaque jour pendant 180 jours. Les rats contrôles reçoivent 0,5ml de sérum physiologique (solvant de la solution). L'évolution pondérale des rats traités et des rats contrôles est comparée (**Figure 7**). Aucune modification dans l'évolution pondérale, ni aucun effet néfaste n'a été observé.

Ces expérimentations soulignent que le dendrimère greffé de lysine (DGL) P3 ne présente aucun caractère de toxicité apparent : tous les animaux sont vivants et ont un développement pondéral homogène et régulier, superposable à celui des contrôles.

**EXEMPLE 16**

**Activité antitumorale des DGL**

Des tests de cytotoxicité ont été réalisés sur des cellules myélomateuses de souris avec des concentrations

variables des DGL P2, P3, P4 et P5. Les résultats obtenus sont présentés dans le **Tableau 8** suivant.

**Tableau 8** : **Etude comparée de l'activité cytotoxique des polylysines dendrimères de l'invention sur cellules de myélome**

| *Concentration μM* | 50 | 25 | 12.5 | 6.25 | 3.125 | 1.56 | 0.78 | 0.39 | 0.195 | 0.09 |
|---|---|---|---|---|---|---|---|---|---|---|
| P2 (8 600 Da) en mg/L | ++++ | ++++ | +++ | ++ 52 | + | - | - | - | - | - |
| P3 (22 000 Da) en mg/L | ++++ | ++++ | ++++ | +++ | +++<br>++ | 34 | + | +/- | - | - |
| P4 (65 300 Da) en mg/L | ++++ | ++++ | +++ | +++ | ++<br>202 | + | +/- | - | - | - |
| P5 (172 300 Da) en mg/L | ++++ | ++++ | ++++ | +++ | ++ | ++<br>263 | + | +/- | - | - |

++++ = amas cellulaires et lyses cellulaires
++++ = amas cellulaires agglutinés et lyses cellulaires
- = cellules vivantes identiques au témoin

Ces tests sur cellules myélomateuses montrent que les DGL sont actifs sur le myélome murin pour des concentrations égales ou supérieures à 34 mg/L selon la génération de DGL étudiée. Les DGL de forte masse molaire sont les moins actifs.

**EXEMPLE 17**

**Activité antifongique des DGL**

Les tests antifongiques ont été réalisés sur un champignon filamenteux, *Fusarium oxysporum* (Collection INRA Saint Christol-lés-Alès, France) selon la technique d'inhibition de croissance en milieu liquide décrite par Fehlbaum et al [37].

80 μl des spores de *F. oxysporum* (concentration finale de $10^4$ spores.$mL^{-1}$) en suspension dans le milieu PDB (Potato Dextrose Broth, Difco) contenant 0,1 mg de tétracycline, sont additionnés à 20 μl des différentes dilutions des dendrimères dans des microplaques. Les dendrimères sont remplacés par 20 μl d'eau stérile pour les contrôles. L'inhibition de croissance est observée sous le microscope après 24 heures d'incubation à 30°C sous agitation et quantifiée après mesure de la densité optique à 48 heures. La concentration minimale de croissance (MIC) est évaluée par des dilutions en série (en doublets) des molécules et définie comme la plus faible concentration en dendrimère inhibant la croissance.

Les résultats des tests antifongiques sont consignés dans le tableau ci-dessous.

**Tableau 9 : Etude comparée de l'activité antifongique des dendrimères greffés de lysine de l'invention** :

| Dendrimères greffés de lysine | P2 | P3 | P4 | P5 |
|---|---|---|---|---|
| **Champignon :**<br>*Fusas-ium oxysporum*<br>*(Collection INRA St Christol lés Alès)* | | | | |
| en μM | 22 | 1.25 | 0.54 | 0.36 |
| *en mg/L* | 183 | 27 | 35 | 61 |
| *(valeurs des MIC en μM et en mg/L)* | | | | |

On peut globalement noter que la MIC est égale à 27 mg/L pour *Fusarium oxysporum*.

**EXEMPLE 18**

**Activité antibactérienne des DGL**

La méthode utilisée pour les tests antibactériens est une modification de la technique de Hancock et al [38]. La mesure de l'activité antibactérienne est réalisée sur 3 souches de bactéries Gram-positif (*Micrococcus lysodeikticus* ATCC 4698, *Bacillus megaterium* ATCC 17748, *Staphylococcits aureus* ATCC 25293), et 4 souches de bactéries Gram négatif (*Escherichia coli* 363 ATCC 11775, *Vibrio penaeicida*, collection IFREMER, *Vibrio angztillarum Listonella anguillarum* ATCC 14181, *Vibrio alginolyticus* ATCC 17749) par des mesures de l'inhibition de croissance en milieu liquide.

**A. En phase homogène :**

Brièvement, 10 µl des molécules de synthèse (P2, P3, P4 et P5) sont incubés dans des microplaques avec 100 µl de chaque suspension bactérienne à la concentration de départ D600 = 0,001, dans du milieu PB (Poor Broth : bactotryptone 1%, NaCl 0.5% w/v, pH 7.5). La croissance bactérienne est mesurée à 600 nm après une incubation de 24 Heures à 30°C sous agitation. La concentration minimale de croissance (MIC) est évaluée par des dilutions en série (en doublets) des molécules et définie comme la plus faible concentration en dendrimère inhibant la croissance des bactéries.

Les résultats des tests antibactériens consignés dans le tableau ci-dessous mettent en évidence que le spectre d'activité des dendrimères P2 à P5 est large, en effet ces molécules sont actives aussi bien sur les bactéries Gram + que Gram -.

**Tableau 10 : Etude comparée de l'activité antibactérienne des DGL**

| **Dendrimères greffés de Lysine** | **P2** | **P3** | **P4** | **P5** |
|---|---|---|---|---|
| **Microorganismes** | | | | |
| **Gram+** | | | | |
| *M. lysodeikticus ATCC 4698* en µM<br>*en mg/L* | 1,56<br>13 | 0,78<br>17 | 1,56<br>100 | 0,39<br>66 |
| *B. megaterium ATCC 17749* en µM<br>*en mg/L* | 0,39<br>3 | 1,56<br>34 | 1,56<br>100 | 0,78<br>132 |
| *Staphylococcus aureus ATCC 25293* en µM<br>*en mg/L* | >12,5<br>104 | 3,125<br>67 | 0,78<br>50 | 0,39<br>66 |
| **Gram-** | | | | |
| *E. coli 363 ATCC 11775* en µM<br>*en mg/L* | 2,8<br>23 | 1,56<br>33,5 | 33,3<br>2131 | 22,2<br>3751 |
| *Vibrio penaeicida collection IFREMER* en µM<br>*en mg/L* | >50<br>417 | 25<br>537 | 3,125<br>200 | 1,56<br>26 |
| *Vibrio anguillarmn* en µM<br>*en mg/L* | 22,2<br>185 | 2,8<br>60 | <1,9<br>121 | <1,9<br>321 |
| *Vibrio alginolyticus ATCC 17749* en µM<br>*en mg/L* | 9,9<br>83 | 2,8<br>60 | <1,9<br>121 | <1,9<br>321 |
| *(valeurs des MIC en µM et en mg/L)* | | | | |

L'activité antimicrobienne exprimée en µM ou mg/L montre une grande variabilité d'un dendrimère à l'autre et selon la souche bactérienne étudiée. On peut globalement noter que les MIC varient, en utilisant dans chaque cas de figure la génération de DGL la plus active, de 3 à 50 mg/L pour les Gram +, et de 23 à 200 mg/L pour les Gram -.

**B. En phase hétérogène :**

L'activité antibactérienne des dendrimères DGL fixés de façon ionique ou covalente sur support a été testée.

1. Fixation ionique :

La fixation a été réalisée comme décrite dans l'**Exemple 13**. Ensuite, les supports (1 g) sont lavés par 5 fois 20 ml d'eau stérile et séchés. Les produits obtenus sont mis en contact avec 2 ml de suspension bactérienne (*Micrococcus lysodeikticus*) pendant 24 heures. Après cette période d'incubation, 100 μL de surnageant sont prélevés et déposés sur boîte LB agar et mis à incuber 24 heures à 37°C. Le nombre de colonies observées (ou non) rend compte de l'activité des supports utilisés. Les résultats sont présentés dans le tableau suivant.

**Tableau 11 : Activité antibactérienne de DGL fixés de manière ionique sur support:**

| | Observation |
|---|---|
| Témoin | Boite envahie |
| Charbon + P3 | Absence de colonies |

Ces résultats indiquent que les DGL exercent également une action antimicrobienne lorsque fixés sur un support de manière ionique.

Les fixations ioniques n'étant pas nécessairement permanentes, l'activité antimicrobienne des DGL fixés de façon covalente a également été testée.

2. Fixation covalente :

La fixation a été réalisée comme décrite dans l'**Exemple 14.** Ensuite, 0,5 g de chacun des matériaux est mis en contact avec 2 ml de suspension bactérienne (*Micrococcus lysodeikticus*), contenant $2.10^6$ bactéries, pendant 24 heures. Après cette période d'incubation, 25 μL de surnageant sont prélevés et déposés sur 75 μL de milieu LB ce mélange est étalé sur boîte LB agar et mis à incuber à 37°C. La lecture après 96 heures d'incubation fait apparaître les résultats consignés dans le tableau ci-dessous.

**Tableau 12 : Activité antibactérienne des DGL fixés de manière covalente sur support :**

| Matériaux | Nombre de colonies à J+3 |
|---|---|
| résine (témoin) | boite envahie |
| résine + 2% P1 | 300 |
| résine + 2% P2 | 0 |
| résine + 2% P3 | 35 |
| résine + 1% P3 | 65 |
| résine + 0,5% P3 | > 300 |

Les résultats montrent que les DGL fixés de façon covalente sur support conservent une excellente activité antibactérienne à des concentrations supérieures ou égales à 1 %. Des études complémentaires doivent être réalisées avec des broyages plus fins et avec de nouvelles résines.

**EXEMPLE 19**

**Activités des DGL sur *Pseudomonas aeruginosa***

**A. En phase homogène :**

Des suspensions de *Pseudomonas aéruginosa* à 10 000 bactéries/ml ont été mises en contact avec des solutions de P2 et P3 à 1, 10, et 100 mg/L. A 6, 24 et 96 heures des contrôles ont été effectués par mise en culture sur cétrimide.

Les résultats présentés dans le tableau suivant montrent la totale inhibition des *Pseudomonas* par des solutions de P3 à 100 mg/L.

**Tableau 13 : Effet des DGL en phase homogène sur une suspension à 10 000 *P. aeruginosa*/ml**

| Temps | témoin PBS + suspension | P2 à 1 mg/L | P2 à 10 mg/L | P2 à 100 mg/L | P3 à 1 mg/L | P3 à 10 mg/L | P3 à 100 |
|---|---|---|---|---|---|---|---|
| T = 0 | >300 | / | / | / | / | / | / |
| T = 6h | >300 | 0 | 0 | 0 | 0 | 0 | 0 |
| T = 24h | boîte envahie | boîte envahie | 1 | 1 | 108 | 0 | 0 |
| T = 96h | boîte envahie | boîte envahie | 300 | 150 | boîte envahie | 250 | 0 |
| *(filtration de 100µl sur membrane + mise en culture sur cétrimide)* | | | | | | | |

**B. En phase supportée :**

Les *Pseudomonas aeruginosa* ont été exposés à des résines d'hexaméthylène-diisocyanate (comme synthétisées précédemment) contenant 10% en poids de P3 (P3S) pendant des temps variables. A 6, 24 et 96 heures, des contrôles ont été effectués par mise en culture sur cétrimide. Les résultats présentés dans le tableau suivant montrent la totale inhibition des *Pseudomonas* par de tels supports sur des suspensions de 1 000 *Pseudomonas/ml.*

**Tableau 14** : **Effet des DGL en phase supportée sur des suspensions de P. *aeruginosa :***

| | *Suspension à 10 000 Pseudomonas/ml (filtration de 100µl sur membrane mise en culture sur cétrimide)* | | *Suspension à 1 000 Pseudomonas/ml + (filtration de 100µl sur membrane + mise en culture sur cétrimide)* | |
|---|---|---|---|---|
| Temps | **Témoin PBS+ suspension** | **P3S à 10o/L** | **Témoin PBS + suspension** | **P3S à 10g/L** |
| **T=0** | 53 | / | 3 | / |
| **T = 6h** | 83 | 1 | 9 | 0 |
| **T= 24h** | boîte envahie | 0 | 8 | 0 |
| **T= 96h** | boîte envahie | boîte envahie | boîte envahie | 0 |

**EXEMPLE 20**

**Activités des DGL sur *Legionella pneumophila***

**A. En phase homogène :**

Des suspensions de *Legionella pneumophila* 10 000/ml ont été mises en contact avec des solutions de P2 et P3 à 10, et 100 mg/L. A 24, 96 et 168 heures des contrôles ont été effectués par mise en culture sur GVPC.

Les résultats présentés dans le tableau suivant et dans la **Figure 12** montrent que *Legionella pneumophila* est plus sensible à P2 qu'à P3.

**Tableau 15 : Effet des DGL en phase homogène sur une suspension de *Legionella pneumophila***

| *Suspension à 10 000 légionelle/ml (étalement de 100µl sur G VPC)* | | | | | |
|---|---|---|---|---|---|
| **Temps** | **Témoin PBS + susp** | **P2 à 10mg/L** | **P2 à 100mg/L** | **P3 à 10mg/L** | **P3 à 100mg/L** |
| J0 | 210 | 210 | 210 | 210 | 210 |
| **J+1** | 180 | 19 | 27 | 45 | 28 |
| **J+4** | 78 | 3 | 8 | 11 | 14 |
| **J+7** | 45 | 4 | 6 | 3 | 3 |

(suite)

| Effet inhibiteur relatif observé par rapport au témoin | | | | | |
|---|---|---|---|---|---|
| **Temps** | **Témoin PBS + susp** | **P2 à 10mg/L** | **P2 à 100mg/L** | **P3 à 10mg/L** | **P3 à 100mg/L** |
| **J0** | 100 | 100 | 100 | 100 | 100 |
| **J+1** | 100 | 10,6 | 15,0 | 25,0 | 15,6 |
| **J+4** | 100 | 3,8 | 10,3 | 14,1 | 17,9 |
| **J+7** | 100 | 8,9 | 13,3 | 6,7 | 6,7 |

Un contrôle PCR a été effectué.

Brièvement, après 96 heures de contact avec les dendrimères P2 et P3 à 10 et 100 mg/L, 5 ml de suspension bactérienne sont filtrés sur une membrane en polycarbonate de 0,45 $\mu$m pour extraction des cellules présentes (les résidus cellulaires sont ainsi éliminés). Les cellules sont récupérées sur le filtre, leur ADN est extrait par chocs thermiques et chimiques. 1 ml est purifié par ultrafiltration, et quantifié par amplification PCR. Les résultats sont présentés dans le tableau suivant :

**Tableau 16 : quantification de l'ADN des suspensions de *L. pneumophila* en présence de P2 ou P3**

| | **Suspension extraite, purifiée + PCR Résultats pour 10 ml de suspension** | **Résultat relatif** |
|---|---|---|
| Témoin 10000/ml | $2,94.10^5$ | 100 |
| **P2 (10mg/ml)** | $2,08.0^4$ | 7 |
| **P2 (100mg/ml)** | $2,68.10^4$ | 9 |
| **P3 (10mg/ml)** | $7,94.10^4$ | 27 |
| **P3 (100mg/ml)** | $6,46.10^4$ | 22 |

Ainsi, l'analyse par PCR montre :

- que la présence de P2 ou de P3 permet en 96 heures d'éliminer du milieu de culture, respectivement plus de 90 %, ou entre 70 et 80 % de bactéries par rapport au témoin. L'activité de P2 est supérieure à celle de P3 sur *Legionella pneumophila*.
- que P2 ou P3 provoque aux concentrations létales une dégradation bactérienne par perforation des parois cellulaires et libération de leur ADN.

**B. En phase supportée :**

Des résines d'hexaméthylène-diisocyanate contenant 2% en poids de P1 (P1S) et 20% en poids de P3 (P3S) sont obtenues comme précédemment.

<u>Test n°1 :</u>

Tube 1 : Le témoin est constitué de 9 ml de PBS + 1 ml PBS contenant 10 000 *Legionella pneumophila.*
Tube 2 : 9ml de PBS + 1 ml PBS contenant 10 000 *Legionella pneumophila* + 0,1g de P3S.

Les résultats sont présentés dans le tableau suivant :

**Tableau 17: quantification de l'ADN de suspensions de *L. pneumophila* mises en présence de P3S**

| | | **Suspension extraite, purifiée + PCR résultats dans 10 ml de susp.** | **Résultat relatif** |
|---|---|---|---|
| **Tube 1** | **Témoin 10000/ml** | $7,36.10^4$ | 100 |

(suite)

| | | Suspension extraite, purifiée + PCR résultats dans 10 ml de susp. | Résultat relatif |
|---|---|---|---|
| **Tube 2** | **P3S** | $7,36.10^3$ | 10 |

Test n°2 :

Tube 1 : Le témoin est constitué de 9 ml de PBS + 1 ml PBS contenant 10 000 *Legionella pneumophila.*
Tube 2 : Temoin résine : 9ml de PBS + 1 ml PBS contenant 10 000 *Legionella pneumophila* + 0.1g de P 1 S
Tube 3 : 9ml de PBS + 1 ml PBS contenant 10 000 *Legionella pneumophila* + 0.1g de P3S

**Tableau 18 : quantification de l'ADN de suspensions de *L. pneuniophila* mises en présence de P3S**

| | | Suspension extraite, purifiée + PCR résultats dans 10 ml de susp. | Résultat relatif |
|---|---|---|---|
| **Tube 1** | **Témoin 10000/ml** | $1,06.10^6$ | 100 |
| **Tube 2** | **Témoin TR** | $3,26.10^5$ | 31 |
| **Tube 3** | **P3S** | $3,59.10^4$ | 3 |

Un contrôle PCR a été réalisé comme suit. Après 96h de contact on laisse décanter les résines au fond des tubes, 5 ml de suspension sont filtrés sur une membrane en polycarbonate de 0.4µm pour extraction des cellules. Celles-ci sont lavées, lysées par chocs thermiques et chimiques. Les ADN sont purifiés, amplifiés par PCR, et comptés.
Ces résultats confirment :

- l'action du dendrimère P3 greffé sur polyisocyanate
- un effet non négligeable du témoin (P1S) même s'il semble inférieur à celui de P3S.

**Exemple 21**

**Activité antimicrobienne des DGL fluorescents**

Les expériences décrites dans l'exemple 18, reprises en utilisant le DGL P2 de l'exemple 11B, ont montré que l'activité antimicrobienne des DGL fluorescents était identique à celle des DGL non fluorescents.
Les DGL fluorescents sont en conséquence utilisables pour étudier le mode d'action des DGL. Par mode d'action, on désigne des mécanismes antimicrobiens, mais également des mécanismes de transport de drogues, suivis dans les circuits sanguins, marquages fluorescents de cibles par des DGL portant des anticorps spécifiques.

**Exemple 22**

**Intérêt des DGL pour la production d'anticorps et leur caractérisation.**

Des immunoconjugués d'histamine dérivés ont été préparés avec deux porteurs : la sérum albumine bovine (BSA) et le DGL de génération 3 (voir formule ci-après) selon la méthode décrite par Vandenabeele-Trambouze, O. et al [39].

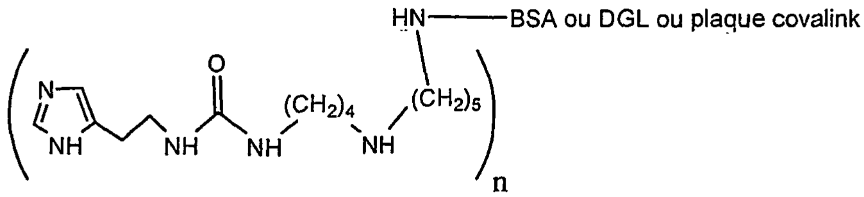

Structure de l'immunoconjugué entre l'histamine (représentant un haptène modèle) et les porteurs BSA, DGL et les plaques covalink

Les couplages sont effectués avec 5 mg d'haptènes, 20 mg de BSA ou DGL et 300 µl de glutaraldéhyde à 5% dans un tampon 1,5 molaire d'acétate de sodium pH 8 et réduits au $NaBH_4$ 3 N. 3 lots de deux lapins ont été immunisés comme suit :

Lot A : 150 µg de d'immunoconjugué-BSA dans de l'adjuvant complet de Freund à J = 0, puis 150 µg de conjugué-DGL dans de l'adjuvant complet de Freund à J = 21, J =36etJ=51.
Lot B : 150 µg de conjugué-DGL dans de l'adjuvant complet de Freund à J = 0, J =21,J=36etJ=51.
Lot C : 300 µg de DGL natif dans de l'adjuvant complet de Freund à J = 0, J = 21, J=36etJ=51.

Les sérums des lots A, B, C, ont été analysés à J = 21, J = 36, J = 51 et J = 65 selon deux dosages ELISA :

- sur plaque Maxisorp (NUNC) préalablement coatée avec des immunoconjugués de la BSA ou du DGL (**Figures 13 et 15**) ou les porteurs libres selon la méthode décrite par Vandenabeele-Trambouze, O. et al [39]. Cette méthode permet d'évaluer le titre total en anticorps (spécifiques à l'antigène ou l'haptène et spécifique au porteur) ainsi que le taux d'anticorps anti-porteur.
- sur plaque Covalink (NUNC) (**Figure 14**) préalablement coatée avec l'haptène (selon le protocole décrit par Claeys-Bruno et al [40, 41]). Cette méthode permet une évaluation directe des anticorps spécifiques uniquement.

L'absence d'anticorps mesurables à J65 (nombre de jours = 65) pour les lots C et B (**Figure 13**) démontre l'immuno-furtivité du DGL de génération 3, y compris lorsque celui-ci est greffé à un haptène. De la même manière, l'absence de réponse ELISA pour les LOT A et C à J65 sur plaque Maxisorp coatée au DGL natif (cercles blancs et croix - **Figure 13**) confirme l'absence d'anticorps anti-DGL et donc leur immuno-furtivité. Ce résultat est confirmé sur plaques covalink pour le LOT B (**Figure 14,** tirets) qui ne présente aucun anticorps anti-haptène.

Cependant, lorsque le DGL est utilisé en second porteur (LOT A) après stimulation du système immunitaire par un porteur immunogènique (BSA) on constate, d'une part, une augmentation du titre en anticorps spécifique de l'haptène (voir **Figure 14** entre J21 et J36) et, d'autre part, la disparition des anticorps anti-BSA (porteur) après plusieurs immunisations au GDL-haptène (**Figure 15**).

L'ensemble de ces résultats démontre que les DGL bien qu'immunofurtifs, permettent d'augmenter le titre en anticorps spécifiques de l'haptène en réduisant simultanément le taux d'anticorps non spécifiques anti-porteur utilisé pour déclencher la réponse immunitaire initiale.

## RÉFÉRENCES

[1]. Denkewalter, R.G., J. Kolc, and W.J. Lukasavage, Macromolecularhighly branched homogeneous compound based on lysine units., in USP. 1981, Allied Corp.: US.
[2]. Denkewalter, R.G., J. Kolc, and W.J. Lukasavage, Macromolecular highly branched homogeneous compound, in USP. 1983, Allied Corp.: US.
[3]. Juan Rodriguez-Hemandez, Marco Gatti, and Harm-Anton Klok, Highly Branched Poly(L-lysine). Biomacromolecules, 2003. 4: p. 249-258.
[4]. Klok Harm-Anton and Rodriguez-Hernandez Juan, Dendritic-Graft Polypeptides. Macromolecules, 2002. 35: p. 8718-8723.
[5]. Matthews Barry Ross, et al., Agent for the prevention and treatement of sexually transmitted diseases-I., in World Intellectual Property Organization (PCT). 2002, Starpharma Limited: USA. p. 36.
[6]. Matthews Barry Ross and H. George, Anionic or cationic dendrimer-antimicrobial or antiparasitic compositions., in USPTO. 2003, Starpharma Limited: USA.

[7]. Baigude H., et al., Synthesis of sphere-type monodispersed olygosaccharide-polypeptide dendrimers. Macromolecules, 2003. 36(19): p. 7100-7106.

[8]. Menz T. L. and Chapman T., Synthesis and characterization of hyperbranched polylysine. Polymer Preprints, 2003. 44(2): p. 842-843.

[9]. Takuro, N., et al., Method of gene delivery into cells using dendritic poly(L-lysine)s. peptide science, 2001. 2000 (37th): p. 201-204.

[10]. Choi Joon Sig, et al., Synthesis of a barbell-like triblock copolymer, poly(L-lysine) dendrimer-bloc-poly(ethylene glycol)-bloc-poly(L-lysine) dendrimer, and its self-assembly with plasmid DNA. Journal of American Chemical Society., 2000. 122: p. 474-480.

[11]. Kricheldorf, H.R., α-Aminoacid-N-Carboxy-Anhydrides and Related Heterocycles. Synthesis, Properties, Peptide Synthesis, Polymerisation. Heterocycles. 1987, Berlin: Springer-Verlag. 213 pages.

[12]. Bartlett, P. and R. Jones, A Kinetic Study of the Leuchs Anhydrides in Aqueous Solution. J. Am. Chem. Soc., 1957. 79(9): p. 2153-2159.

[13]. Commeyras, A., et al., Prebiotic Synthesis of Sequential Peptides on the Hadean Beach by a Molecular Engine Working with Nitrogen Oxides as Energy Sources. Polymer International., 2002. 51: p. 661-665.

[14]. Denkewalter, R.G.S., H.; Strachan, R. G.; Beesley, Thomas E.; Veber, Daniel F.; Schoenwaldt, Erwin F.; Barkemeyer, H.; Paleveda, William J., Jr.; Jacob, Theodore A.; Hirschmann, Ralph. , Controlled synthesis of peptides in aqueous medium. I. Use of α-amino acid N-carboxyanhydrides. Journal of the American Chemical Society 1966. 88(13): p. 3163-4.

[15]. Hirschmann R., et al., The controlled Synthesis of peptides in aqueous medium. III. Use of Leuchs' anhydrides in the synthesis of dipeptides. Mécanism and control of side reactions. Journal of Organic Chemistry 1967. 32(11): p. 3415-3425.

[16]. Iwakura Y., et al., Stepwise synthesis of oligopeptides with N-carboxy α-amino acid anhydrides. Biopolymers, 1970. 9: p. 1419-1427.

[17]. Hitz T. H. and Luisi P. L., Spontaneous onset of homochirality in oligopeptide chain generated in the polymerisation of N-carboxyanydride amino acids in water. Origin of life and evolution of the biosphere, 2004. 34: p. 93-110.

[18]. Sakamoto M. and Kuroyanagi Y., Multichain copoly(α-aminoacid). Journal of polymer science, 1978. 16: p. 1107-1122.

[19]. Birchall A.C. and North M., Synthesis of highly branched block copolymers of enantiomerically pure amino acids. Chem. Commun., 1998: p. 1335-1336.

[20]. Zigang Yang, Jianjun Yuan, and Shiyuan Cheng, Self-assembling of biocompatible BAB amphiphilic triblock copolymers PLL(Z)-PEG-PLL(Z) in aqueous medium. European polymer journal., 2005. 41: p. 267-274.

[21]. Poche D., Moore M., and Bowles J., An unconventional method for purifying the N-carboxyanhydride derivatives of γ-alkyl-L-glutamates. Synthetic communications, 1999. 29(5): p. 843-854.

[22]. Rekker R. F., The hydrophobic fragmental constant., ed. P. library. Vol. Vol. 1. 1977, Amsterdam: Elsevier.

[23]. Leuchs, H., Ber. Dtsch. Chem. Ges., 1906. 39: p. 857-859.

[24]. Curtius, T., et al., J. Prakt. Chem., 1930. 125: p. 211-302.

[25]. Coleman D. and Farthing A. C., J. Chem. Soc., 1951: p. 3218-3222.

[26]. Mobashery, S. and M. Johnston, A new appr-oaclz to the preparation of N-carboxy α-amino acid anhydrides. J. Org. Chem., 1985. 50: p. 2200-2202.

[27]. Daly, W.H. and D. Poche, The preparation of N-carboxyanhydrides of α-amino acids using bis(trichloromethil) carbonate, Tetrahedron Lett., 1988. 29: p. 5859-5862.

[28]. Wilder, R. and S. Mobashery, The use of triphosgene in preparation of N-carboxy-α-amino acid anhydrides. J. Org. Chem., 1992. 57: p. 2755-2756.

[29]. Collet, H., et al., A new simple and quantitative synthesis of α-Aminoacid-N-Carboxyanhydrides. Tetrahedron Letters, 1996. 37(50): p. 9043-9046.

[30]. Pascal R., Boiteau L., and Commeyras A., From the prebiotic synthesis of α-aminoacids, towards a primitive translation apparatus for the synthesis of peptides. Top. Curr. Chem., 2005(259): p. 69-122.

[31]. Fréchet, J.M. and D.A. Tomalia, eds. Dendrimers and other dendritic polymers., ed. W.s.i.p. science. 2001, John Wiley & Sons, Ltd. 646.

[32]. Commeyras, A., et al., *Procédé de synthèse peptidique à partir des N-(N'-Nitroso)carbamoylaminoacides, in patent,* PCT/FR 95/01380*.* 1995: Fr.

[33]. Matthews Barry Ross, et al., Agent for the prevention and treatment of sexually transmitted diseases-II, in World Intellectual Property Organization. 2002, Starpharma Limited: USA. p. 30.

[34]. Bello, M.S., R. Rezzonico, and P.G. Righetti, Use of Taylor-Aris dispersion for measurement of a solute diffusion coefficient in thin capillaries. Science, 1994. 266: p. 773.

[35]. Mes E. P. C., et al., Comparison of methods for the determination of diffusion coefficients of polymers in dilute solutions the influence of polydispersity. Journal ofPolymer Science: Part B: Polymer Physics, 1999. 37: p. 593-603.

[36]. Edward J. T., Molecular volumes and the stokes-einstein equation. Journal of Chemical Education, 1970. 47: p. 261.
[37]. Fehlbaum, P., et al., Insect imunity. Septic injury of Drosophila induces the synthesis of a potent antifungal peptide with sequence homology to plant antifungal peptides. J. Biol. Chem., 1994. 269: p. 33159-33163.
[38]. Hancock, R.E., T. Falla, and M. Brown, Cationic bactericidal peptides. Adv. Microb. Physiol., 1995. 37: p. 135-175.
[39]. Vandenabeele-Trambouze, O., et al., Antibodies directed against L and D isovaline using a chemical derivatizating reagent for the measurement of their enantiomeric ratio in extraterrestrial samples: first step-production and characterization of antibodies. Chirality, 2002. 14: p. 519-526.
[40]. Claeys-Bruno, M., et al., Methodological approaches for histamine quantification using derivatization by chloroethylnitrosourea and ELISA measurement. Part I: Optimisation of derivated histamine detection with coated-plates using optimal design. Chemometrics and intelligent Laboratory Systems, 2006. 80,((2)): p. 176-185.
[41]. Claeys-Bruno, M., et al., Methodological approaches for histamine quantification using derivatization by chloroethylnitrosourea and ELISA measurement. Part II: Optimisation of the derivatization step. Chemometrics and intelligent Laboratory Systems, 2006. 80((2),): p. 186-197.

## Revendications

1. Utilisation de monomères d'α-aminoacides activés pour la préparation de polypeptides hydrophobes sous forme de précipités, lesdits polypeptides résultant de la polymérisation desdits monomères d'α-aminoacides activés en solvant aqueux et étant susceptibles d'être resolubilisés dans ledit solvant.

2. Utilisation selon la revendication 1, dans laquelle les α-aminoacides activés sont choisis parmi des α-aminoacides N-carboxyanhydrides (NCA), des α-aminoacides-N,N'-carbonyldiimidazole, des α-aminoacides-sulfure de carbonyle, des α-aminoacides-anhydride carbonique et des amino thioacides-agents oxydants.

3. Utilisation selon la revendication 1 ou 2, d'α-aminoacides N-carboxyanhydrides (NCA) de formule (1) suivante :

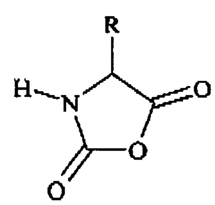

dans laquelle R représente une chaîne latérale d'un α-aminoacide naturel ou modifié.

4. Utilisation selon la revendication 2 ou 3, de monomères de L-lysine-NCA pour la préparation de polylysine en solvant aqueux.

5. Procédé de préparation d'une homo ou hétéropolylysine dendrimère greffé, à partir d'un amorceur comprenant au moins un groupement amine primaire ou secondaire, comprenant une étape d'addition d'un monomère de L-lysine-NCA, et éventuellement d'un ou plusieurs autres monomères d'α-aminoacides-NCA, choisis parmi la liste comprenant la L-ornithine-NCA, l'acide L-glutamique-NCA et sa γ-amide, l'acide L-aspartique-NCA et sa β-amide, l'acide L-diamino-2,4,-butyrique-NCA et sa β-amide, la L-tyrosine-NCA, la L-sérine-NCA, la L-thréonine-NCA, la L-phenylalanine-NCA, la L-valine-NCA, la L-leucine-NCA, la L-isoleucine-NCA, la L-alanine-NCA, et la glycine-NCA, audit amorceur dans un solvant aqueux.

6. Procédé de préparation d'une homo ou hétéropolylysine dendrimère greffé selon la revendication 5, dans lequel le monomère est uniquement de la L-lysine-NCA.

7. Procédé de préparation d'une homo ou hétéropolylysine dendrimère greffé selon la revendication 5 ou 6, dans lequel la L-lysine-NCA est protégée en $N^{\varepsilon}$, par un groupement choisi parmi :

-COH (Formyl), $-COCF_3$ (TFA), $-OCOC(CH_3)_3$ (Boc), $-COOCH_2\Phi$ (Z),

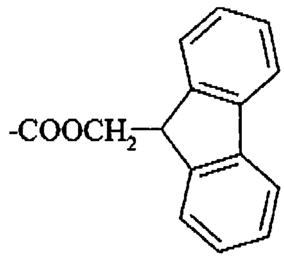

(Fmoc)

ou -C($\Phi$)$_3$ (trityl).

**8.** Procédé de préparation d'une homo ou hétéropolylysine dendrimère greffé selon la revendication 6 ou 7, dans lequel l'amorceur est choisi parmi la L-lysine, la L-ornithine, une homopolylysine, un poly(éthylène glycol)-$\alpha,\omega$-diamine, une hétéropolylysine, un hétéropeptide, ou un homopeptide.

**9.** Procédé de préparation d'une homo ou hétéropolylysine dendrimère greffé selon l'une des revendications 6 à 8, dans lequel le pH du solvant est de 3 à 9.

**10.** Procédé de préparation d'une homo ou hétéropolylysine dendrimère greffé selon l'une des revendications 6 à 9, ladite polylysine dendrimère étant de génération 1, comprenant les étapes suivantes :

- l'addition de la L-lysine-NCA protégée en $N^{\varepsilon}$ à un amorceur dans un solvant aqueux, à un pH approprié, pour obtenir de la polylysine dendrimère protégée de génération 1 sous forme d'un précipité,
- la déprotection de la polylysine dendrimère protégée de génération 1 obtenue à l'étape précédente, pour obtenir de la polylysine dendrimère de génération 1.

**11.** Procédé de préparation d'une homopolylysine dendrimère greffé selon la revendication 10, ladite polylysine dendrimère étant de génération 1, dans lequel l'amorceur est de la L-lysine protégée en $N^{\varepsilon}$ ou non protégée.

**12.** Procédé de préparation d'une hétéropolylysine dendrimère greffé selon l'une des revendications 6 à 10, ladite polylysine dendrimère étant de génération 1, dans lequel l'amorceur est un poly(éthylène glycol)-$\alpha,\omega$-diamine, dont la masse moléculaire est de 100 Da à 10 000 Da.

**13.** Procédé de préparation d'une homopolylysine dendrimère greffé selon l'une des revendications 10 ou 11, ladite polylysine dendrimère étant de génération 1, comportant :

- une étape d'addition de L-lysine-NCA protégée en $N^{\varepsilon}$ par un groupement TFA, à une solution aqueuse de pH 6 à 8, sans ajout d'amorceur ou avec un amorceur L-lysine protégée en $N^{\varepsilon}$ par un groupement TFA, pour obtenir un précipité de polylysine dendrimère protégée de génération 1, et
- une étape de déprotection du polymère de polylysine obtenu à l'étape précédente pour obtenir une polylysine dendrimère de génération 1, linéaire, de masse moléculaire moyenne 1 450 Da, présentant une polydispersité de 1,2 et correspondant à un degré de polymérisation moyen de 8 unités de lysine.

**14.** Procédé de préparation d'une homo ou hétéropolylysine dendrimère greffé, selon l'une des revendications 6 à 9, dans lequel la polylysine dendrimère greffé est de génération n, n étant un nombre entier de 2 à 10, comportant :

- une étape d'addition de la L-lysine-NCA protégée en $N^{\varepsilon}$ à un amorceur constitué d'une polylysine dendrimère greffé de génération n-1, dans un solvant aqueux, à un pH approprié, pour obtenir de la polylysine dendrimère greffé protégée de génération n sous forme d'un précipité,

  ○ ladite polylysine dendrimère greffé de génération n-1 étant elle-même obtenue à partir de l'addition de la L-lysine-NCA protégée en $N^{\varepsilon}$ à un amorceur constitué d'une polylysine dendrimère greffé de génération n-2, dans un solvant aqueux, à un pH approprié, pour obtenir une polylysine dendrimère greffé protégée de génération n-1, et la déprotection de ladite polylysine,
  ○ ladite polylysine dendrimère greffé de génération n-2 étant elle-même obtenue comme indiqué à propos de la polylysine dendrimère greffé de génération n-1,
  ○ et lorsque n = 2, la polylysine dendrimère de génération 1 est telle que définie dans l'une des revendications 10 à 13, ladite polylysine dendrimère de génération 1 formant le coeur de la polylysine dendrimère de génération n,

- une étape de déprotection de la polylysine dendrimère greffé protégée de génération n pour obtenir la polylysine dendrimère greffé de génération n.

**15.** Procédé de préparation d'une homopolylysine dendrimère greffé selon la revendication 14, ladite polylysine dendrimère greffé étant de génération n, dans lequel la L-lysine-NCA est protégée en $N^ε$ par du TFA, le coeur de ladite homopolylysine dendrimère greffé de génération n étant formé d'une polylysine linéaire comprenant 8 résidus de L-lysine, telle que pouvant être obtenue par la mise en oeuvre du procédé de la revendication 13, et le degré de ramification de ladite homopolylysine dendrimère greffé de génération n étant de 40% à 100%.

**16.** Procédé de préparation d'une homopolylysine dendrimère greffé selon la revendication 14 ou 15, ladite polylysine dendrimère greffé étant de génération 2, comportant :

- une étape d'addition de la L-lysine-NCA protégée en $N^ε$ par du TFA à un amorceur constitué d'une polylysine dendrimère de génération 1,

  ○ ladite polylysine dendrimère de génération 1 étant telle que celle obtenue par la mise en oeuvre du procédé selon la revendication 13,
  ○ le rapport massique (L-lysine-NCA protégée en $N^ε$ par du TFA) / (polylysine dendrimère de génération 1) étant de 2,6 à 3,9,

ladite étape d'addition ayant lieu dans un solvant aqueux, à un pH approprié, pour obtenir une polylysine dendrimère protégée de génération 2,
- une étape de déprotection de la polylysine obtenue à l'étape précédente, pour obtenir une polylysine dendrimère de génération 2 ayant un poids moléculaire moyen de 8 600 Da, une polydispersité de 1,4, et 48 groupements $-NH_2$ externes libres.

**17.** Procédé de préparation d'une homopolylysine dendrimère greffé selon la revendication 14 ou 15, ladite polylysine dendrimère greffé étant de génération 3, comportant :

- une étape d'addition de la L-lysine-NCA protégée en $N^ε$ par du TFA à un amorceur constitué d'une polylysine dendrimère greffé de génération 2,

  ○ ladite polylysine dendrimère greffé de génération 2 étant telle que celle obtenue par la mise en oeuvre du procédé selon la revendication 16,
  ○ le rapport massique (L-lysine-NCA protégée en Nε par du TFA) / (polylysine dendrimère greffé de génération 2) étant de 2,6 à 3,9,

ladite étape d'addition ayant lieu dans un solvant aqueux, à un pH approprié, pour obtenir une polylysine dendrimère greffé protégée de génération 3,
- une étape de déprotection de la polylysine obtenue à l'étape précédente pour obtenir une polylysine dendrimère de génération 3 ayant un poids moléculaire moyen de 22 000 Da, une polydispersité de 1,4, et 123 groupements $-NH_2$ externes libres.

**18.** Procédé de préparation d'une homopolylysine dendrimère greffé selon la revendication 14 ou 15, ladite polylysine dendrimère greffé étant de génération 4, comportant :

- une étape d'addition de la L-lysine-NCA protégée en $N^ε$ par du TFA à un amorceur constitué d'une polylysine dendrimère greffé de génération 3,

  ○ ladite polylysine dendrimère greffé de génération 3 étant telle que celle obtenue par la mise en oeuvre du procédé selon la revendication 17,○ le rapport (L-lysine-NCA protégée en Nε par du TFA) / (polylysine dendrimère greffé de génération 3) étant de 2,6 à 3,9,

ladite étape d'addition ayant lieu dans un solvant aqueux, à un pH approprié, pour obtenir une polylysine dendrimère greffé protégée de génération 4,

- une étape de déprotection de la polylysine obtenue à l'étape précédente pour obtenir une polylysine dendrimère greffé de génération 4 ayant un poids moléculaire moyen de 65 300 Da, une polydispersité de 1,4, et 365, groupements $-NH_2$ externes libres.

**19.** Procédé de préparation d'une homopolylysine dendrimère greffé selon la revendication 14 ou 15, ladite polylysine dendrimère greffé étant de génération 5, comportant :

- une étape d'addition de la L-lysine-NCA protégée en $N^{\varepsilon}$ par du TFA à un amorceur constitué d'une polylysine dendrimère greffé de génération 4,

  ○ ladite polylysine dendrimère greffé de génération 4 étant telle que celle obtenue par la mise en oeuvre du procédé selon la revendication 18,
  ○ le rapport (L-lysine-NCA protégée en Nε par du TFA) / (polylysine dendrimère greffé de génération 4) étant de 2,6 à 3,9

ladite étape d'addition ayant lieu dans un solvant aqueux, à un pH approprié, pour obtenir une polylysine dendrimère greffé protégée de génération 5,
- une étape de déprotection de la polylysine obtenue à l'étape précédente pour obtenir une polylysine dendrimère greffé de génération 5 ayant un poids moléculaire moyen de 172 300 Da, une polydispersité de 1,5, et 963, groupements $-NH_2$ externes libres.

**20.** Procédé de préparation d'une homo- ou hétéropolylysine dendrimère greffé selon l'une quelconque des revendications 5 à 19, dans lequel l'amorceur est fixé de façon covalente au dendrimère greffé, ledit amorceur comprenant un produit marqueur détectable.

**21.** Polylysine dendrimère greffé telle que pouvant être obtenue par la mise en oeuvre d'un procédé selon l'une des revendications 6 à 20.

**22.** Polylysine dendrimère greffé selon la revendication 21, **caractérisée en ce que** les groupements $-NH_2$ externes sont liés de manière covalente ou non-covalente, à des groupes choisis parmi la liste comprenant des oses, des acides nucléiques, des protéines, ou des groupements portant des fonctions carboxyliques, sulfoniques, phosphoriques, des polyoxydes d'éthylène, et des chaînes hydrocarbonées ou perfluorohydrocarbonées, des aldéhydes ou leur précurseur, ou des fonctions réactives masquées telles que des groupements carbamoyle ou chloroéthylnitrosourées.

**23.** Polylysine dendrimère greffé selon la revendication 21 ou 22, **caractérisée en ce qu'**elle est fixée sur un support de manière covalente ou non-covalente.

**24.** Polylysine dendrimère greffé selon la revendication 21 ou 22, **caractérisée en ce qu'**elle est furtive vis-à-vis des systèmes immunitaires vis-à-vis desquels elle est mise en présence, et **en ce qu'**elle est avantageusement utilisée comme porteur d'haptènes ou d'antigènes contre lesquels les systèmes immunitaires réagissent pour former des anticorps.

**25.** Utilisation d'une polylysine dendrimère greffé selon la revendication 21 ou 22, pour la préparation de complexes antigène - polylysine dendrimère greffé ou de complexes haptène - polylysine dendrimère greffé, destinés à la production d'anticorps dirigés contre ledit antigène ou ledit haptène.

**26.** Composition de polylysines dendrimères greffés telle que pouvant être obtenue par la mise en oeuvre d'un procédé selon l'une des revendications 6 à 20.

**27.** Polylysine dendrimère greffé selon l'une des revendications 21 à 24, en tant qu'antibactérien ou antifongique, sous réserve qu'elle ne soit pas utilisée pour le traitement thérapeutique du corps humain ou animal.

**28.** Composition pharmaceutique, **caractérisée en ce qu'**elle comprend, à titre de substance active au moins une polylysine dendrimère greffé selon l'une des revendications 21 à 24, en association avec un véhicule pharmaceutiquement acceptable.

**29.** Utilisation d'une polylysine dendrimère greffé selon l'une des revendications 21 à 24, pour la préparation d'un

médicament destiné au traitement des infections bactériennes ou fongiques, ou des cancers.

**30.** Utilisation selon la revendication 29, dans laquelle les infections bactériennes sont choisies parmi les infections à bactéries GRAM- et GRAM+ appartenant aux familles choisies dans la liste comprenant les *Pseudomonadaceae*, les *Legionellaceae*, *les Enterobacteriaceae*, les *Vibrionaceae*, les *Pasteurellaceae,* les *Alcaligenaceae,* les *Brucellaceae*, les *Francisellaceae*, les *Neisseriaceae*, les *Micrococcaceae*.

## Claims

**1.** Use of activated α-amino acid monomers for the preparation of hydrophobic polypeptides in the form of precipitates, said polypeptides resulting from the polymerization of said activated α-amino acid monomers in an aqueous solvent and capable of being resolubilized in said solvent.

**2.** Use according to claim 1, in which the activated α-amino acids are chosen from α-amino acid N-carboxyanhydrides (NCA), α-amino acid N,N'-carbonyldiimidazoles, α-amino acid carbonyl sulphide, α-amino acid carbonic anhydride, and amino thioacids-oxidizing agents.

**3.** Use according to claim 1 or 2, of α-amino acid N-carboxyanhydrides (NCA) of the following formula (1):

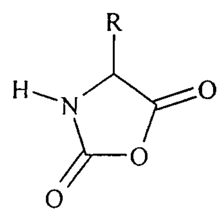

in which R represents a side chain of a natural or modified α-amino acid.

**4.** Use according to claim 2 or 3, of L-lysine-NCA monomers for the preparation of polylysine in aqueous solvent.

**5.** Method for the preparation of a grafted homo or heteropolylysine dendrimer, from a primer comprising at least one primary or secondary amine group, comprising a step of addition of an L-lysine-NCA monomer, and optionally one or more other α-amino-acid-NCA monomers, chosen from the list comprising L-ornithine-NCA, L-glutamic-acid-NCA and its γ-amide, L-aspartic-acid-NCA and its β-amide, L-diamino-2,4,-butyric-acid-NCA and its β-amide, L-tyrosine-NCA, L-serine-NCA, L-threonine-NCA, L-phenylalanine-NCA, L-valine-NCA, L-leucine-NCA, L-isoleucine-NCA, L-alanine-NCA, and glycine-NCA, to said primer in an aqueous solvent.

**6.** Method for the preparation of a grafted homo or heteropolylysine dendrimer according to claim 5, in which the monomer is L-lysine-NCA only.

**7.** Method for the preparation of a grafted homo or heteropolylysine dendrimer according to claim 5 or 6. in which the L-lysine-NCA is $N^{\varepsilon}$-protected, by a group chosen from:

-COH (Formyl), $-COCF_3$ (TFA), $-OCOC(CH_3)_3$ (Boc), $-COOCH_2\Phi$ (Z),

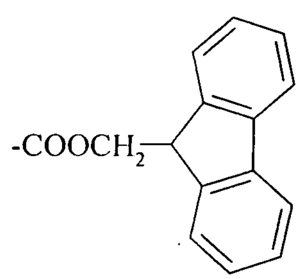
(Fmoc)

or -C(Φ)$_3$ (trityl).

**8.** Method for the preparation of a grafted homo or heteropolylysine dendrimer according to claim 6 or 7, in which the primer is chosen from L-lysine, L-ornithine, a homopolylysine, a poly(ethylene glycol)-$\alpha,\omega$-diamine, a heteropolylysine, a heteropeptide, or a homopeptide.

**9.** Method for the preparation of a grafted homo or heteropolylysine dendrimer according to one of claims 6 to 8, in which the pH of the solvent is 3 to 9.

**10.** Method for the preparation of a grafted homo or heteropolylysine dendrimer according to one of claims 6 to 9, said polylysine dendrimer being of generation 1, comprising the following steps:

- addition of the $N^{\varepsilon}$-protected L-lysine-NCA to a primer in an aqueous solvent, at an appropriate pH, in order to obtain a protected polylysine dendrimer of generation 1 in the form of a precipitate,
- deprotection of the protected polylysine dendrimer of generation 1 obtained in the previous step, in order to obtain the polylysine dendrimer of generation 1.

**11.** Method for the preparation of a grafted homopolylysine dendrimer according to claim 10, said polylysine dendrimer being of generation 1, in which the primer is $N^{\varepsilon}$ protected or unprotected L-lysine.

**12.** Method for the preparation of a grafted heteropolylysine dendrimer according to one of claims 6 to 10, said polylysine dendrimer being of generation 1, in which the primer is a poly(ethylene glycol)-$\alpha,\omega$-diamine, the molecular weight of which is 100 Da to 10,000 Da.

**13.** Method for the preparation of a grafted homopolylysine dendrimer according to one of claims 10 or 11, said polylysine dendrimer being of generation 1, comprising:

- a step of addition of L-lysine-NCA $N^{\varepsilon}$-protected by a TFA group, to an aqueous solution with a pH of 6 to 8, without addition of primer or with an L-lysine primer $N^{\varepsilon}$-protected by a TFA group, in order to obtain a precipitate of protected polylysine dendrimer of generation 1, and
- a step of deprotection of the polylysine polymer obtained in the previous step in order to obtain a linear polylysine dendrimer of generation 1, of molecular weight 1,450 Da, having a polydispersity index of 1.2 and corresponding to an average degree of polymerization of 8 units of lysine.

**14.** Method for the preparation of a grafted homo or heteropolylysine dendrimer according to one of claims 6 to 9, in which the grafted polylysine dendrimer is of generation n, n being an integer from 2 to 10, comprising:

- a step of addition of $N^{\varepsilon}$-protected L-lysine-NCA to a primer constituted by a grafted polylysine dendrimer of generation n-1, in an aqueous solvent, at an appropriate pH, in order to obtain the protected grafted polylysine dendrimer of generation n in the form of a precipitate,
    - ○ said grafted polylysine dendrimer of generation n-1 being itself obtained from the addition of $N^{\varepsilon}$-protected L-lysine-NCA to a primer constituted by a grafted polylysine dendrimer of generation n-2, in an aqueous solvent, at an appropriate pH. in order to obtain a protected grafted polylysine dendrimer of generation n-1, and the deprotection of said polylysine,
    - ○ said grafted polylysine dendrimer of generation n-2 being itself obtained as indicated in relation to the grafted polylysine dendrimer of generation n-1,
    - ○ and when n = 2, the polylysine dendrimer of generation 1 is as defined in one of claims 10 to 13, said polylysine dendrimer of generation 1 forming the core of the polylysine dendrimer of generation n,
- a step of deprotection of the protected grafted polylysine dendrimer of generation n to obtain the grafted polylysine dendrimer of generation n.

**15.** Method for the preparation of a grafted homopolylysine dendrimer according to claim 14, said grafted polylysine dendrimer being of generation n, in which the L-lysine-NCA is $N^{\varepsilon}$-protected by TFA, the core of said grafted homopolylysine dendrimer of generation n being formed from a linear polylysine comprising 8 residues of L-lysine, such as can be obtained by the implementation of the method of claim 13, and the degree of branching of said grafted homopolylysine dendrimer of generation n being 40% to 100%.

**16.** Method for the preparation of a grafted homopolylysine dendrimer according to claim 14 or 15, said grafted polylysine dendrimer being of generation 2, comprising:

- a step of addition of the L-lysine-NCA $N^{\varepsilon}$-protected by TFA to a primer constituted by a polylysine dendrimer of generation 1,

  ○ said polylysine dendrimer of generation 1 being such as that obtained by the implementation of the method according to claim 13,
  ○ the mass ratio (L-lysine-NCA $N^{\varepsilon}$-protected by TFA) / (polylysine dendrimer of generation 1) being 2.6 to 3.9,

said step of addition taking place in an aqueous solvent, at an appropriate pH, in order to obtain a protected polylysine dendrimer of generation 2,
- a step of deprotection of the polylysine obtained in the previous step, in order to obtain a polylysine dendrimer of generation 2 having an average molecular weight of 8,600 Da, a polydispersity of 1.4. and 48, free external $-NH_2$ groups.

**17.** Method for the preparation of a grafted homopolylysine dendrimer according to claim 14 or 15, said grafted polylysine dendrimer being of generation 3, comprising:

- a step of addition of the L-lysine-NCA $N^{\varepsilon}$-protected by TFA to a primer constituted by a generation 2 grafted polylysine dendrimer,

  ○ said generation 2 grafted polylysine dendrimer being such as that obtained by the implementation of the method according to claim 16,
  ○ the mass ratio (L-lysine-NCA $N^{\varepsilon}$-protected by TFA) / (generation 2 grafted polylysine dendrimer) being 2.6 to 3.9,

said step of addition taking place in an aqueous solvent, at an appropriate pH, in order to obtain a protected grafted polylysines dendrimer of generation 3,
- a step of deprotection of the polylysine obtained in the previous step, in order to obtain a polylysine dendrimer of generation 3 having an average molecular weight of 22,000 Da, a polydispersity of 1.4. and 123, free external $-NH_2$ groups.

**18.** Method for the preparation of a grafted homopolylysine dendrimer according to claim 14 or 15, said grafted polylysine dendrimer being of generation 4, comprising:

- a step of addition of the L-lysine-NCA $N^{\varepsilon}$-protected by TFA to a primer constituted by a generation 3 grafted polylysine dendrimer,

  ○ said generation 3 grafted polylysine dendrimer being such as that obtained by the implementation of the method according to claim 17,
  ○ the ratio (L-lysine-NCA $N^{\varepsilon}$ protected by TFA) / (generation 3 grafted polylysine dendrimer) being 2.6 to 3.9,

said step of addition taking place in an aqueous solvent, at an appropriate pH, in order to obtain a protected grafted polylysine dendrimer of generation 4,
- a step of deprotection of the polylysine obtained in the previous step, in order to obtain a grafted polylysine dendrimer of generation 4 having an average molecular weight of 65,300 Da, a polydispersity of 1.4, and 365, free external $-NH_2$ groups.

**19.** Method for the preparation of a grafted homopolylysine dendrimer according to claim 14 or 15, said grafted polylysine dendrimer being of generation 5, comprising:

- a step of addition of the L-lysine-NCA $N^{\varepsilon}$-protected by TFA to a primer constituted by a generation 4 grafted polylysine dendrimer,

  ○ said generation 4 grafted polylysine dendrimer being such as that obtained by the implementation of the method according to claim 18,
  ○ the ratio (L-lysine-NCA $N^{\varepsilon}$-protected by TFA) / (generation 4 grafted polylysine dendrimer) being 2.6 to 3.9,

said step of addition taking place in an aqueous solvent, at an appropriate pH, in order to obtain a protected polylysine dendrimer of generation 5,
- a step of deprotection of the polylysine obtained in the previous step, in order to obtain a grafted polylysine dendrimer of generation 5 having an average molecular weight of 172,300 Da, a polydispersity of 1.5, and 963, free external $-NH_2$ groups.

**20.** Method for the preparation of a grafted homo- or hetero polylysine dendrimer according to any one of claims 5 to 19, in which the primer is fixed covalently to the grafted dendrimer, said primer comprising a detectable marker product.

**21.** Grafted polylysine dendrimer such as can be obtained by the implementation of a method according to one of claims 6 to 20.

**22.** Grafted polylysine dendrimer according to claim 21, **characterized in that** the external $-NH_2$ groups are bonded covalently or non-covalently to groups chosen from the list comprising monosaccharides, nucleic acids, proteins, or groups having carboxylic, sulphonic and phosphoric functions, ethylene polyoxides and hydrocarbonated or perfluorohydrocarbonated chains, aldehydes or their precursor, or masked reactive functions such as carbamoyl or chloroethylnitrosourea groups.

**23.** Grafted polylysine dendrimer according to claim 21 or 22, **characterized in that** it is fixed covalently or non-covalently onto a support.

**24.** Grafted polylysine dendrimer according to claim 21 or 22, **characterized in that** it is furtive vis-à-vis the immune systems with which it is brought into contact, and **in that** it is advantageously used as a carrier of haptens or antigens against which the immune systems react to form antibodies.

**25.** Use of a grafted polylysine dendrimer according to claim 21 or 22, for the preparation of antigen-grafted polylysine dendrimer complexes or hapten-grafted polylysine dendrimer complexes, intended for the production of antibodies directed against said antigen or said hapten.

**26.** Composition of grafted polylysine dendrimers such as can be obtained by the implementation of a method according to one of claims 6 to 20.

**27.** Grafted polylysine dendrimer according to one of the claims 21 to 24, as an antibacterial or antifungal, with the proviso that it is not used for the therapeutic treatment of the human or animal body.

**28.** Pharmaceutical composition, **characterized in that** it comprises, as an active ingredient at least one grafted polylysine dendrimer according to one of claims 21 to 24, in combination with a pharmaceutically acceptable vehicle.

**29.** Use of a grafted polylysine dendrimer according to one of claims 21 to 24, for the preparation of a medicament intended for the treatment of bacterial or fungal infections, or cancers.

**30.** Use according to claim 29, in which the bacterial infections are chosen from infections by GRAM- and GRAM+ bacteria belonging to the families chosen from the list comprising *Pseudomonadaceae, Legionellaceae, Enterobacteriaceae, Vibrionaceae, Pasteurellaceae, Alcaligenaceae, Brucellaceae, Francisellaceae, Neisseriaceae, Micrococcacecae*.

## Patentansprüche

**1.** Verwendung von aktivierten α-Aminosäuremonomeren zur Herstellung von hydrophoben Polypeptiden in Form von Präzipitaten, wobei die Polypeptide aus der Polymerisation der aktivierten α-Aminosäuremonomere in wässrigem Lösemittel resultieren und geeignet sind, erneut in dem Lösemittel gelöst zu werden.

**2.** Verwendung nach Anspruch 1, wobei die aktivierten α-Aminosäuren aus α-Aminosäure-N-carboxyanhydriden (NCA), α-Aminoasäure-N,N'-carbonyldiimidazol, α-Aminosäure-carbonylsulfid, α-Aminosäure-carbonanhydrid und Aminothiosäure-Oxidationsmitteln ausgewählt sind.

**3.** Verwendung nach Anspruch 1 oder 2 von α-Aminosäure-N-carboxyanhydriden (NCA) der folgenden Formel (1):

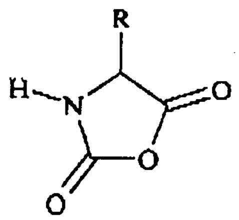

wobei R für eine Seitenkette einer natürlichen oder modifizierten α-Aminosäure steht.

**4.** Verwendung nach Anspruch 2 oder 3 von L-Lysin-NCA-Monomeren zur Herstellung von Polylysin in wässrigem Lösemittel.

**5.** Verfahren zur Herstellung eines gepfropften Homo- oder Heteropolylysindendrimers ausgehend von einem Primer, der mindestens eine primäre oder sekundäre Amingruppe umfasst, das einen Schritt des Zugebens eines L-Lysin-NCA-Monomers und gegebenenfalls eines oder mehrerer anderer α-Aminosäure-NCA-Monomere, ausgewählt aus der Liste, die L-Ornithin-NCA, L-Glutaminsäure-NCA und dessen γ-Amid, L-Asparaginsäure-NCA und dessen β-Amid, L-Diamino-2,4,-buttersäure-NCA und dessen β-Amid, L-Tyrosin-NCA, L-Serin-NCA, L-Threonin-NCA, L-Phenylalanin-NCA, L-Valin-NCA, L-Leucin-NCA, L-Isoleucin-NCA, L-Alanin-NCA und Glycin-NCA umfasst, zu dem Primer in einem wässrigen Lösemittel umfasst.

**6.** Verfahren zur Herstellung eines gepfropften Homo- oder Heteropolylysindendrimers nach Anspruch 5, wobei das Monomer ausschließlich L-Lysin-NCA ist.

**7.** Verfahren zur Herstellung eines gepfropften Homo- oder Heteropolylysindendrimers nach Anspruch 5 oder 6, wobei das L-Lysin-NCA an $N^{\varepsilon}$ durch eine Gruppe geschützt ist, die ausgewählt ist aus:

-COH (Formyl), $-COCF_3$ (TFA), $-OCOC(CH_3)_3$ (Boc), - $COOCH_2\Phi$ (Z),

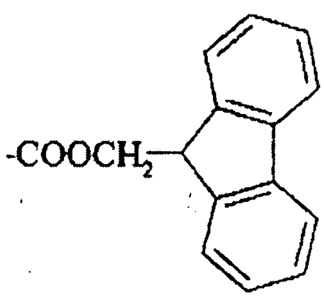

(Fmoc)

oder $-C(\Phi)_3$ Ttrityl).

**8.** Verfahren zur Herstellung eines gepfropften Homo- oder Heteropolylysindendrimers nach Anspruch 6 oder 7, wobei der Primer aus L-Lysin, L-Ornithin, einem Homopolylysin, einem Poly(ethylenglycol)-α,ω-diamin, einem Heteropolylysin, einem Heteropeptid oder einem Homopeptid ausgewählt ist.

**9.** Verfahren zur Herstellung eines gepfropften Homo- oder Heteropolylysindendrimers nach einem der Ansprüche 6 bis 8, wobei der pH-Wert des Lösemittels 3 bis 9 beträgt.

**10.** Verfahren zur Herstellung eines gepfropften Homo- oder Heteropolylysindendrimers nach einem der Ansprüche 6 bis 9, wobei das Polylysindendrimer ein Polylysindendrimer 1. Generation ist, das die folgenden Schritte umfasst:

- das Zugeben von L-Lysin-NCA, das an $N^{\varepsilon}$ geschützt ist, zu einem Primer in einem wässrigen Lösemittel, bei einem geeigneten pH-Wert, um geschütztes Polylysindendrimer 1. Generation in Form eines Präzipitats zu erhalten,
- das Entschützen des geschützten Polylysindendrimers 1. Generation, das im vorhergehenden Schritt erhalten wurde, um das Polylysindendrimer 1. Generation zu erhalten.

**11.** Verfahren zur Herstellung eines gepfropften Homopolylysindendrimers nach Anspruch 10, wobei das Polylysindendrimer ein Polylysindendrimer 1. Generation ist, wobei der Primer L-Lysin ist, das an $N^{\varepsilon}$ geschützt oder nicht geschützt ist.

**12.** Verfahren zur Herstellung eines gepfropften Heteropolylysindendrimers nach einem der Ansprüche 6 bis 10, wobei das Polylysindendrimer ein Polylysindendrimer 1. Generation ist, wobei der Primer ein Poly(ethylenglycol)-$\alpha,\omega$-diamin ist, dessen Molekülmasse 100 Da bis 10.000 Da beträgt.

**13.** Verfahren zur Herstellung eines gepfropften Homopolylysindendrimers nach einem der Ansprüche 10 oder 11, wobei das Polylysindendrimer ein Polylysindendrimer 1. Generation ist, umfassend:

- einen Schritt des Zugebens von L-Lysin-NCA, das an $N^{\varepsilon}$ durch eine TFA-Gruppe geschützt ist, zu einer wässrigen Lösung mit einem pH-Wert von 6 bis 8, ohne Zufügen eines Primer oder mit einem Primer L-Lysin, das an $N^{\varepsilon}$ durch eine TFA-Gruppe geschützt ist, um ein Präzipitat aus geschütztem Polylysindendrimer 1. Generation zu erhalten, und
- einen Schritt des Entschützens des Polylysinpolymers, das im vorhergehenden Schritt erhalten wurde, um ein lineares Polylysindendrimer 1. Generation zu erhalten, mit einer mittleren Molekülmasse von 1.450 Da, das eine Polydispersität von 1,2 aufweist und einem mittleren Polymerisationsgrad von 8 Lysineinheiten entspricht.

**14.** Verfahren zur Herstellung eines gepfropften Homo- oder Heteropolylysindendrimers nach einem der Ansprüche 6 bis 9, wobei das gepfropfte Polylysindendrimer ein Polylysindendrimer n. Generation ist, wobei n eine ganze Zahl zwischen 2 und 10, ist, umfassend:

- einen Schritt des Zugebens von L-Lysin-NCA, das an $N^{\varepsilon}$ geschützt ist, zu einem Primer, der aus einem gepfropften Polylysindendrimer n-1. Generation besteht, in einem wässrigen Lösemittel, bei einem geeigneten pH-Wert, um geschütztes gepfropftes Polylysindendrimer n. Generation in Form eines Präzipitats zu erhalten,

  ○ wobei das gepfropfte Polylysindendrimer n-1. Generation selbst ausgehend von der Zugabe von L-Lysin-NCA, das an $N^{\varepsilon}$ geschützt.ist, zu einem Primer, der aus einem gepfropften Polylysindendrimer n-2. Generation besteht, in einem wässrigen Lösemittel, bei einem geeigneten pH-Wert, um ein geschütztes gepfropftes Polylysindendrimer n-1. Generation zu erhalten, und der Entschützung des Polylysins erhalten wurde,○ wobei das gepfropfte Polylysindendrimer n-2. Generation selbst so erhalten wurde, wie es für das gepfropfte Polylysindendrimer n-1. Generation angegeben ist,
  ○ und wenn n = 2, das Polylysindendrimer 1. Generation so ist, wie in einem der Ansprüche 10 bis 13 definiert, wobei das Polylysindendrimer 1. Generation den Kern des Polylysindendrimers n. Generation bildet,

- einen Schritt des Entschützens des geschützten gepfropften Polylysindendrimers n. Generation, um das gepfropfte Polylysindendrimer n. Generation zu erhalten.

**15.** Verfahren zur Herstellung eines gepfropften Homopolylysindendrimers nach Anspruch 14, wobei das gepfropfte Polylysindendrimer ein Polylysindendrimer n. Generation ist, wobei das L-Lysin-NCA an $N^{\varepsilon}$ durch TFA geschützt ist, wobei der Kern des gepfropften Homopolylysindendrimers n. Generation aus einem linearen Polylysin gebildet ist, das 8 L-Lysinreste umfasst, das durch das Durchführen des Verfahrens aus Anspruch 13 erhalten werden kann, und der Verzweigungsgrad des gepfropften Homopolylysindendrimers n. Generation 40 % bis 100 % beträgt.

**16.** Verfahren zur Herstellung eines gepfropften Homopolylysindendrimers nach Anspruch 14 oder 15, wobei das gepfropfte Polylysindendrimer ein Polylysindendrimer 2. Generation ist, umfassend:

- einen Schritt des Zugebens von L-Lysin-NCA, das an $N\varepsilon$ durch TFA geschützt ist, zu einem Primer, der aus einem Polylysindendrimer 1. Generation besteht,

○ wobei das Polylysindendrimer 1. Generation so ist, wie dasjenige, das durch das Durchführen des Verfahrens nach Anspruch 13 erhalten wurde,
○ wobei das Masseverhältnis (L-Lysin-NCA, das an Nε durch TFA geschützt ist)/(Polylysindendrimer 1. Generation) 2,6 bis 3,9 beträgt,

wobei der Schritt des Zugebens in einem wässrigen Lösemittel bei einem geeigneten pH-Wert stattfindet, um ein geschütztes Polylysindendrimer 2. Generation zu erhalten,
- einen. Schritt des Entschützens des Polylysins, das im vorhergehenden Schritt erhalten wurde, um ein Polylysindendrimer 2. Generation zu erhalten, das ein mittleres Molekulargewicht von 8.600 Da, eine Polydispersität von 1,4, und 48 freie externe -$NH_2$-Gruppen hat.

**17.** Verfahren zur Herstellung eines gepfropften Homopolylysindendrimers nach Anspruch 14 oder 15, wobei das gepfropfte Polylysindendrimer ein Polylysindendrimer 3. Generation ist, umfassend:

- einen Schritt des Zugebens von L-Lysin-NCA, das an $N^\varepsilon$ durch TFA geschützt ist, zu einem Primer, der aus einem gepfropften Polylysindendrimer 2. Generation besteht,

○ wobei das gepfropfte Polylysindendrimer 2. Generation so ist, wie dasjenige, das durch das Durchführen des Verfahrens nach Anspruch 16 erhalten wurde,
○ wobei das Masseverhältnis (L-Lysin-NCA, das an Nε durch TFA geschützt ist) / (gepfropftes Polylysindendrimer 2. Generation) 2,6 bis 3,9 beträgt,

wobei der Schritt des Zugebens in einem wässrigen Lösemittel bei einem geeigneten pH-Wert stattfindet, um ein geschütztes gepfropftes Polylysindendrimer 3. Generation zu erhalten,
- einen Schritt des Entschützens des Polylysins, das im vorhergehenden Schritt erhalten wurde, um ein Polylysindendrimer 3. Generation zu erhalten, das ein mittleres Molekulargewicht von 22.000 Da, eine Polydispersität von 1,4, und 123 freie externe -$NH_2$-Gruppen hat.

**18.** Verfahren zur Herstellung eines gepfropften Homopolylysindendrimers nach Anspruch 14 oder 15, wobei das gepfropfte Polylysindendrimer ein Polylysindendrimer 4. Generation ist, umfassend:

- einen Schritt des Zugebens von L-Lysin-NCA, das an $N^\varepsilon$ durch TFA geschützt ist, zu einem Primer, der aus einem gepfropften Polylysindendrimer 3. Generation besteht,

○ wobei das gepfropfte Polylysindendrimer 3. Generation so ist, wie dasjenige, das durch das Durchführen des Verfahrens nach Anspruch 17 erhalten wurde,
○ wobei das Verhältnis (L-Lysin-NCA, das an Nε durch TFA geschützt ist) / (gepfropftes Polylysindendrimer 3. Generation) 2,6 bis 3,9 beträgt,

wobei der Schritt des Zugebens in einem wässrigen Lösemittel bei einem geeigneten pH-Wert stattfindet, um ein geschütztes gepfropftes Polylysindendrimer 4. Generation zu erhalten,
- einen Schritt des Entschützens des Polylysins, das im vorhergehenden Schritt erhalten wurde, um ein gepfropftes Polylysindendrimer 4. Generation zu erhalten, das ein mittleres Molekulargewicht von 65.300 Da, eine Polydispersität von 1,4, und 365 freie externe -$NH_2$-Gruppen hat.

**19.** Verfahren zur Herstellung eines gepfropften Homopolylysindendrimers nach Anspruch 14 oder 15, wobei das gepfropfte Polylysindendrimer ein Polylysindendrimer 5. Generation ist, umfassend:

- einen Schritt des Zugebens von L-Lysin-NCA, das an $N^\varepsilon$ durch TFA geschützt ist, zu einem Primer, der aus einem gepfropften Polylysindendrimer 4. Generation besteht,

○ wobei das gepfropfte Polylysindendrimer 4. Generation so ist, wie dasjenige, das durch das Durchführen des Verfahrens nach Anspruch 18 erhalten wurde,
○ wobei das Verhältnis (L-Lysin-NCA, das an Nε durch TFA geschützt ist)/(gepfropftes Polylysindendrimer 4. Generation) 2,6 bis 3,9 beträgt,

wobei der Schritt des Zugebens in einem wässrigen Lösemittel bei einem geeigneten pH-Wert stattfindet, um ein geschütztes gepfropftes Polylysindendrimer 5. Generation zu erhalten,

- einen Schritt des Entschützens des Polylysins, das im vorhergehenden Schritt erhalten wurde, um ein gepfropftes Polylysindendrimer 5. Generation zu erhalten, das ein mittleres Molekulargewicht von 172.300 Da, eine Polydispersität von 1,5, und 963 freie externe $-NH_2$-Gruppen hat.

**20.** Verfahren zur Herstellung eines gepfropften Homo- oder Heteropolylysindendrimers nach irgendeinem der Ansprüche 5 bis 19, wobei der Primer auf kovalente Weise an dem gepfropften Dendrimer fixiert ist, wobei der Primer ein nachweisbares Markerprodukt umfasst.

**21.** Gepfropftes Polylysindendrimer, das durch das Durchführen eines Verfahrens nach einem der Ansprüche 6 bis 20 erhalten werden kann.

**22.** Gepfropftes Polylysindendrimer nach Anspruch 21, **dadurch gekennzeichnet, dass** die externen $-NH_2$-Gruppen auf kovalente oder nicht kovalente Weise an Gruppen gebunden sind, die aus der Liste ausgewählt sind, die Osen, Nukleinsäuren, Proteine, oder Gruppen, die carboxylische, sulfonische, phosphorische Funktionen tragen, Polyethylenoxide und Kohlenwasserstoff- oder Perfluorkohlenwasserstoffketten, Aldehyde oder deren Vorläufer oder maskierte reaktive Funktionen, wie etwa Carbamoyl- oder Chlorethylnitroharnstoffgruppen umfasst.

**23.** Gepfropftes Polylysindendrimer nach Anspruch 21 oder 22, **dadurch gekennzeichnet, dass** es auf kovalente oder nicht kovalente Weise auf einem Träger fixiert ist.

**24.** Gepfropftes Polylysindendrimer nach Anspruch 21 oder 22, **dadurch gekennzeichnet, dass** es gegenüber Immunsystemen, in deren Gegenwart es gebracht wird, furtiv ist, und **dadurch**, dass es vorteilhafterweise als Träger von Haptenen oder Antigenen verwendet wird, gegen die die Immunsysteme reagieren, um Antikörper zu bilden.

**25.** Verwendung eines gepfropften Polylysindendrimers nach Anspruch 21 oder 22 zur Herstellung von Komplexen aus Antigen und gepfropftem Polylysindendrimer oder von Komplexen aus Hapten und gepfropftem Polylysiridendrimer, die der Produktion von Antikörpern dienen, die gegen das Antigen oder das Hapten gerichtet sind.

**26.** Zusammensetzung aus gepfropften Polylysindendrimeren, die durch das Durchführen eines Verfahrens nach einem der Ansprüche 6 bis 20 erhalten werden kann.

**27.** Gepfropftes Polylysindendrimer nach einem der Ansprüche 21 bis 24 als Bakterizid oder Fungizid unter der Bedingung, dass es nicht zur therapeutischen Behandlung des menschlichen oder tierischen Körpers verwendet wird.

**28.** Pharmazeutische Zusammensetzung, **dadurch gekennzeichnet, dass** sie als Wirkstoff mindestens ein gepfropftes Polylysindendrimer nach einem der Ansprüche 21 bis 24 in Verbindung mit einem pharmazeutisch unbedenklichen Vehikel umfasst.

**29.** Verwendung eines gepfropften Polylysindendrimers nach einem der Ansprüche 21 bis 24 zur Herstellung eines Medikaments, das der Behandlung bakterieller Infektionen oder Pilzinfektionen oder von Krebsarten dient.

**30.** Verwendung nach Anspruch 29, wobei die bakteriellen Infektionen aus den Infektionen mit gramnegativen und grampositiven Bakterien ausgewählt sind, die zu den Familien gehören, die aus der Liste ausgewählt sind, die *Pseudomonadaceae, Legionellaceae, Enterobacteriaceae, Vibrionaceae, Pasteurellaceae, Alcaligenaceae, Brucellaceae, Francisellaceae, Neisseriaceae, Micrococcaceae* umfasst.

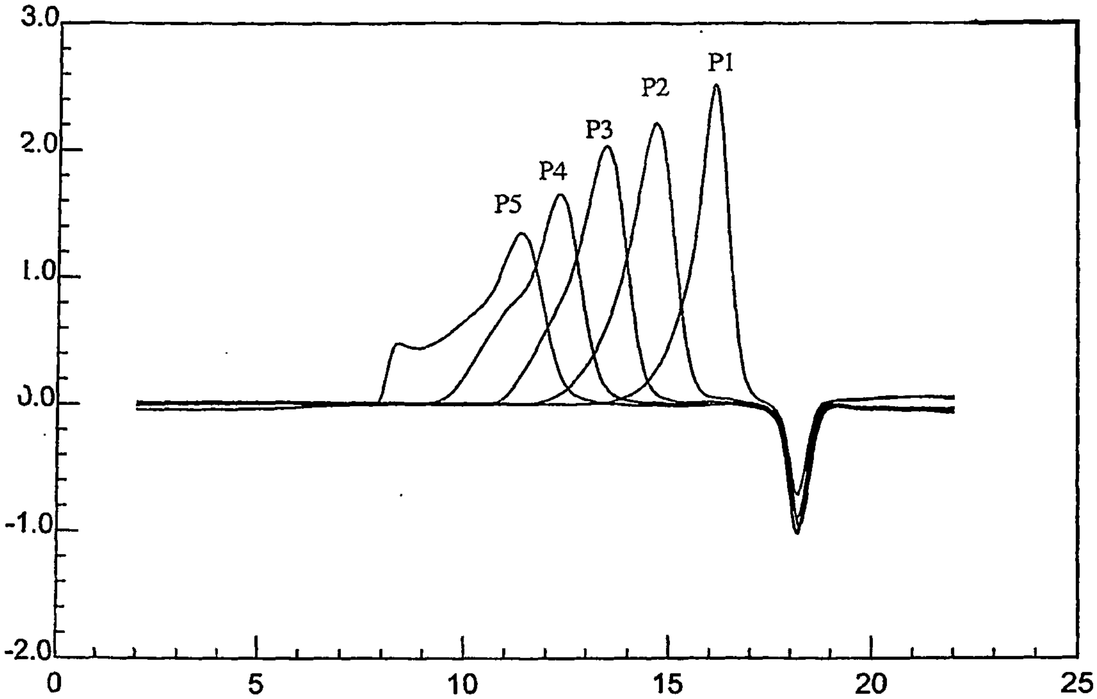


**Figure 1**

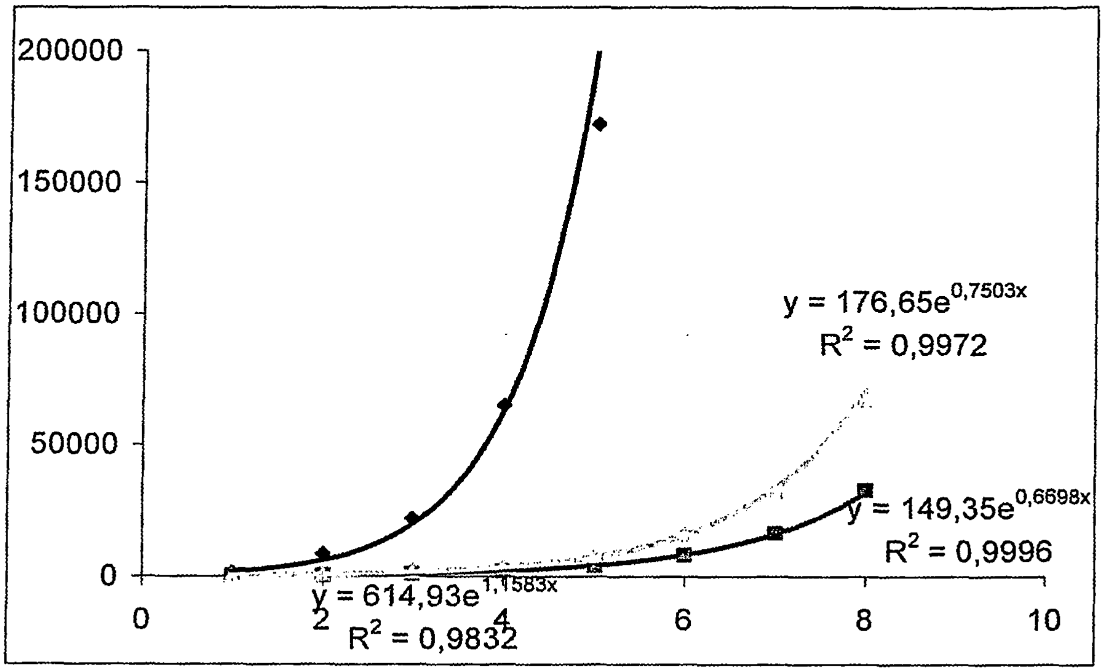


Figure 2A

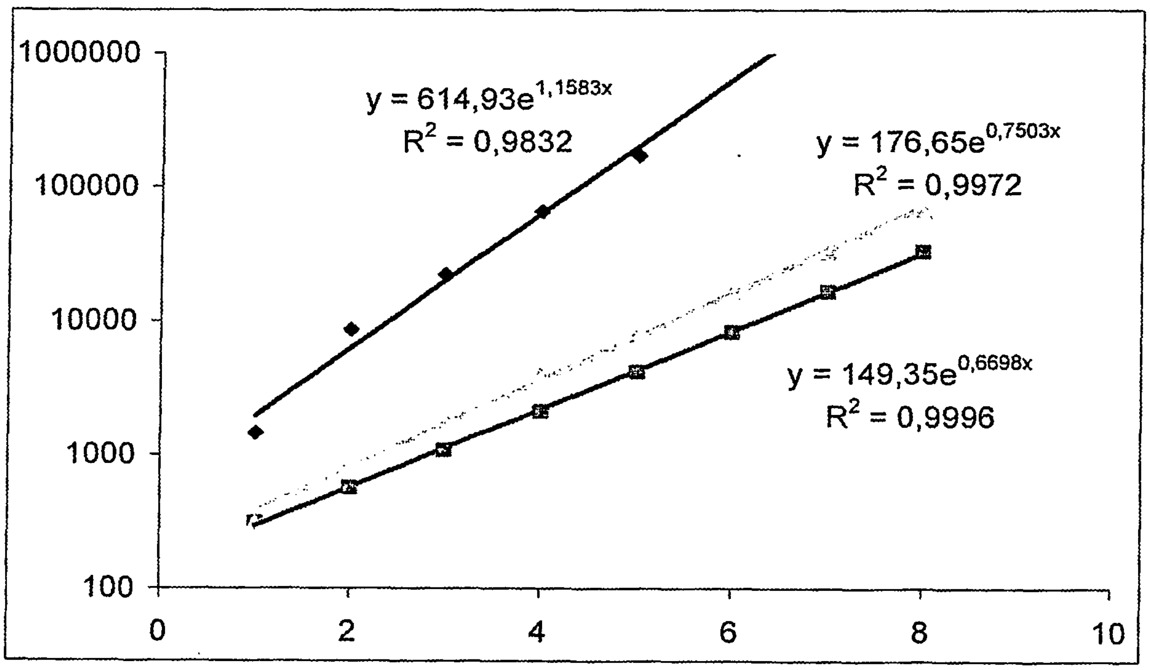


Figure 2B

## Figure 3

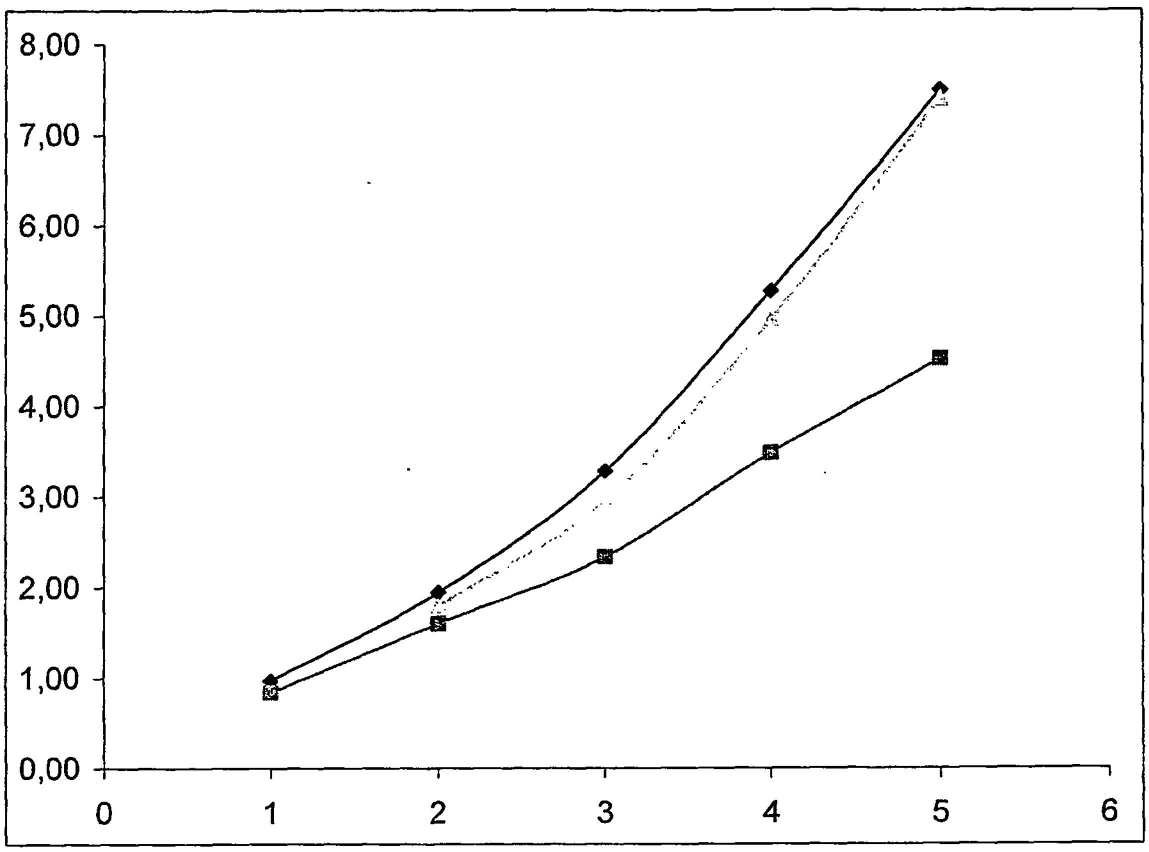

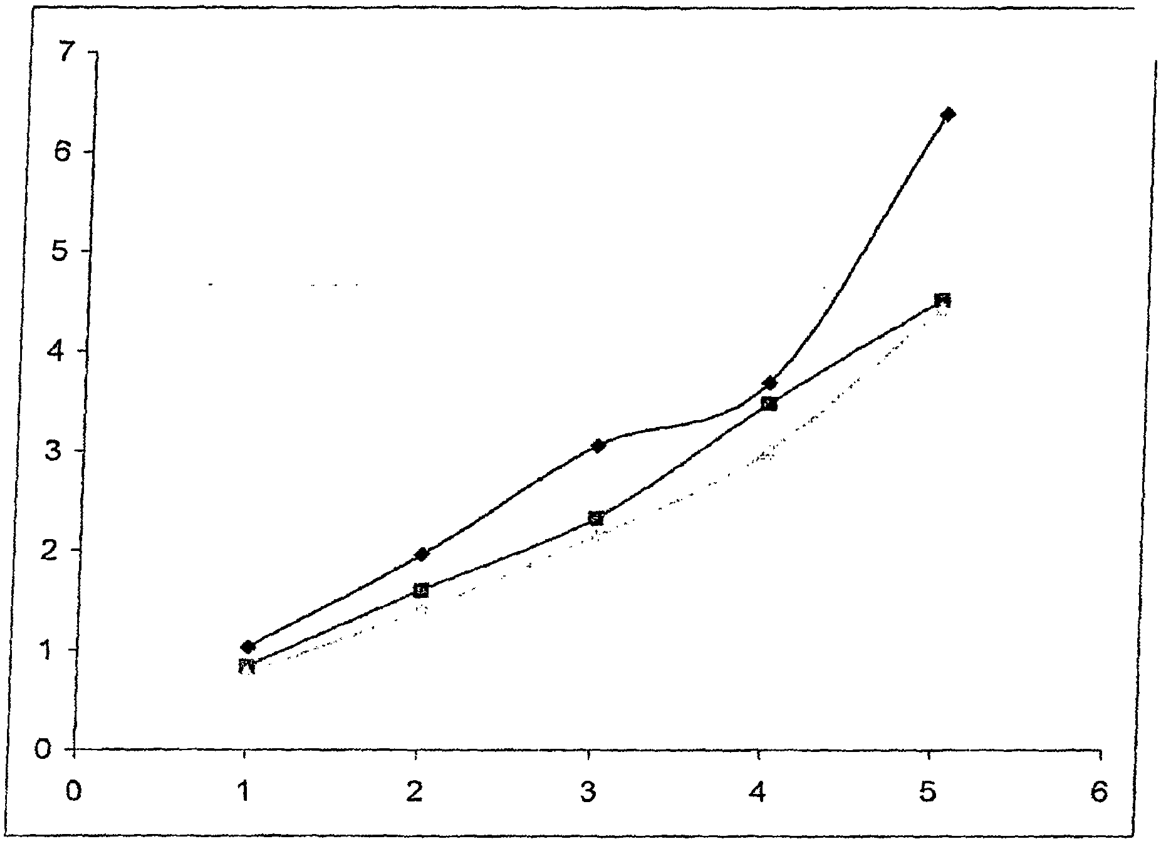


Figure 4

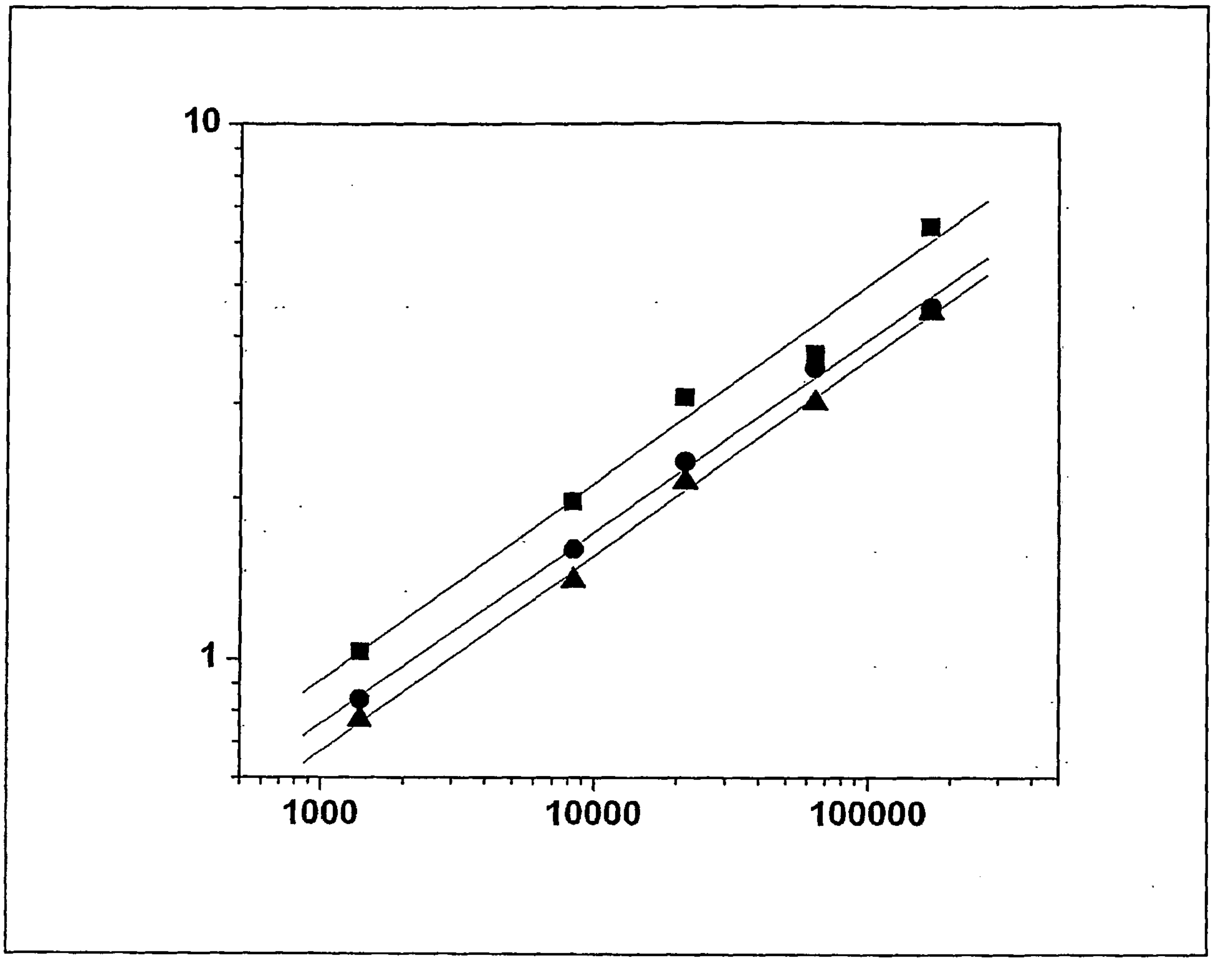


[Fig. 5]

Figure 6A

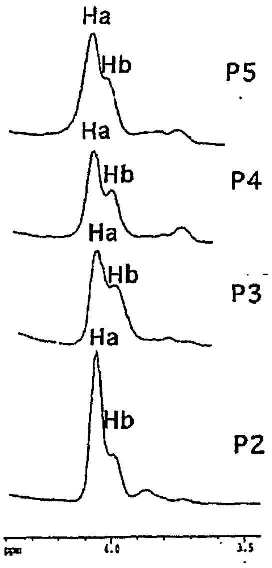


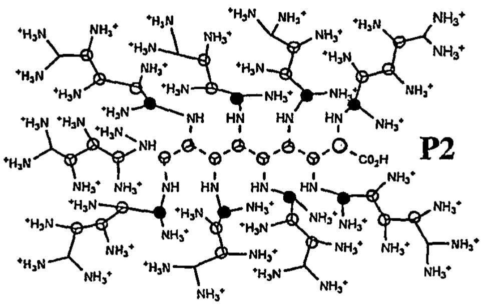
P2

Figure 6E

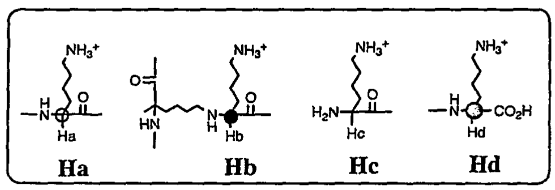
Ha Hb Hc Hd

Figure 6I

Figure 6B

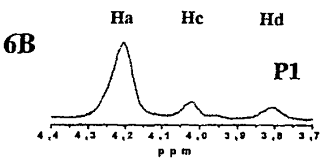


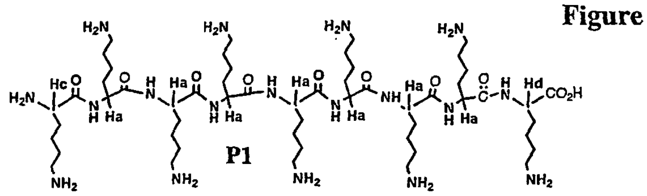
P1

Figure 6F

Figure 6C

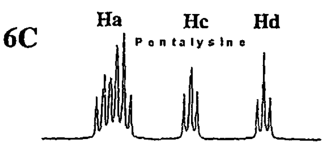


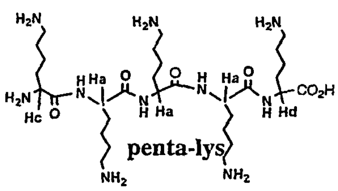
penta-lys

Figure 6G

Figure 6D

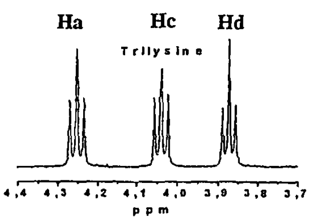


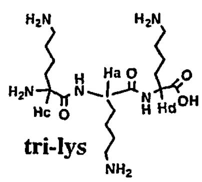
tri-lys

Figure 6H

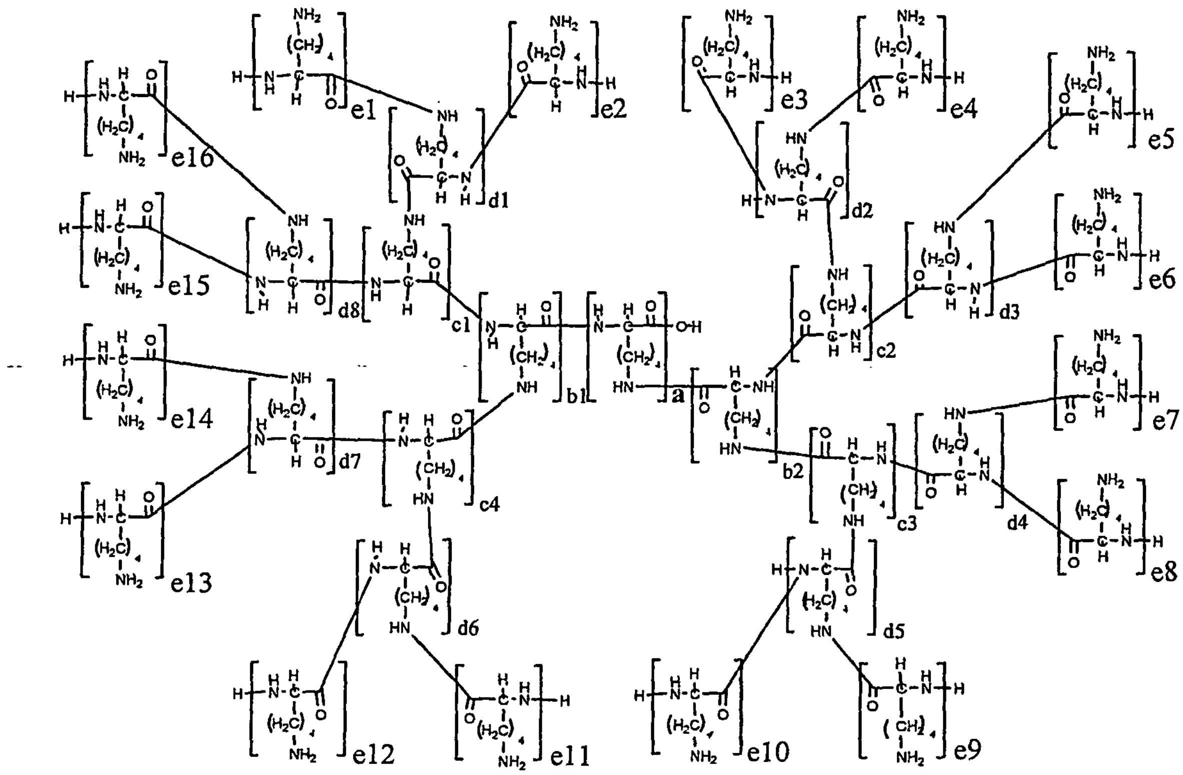

Figure 7

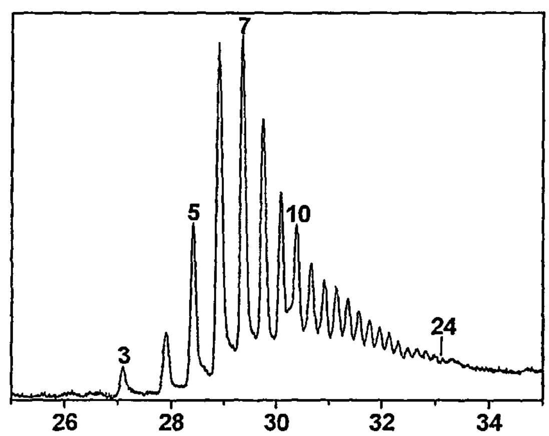


Figure 8

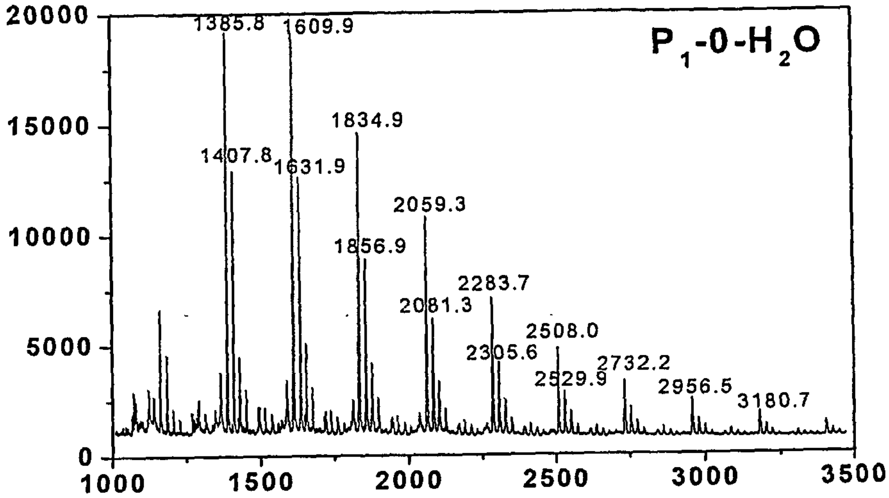


Figure 9A

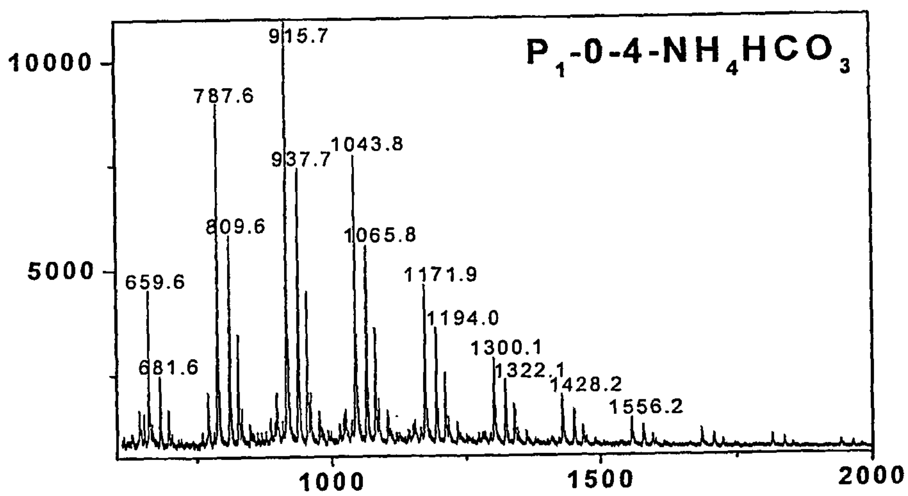


Figure 9B

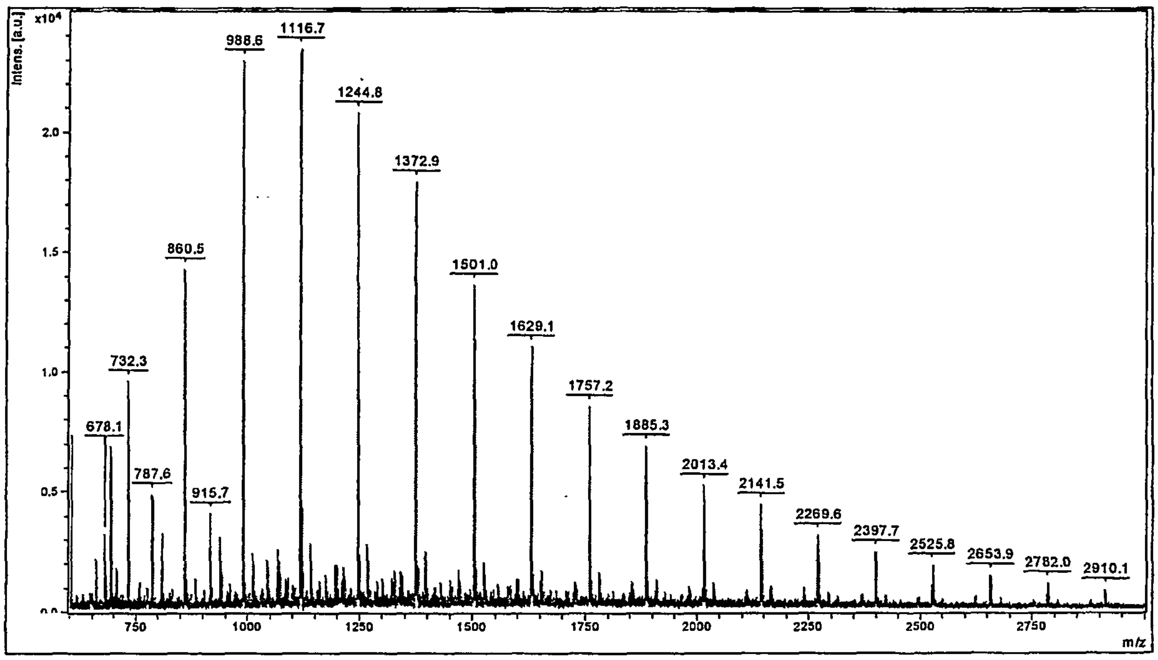
Intens. [a.u.]
x10⁴
2.0
1.5
1.0
0.5
0.0
988.6
1116.7
1244.8
1372.9
860.5
1501.0
1629.1
732.3
1757.2
678.1
1885.3
787.6
915.7
2013.4
2141.5
2269.6
2397.7
2525.8
2653.9
2782.0
2910.1
750
1000
1250
1500
1750
2000
2250
2500
2750
m/z

Figure 10

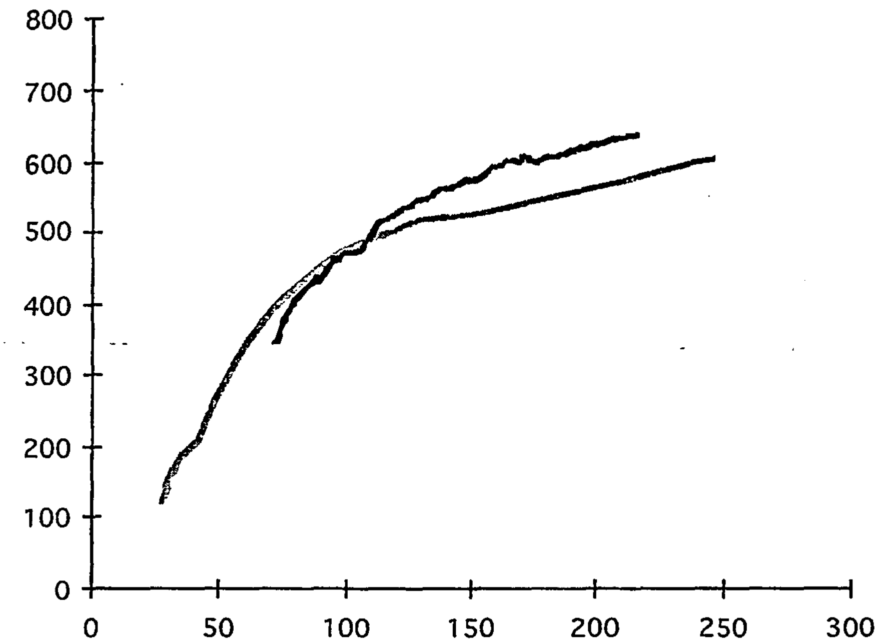


Figure 11

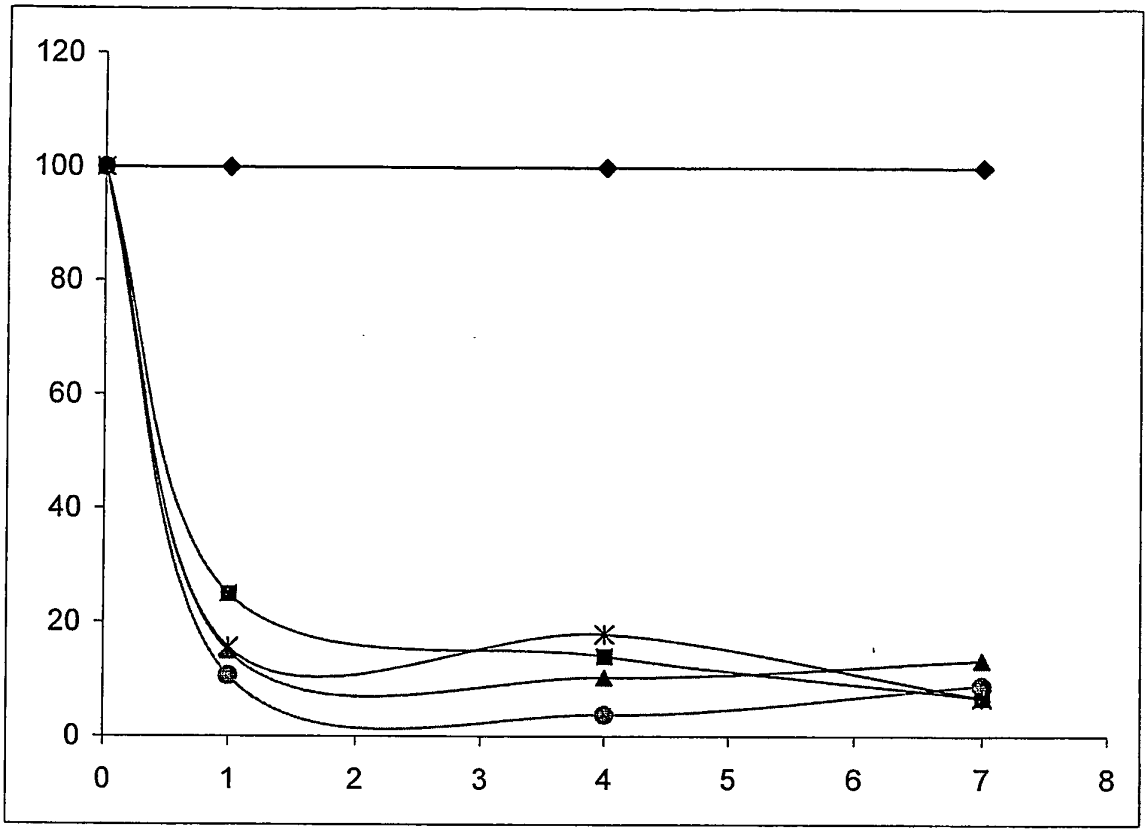


Figure 12

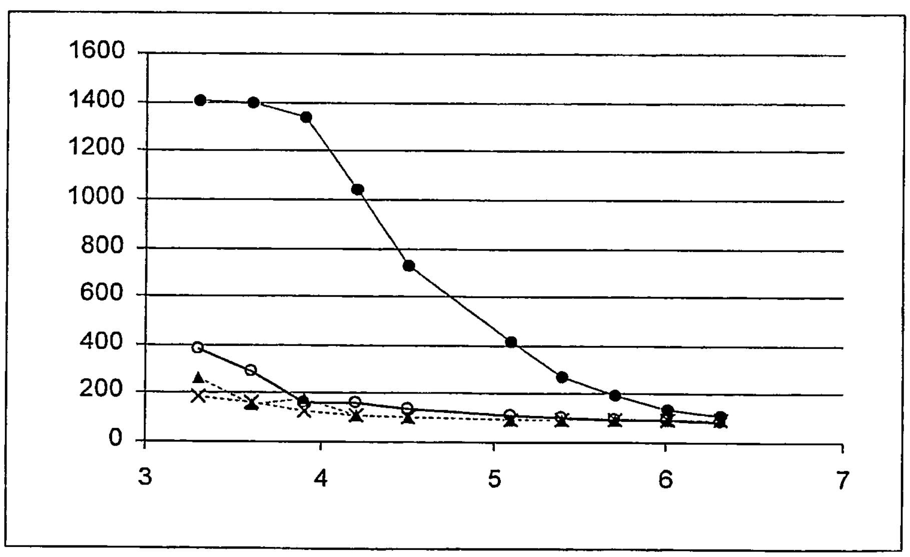


Figure 13

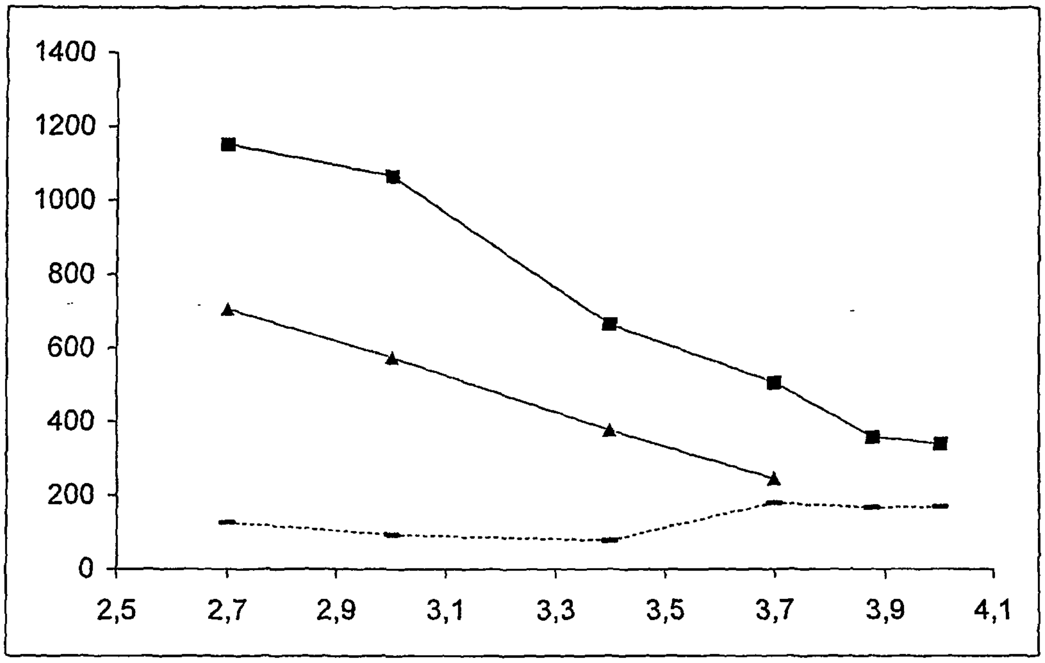


**Figure 14**

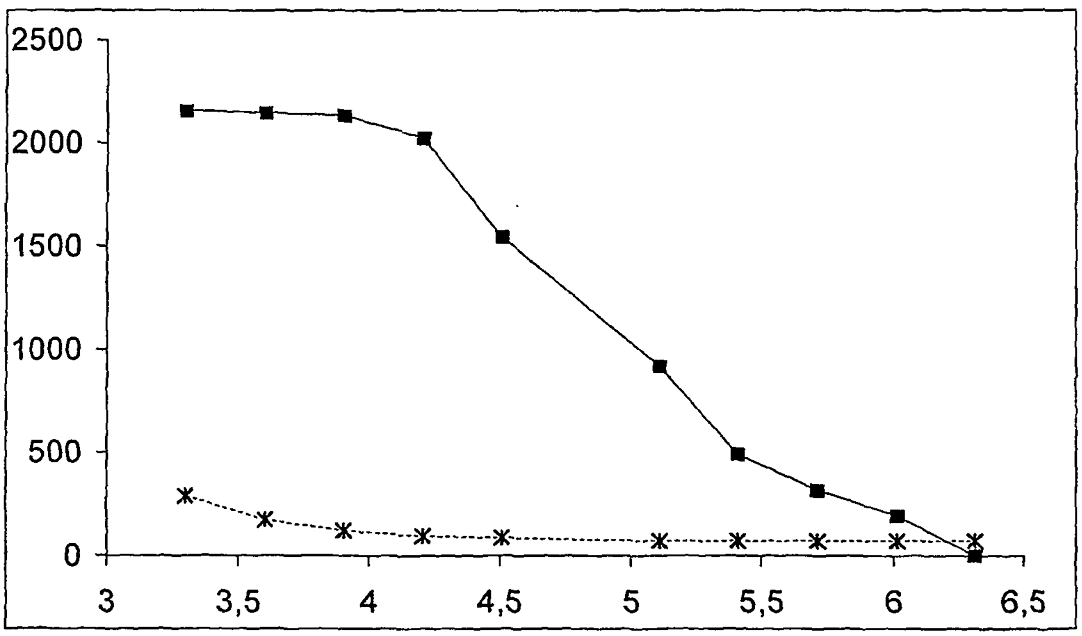


**Figure 15**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 9501380 W
- FR 1995 W


### Littérature non-brevet citée dans la description


- Macromolecularhighly branched homogeneous compound based on lysine units. **Denkewalter, R.G. ; J. Kolc ; W.J. Lukasavage.** USP. Allied Corp, 1981
- Macromolecular highly branched homogeneous compound. **Denkewalter, R.G. ; J. Kolc ; W.J. Lukasavage.** Macromolecular highly branched homogeneous compound. Allied Corp, 1983
- **Juan Rodriguez-Hemandez ; Marco Gatti ; Harm-Anton Klok.** Highly Branched Poly(L-lysine). *Biomacromolecules,* 2003, vol. 4, 249-258
- **Klok Harm-Anton ; Rodriguez-Hernandez Juan.** Dendritic-Graft Polypeptides. *Macromolecules,* 2002, vol. 35, 8718-8723
- Agent for the prevention and treatement of sexually transmitted diseases-I. **Matthews Barry Ross et al.** World Intellectual Property Organization (PCT). Starpharma Limited, 2002, 36
- Anionic or cationic dendrimer-antimicrobial or antiparasitic compositions. **Matthews Barry Ross ; H. George.** USPTO. Starpharma Limited, 2003
- **Baigude H. et al.** Synthesis of sphere-type monodispersed olygosaccharide-polypeptide dendrimers. *Macromolecules,* 2003, vol. 36 (19), 7100-7106
- **Menz T. L. ; Chapman T.** Synthesis and characterization of hyperbranched polylysine. *Polymer Preprints,* 2003, vol. 44 (2), 842-843
- **Takuro, N. et al.** Method of gene delivery into cells using dendritic poly(L-lysine)s. *peptide science,* 2001, vol. 2000 (37th), 201-204
- **Choi Joon Sig et al.** Synthesis of a barbell-like triblock copolymer, poly(L-lysine) dendrimer-bloc-poly(ethylene glycol)-bloc-poly(L-lysine) dendrimer, and its self-assembly with plasmid DNA. *Journal of American Chemical Society.,* 2000, vol. 122, 474-480
- **Kricheldorf, H.R.** α-Aminoacid-N-Carboxy-Anhydrides and Related Heterocycles. Synthesis, Properties, Peptide Synthesis, Polymerisation. Heterocycles. Springer-Verlag, 1987, 213
- **Bartlett, P. ; R. Jones.** A Kinetic Study of the Leuchs Anhydrides in Aqueous Solution. *J. Am. Chem. Soc.,* 1957, vol. 79 (9), 2153-2159
- **Commeyras, A. et al.** Prebiotic Synthesis of Sequential Peptides on the Hadean Beach by a Molecular Engine Working with Nitrogen Oxides as Energy Sources. *Polymer International.,* 2002, vol. 51, 661-665
- **Denkewalter, R.G.S., H. ; Strachan, R. G. ; Beesley, Thomas E. ; Veber, Daniel F. ; Schoenwaldt, Erwin F. ; Barkemeyer, H. ; Paleveda, William J., Jr. ; Jacob, Theodore A. ; Hirschmann, Ralph.** Controlled synthesis of peptides in aqueous medium. I. Use of α-amino acid N-carboxyanhydrides. *Journal of the American Chemical Society,* 1966, vol. 88 (13), 3163-4
- **Hirschmann R. et al.** The controlled Synthesis of peptides in aqueous medium. III. Use of Leuchs' anhydrides in the synthesis of dipeptides. Mécanism and control of side reactions. *Journal of Organic Chemistry,* 1967, vol. 32 (11), 3415-3425
- **Iwakura Y. et al.** Stepwise synthesis of oligopeptides with N-carboxy α-amino acid anhydrides. *Biopolymers,* 1970, vol. 9, 1419-1427
- **Hitz T. H. ; Luisi P. L.** Spontaneous onset of homochirality in oligopeptide chain generated in the polymerisation of N-carboxyanydride amino acids in water. *Origin of life and evolution of the biosphere,* 2004, vol. 34, 93-110
- **Sakamoto M. ; Kuroyanagi Y.** Multichain copoly(α-aminoacid). *Journal of polymer science,* 1978, vol. 16, 1107-1122
- **Birchall A.C. ; North M.** Synthesis of highly branched block copolymers of enantiomerically pure amino acids. *Chem. Commun.,* 1998, 1335-1336
- **Zigang Yang ; Jianjun Yuan ; Shiyuan Cheng.** Self-assembling of biocompatible BAB amphiphilic triblock copolymers PLL(Z)-PEG-PLL(Z) in aqueous medium. *European polymer journal.,* 2005, vol. 41, 267-274
- **Poche D. ; Moore M. ; Bowles J.** An unconventional method for purifying the N-carboxyanhydride derivatives of γ-alkyl-L-glutamates. *Synthetic communications,* 1999, vol. 29 (5), 843-854
- **Rekker R. F.** The hydrophobic fragmental constant. Elsevier, 1977, vol. 1

- **Leuchs, H.** *Ber. Dtsch. Chem. Ges.,* 1906, vol. 39, 857-859
- **Curtius, T. et al.** *J. Prakt. Chem.,* 1930, vol. 125, 211-302
- **Coleman D. ; Farthing A. C.** *J. Chem. Soc.,* 1951, 3218-3222
- **Mobashery, S. ; M. Johnston.** A new appr-oaclz to the preparation of N-carboxy α-amino acid anhydrides. *J. Org. Chem.,* 1985, vol. 50, 2200-2202
- **Daly, W.H. ; D. Poche.** The preparation of N-carboxyanhydrides of α-amino acids using bis(trichloromethil)carbonate. *Tetrahedron Lett.,* 1988, vol. 29, 5859-5862
- **Wilder, R. ; S. Mobashery.** The use of triphosgene in preparation of N-carboxy-α-amino acid anhydrides. *J. Org. Chem.,* 1992, vol. 57, 2755-2756
- **Collet, H. et al.** A new simple and quantitative synthesis of α-Aminoacid-N-Carboxyanhydrides. *Tetrahedron Letters,* 1996, vol. 37 (50), 9043-9046
- **Pascal R. ; Boiteau L. ; Commeyras A.** From the prebiotic synthesis of α-aminoacids, towards a primitive translation apparatus for the synthesis of peptides. *Top. Curr. Chem.,* 2005, 69-122
- Dendrimers and other dendritic polymers. John Wiley & Sons, Ltd, 2001, 646
- Agent for the prevention and treatment of sexually transmitted diseases-II. **Matthews Barry Ross et al.** World Intellectual Property Organization. Starpharma Limited, 2002, 30
- **Bello, M.S. ; R. Rezzonico ; P.G. Righetti.** Use of Taylor-Aris dispersion for measurement of a solute diffusion coefficient in thin capillaries. *Science,* 1994, vol. 266, 773
- **Mes E. P. C. et al.** Comparison of methods for the determination of diffusion coefficients of polymers in dilute solutions the influence of polydispersity. *Journal ofPolymer Science: Part B: Polymer Physics,* 1999, vol. 37, 593-603
- **Edward J. T.** Molecular volumes and the stokes-einstein equation. *Journal of Chemical Education,* 1970, vol. 47, 261
- **Fehlbaum, P. et al.** Insect imunity. Septic injury of Drosophila induces the synthesis of a potent antifungal peptide with sequence homology to plant antifungal peptides. *J. Biol. Chem.,* 1994, vol. 269, 33159-33163
- **Hancock, R.E. ; T. Falla ; M. Brown.** Cationic bactericidal peptides. *Adv. Microb. Physiol.,* 1995, vol. 37, 135-175
- **Vandenabeele-Trambouze, O. et al.** Antibodies directed against L and D isovaline using a chemical derivatizating reagent for the measurement of their enantiomeric ratio in extraterrestrial samples: first step-production and characterization of antibodies. *Chirality,* 2002, vol. 14, 519-526
- **Claeys-Bruno, M. et al.** Methodological approaches for histamine quantification using derivatization by chloroethylnitrosourea and ELISA measurement. Part I: Optimisation of derivated histamine detection with coated-plates using optimal design. *Chemometrics and intelligent Laboratory Systems,* 2006, vol. 80 (2), 176-185
- **Claeys-Bruno, M. et al.** Methodological approaches for histamine quantification using derivatization by chloroethylnitrosourea and ELISA measurement. Part II: Optimisation of the derivatization step. *Chemometrics and intelligent Laboratory Systems,* 2006, vol. 80 (2), 186-197